# EUROPEAN PATENT APPLICATION

(11) **EP 4 512 807 A1**
(43) Date of publication of application: **26.02.2025**
(21) Application number: 23791939.4
(22) Date of filing: 21.04.2023
(51) Int. Cl.: C07D 487/04, A61K 31/496, A61P 3/04, A61P 3/10, A61P 9/00, A61P 9/10, A61P 9/12, A61P 15/00, A61P 25/00, A61P 25/16, A61P 25/18, A61P 25/22, A61P 25/24, A61P 25/30, A61P 27/02, A61P 27/16, A61P 35/00, A61P 43/00, C07D 209/52, C07D 413/04

(54) **BICYCLOAMINE CARBOXAMIDE DERIVATIVE**

(30) Priority: 22.04.2022 JP 2022071112
(71) Applicant: Sumitomo Pharma Co., Ltd., Osaka-shi, Osaka 541-0045 (JP)
(72) Inventor: FUNAKOSHI, Yuta, Osaka-shi, Osaka 554-0022 (JP); IDEUE, Eiji, Osaka-shi, Osaka 554-0022 (JP); SAKAMOTO, Keita, Osaka-shi, Osaka 554-0022 (JP); KOMIYA, Masafumi, Suita-shi, Osaka 564-0053 (JP); NAGAHAMA, Riko, Osaka-shi, Osaka 554-0022 (JP)
(74) Representative: Carpmaels & Ransford LLP
(86) International application number: PCT/JP2023/015863
(87) International publication number: WO 2023/204291

(57) **Abstract**

The present invention relates to a compound of formula (1) or a pharmaceutically acceptable salt thereof with therapeutic and prophylactic effects for a disease associated with orexin receptor type 2, specifically, a disease such as narcolepsy, idiopathic hypersomnia, hypersomnia, sleep apnea syndrome, wherein R¹ and R² are each independently hydrogen atom, etc.; R³ and R⁴ are each independently hydrogen atom, etc.; R⁵ is optionally-substituted C₁₋₄ alkyl; -L¹-ring G is -ring G, -CH₂-ring G, etc.; R⁶ is each independently hydrogen atom, etc.; n is an integer of 1 or 2; ring G is optionally-substituted C₆₋₁₀ aromatic carbocyclyl group, etc.; A¹ is oxygen atom, etc.; A² is oxygen atom, etc.; j, k, 1, and m are each independently an interger of 0, 1, or 2; dashed bond is single bond or double bond; X is -CR⁷-, etc.; R⁷ is hydrogen atom, etc.; and ring E is formula (1a-1), (1a-2), or (1a-3).

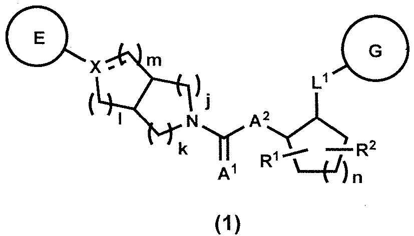

## Description

### TECHNICAL FIELD

The present invention relates to a medicament for treating or preventing a disease associated with orexin receptor, especially orexin receptor type 2, comprising a new compound having a fused ring skeleton or a pharmaceutically acceptable salt thereof as an active ingredient. Specifically, the present invention relates to a medicament for treating or preventing a disease such as narcolepsy, idiopathic hypersomnia, hypersomnia, sleep apnea syndrome.

### BACKGROUND ART

Orexin is a neuropeptide which is specifically produced in a specific neuron spreading across lateral hypothalamus and its adjacent region. Orexin is an endogenous ligand of orexin receptor that is a G-protein-coupled receptor existing mainly in brain, which binds to orexin receptor. It is known that orexin receptor has two subtypes, type 1 and type 2 (Non-Patent Document 1).

It was reported that a narcolepsy-like symptom in a transgenic mouse whose orexin neuron was denatured could be improved by intraventricular injection of an orexin peptide (Non-Patent Document 2), and a narcolepsy-like symptom could be initiated by knocking out (KO) prepro-orexin which is a precursor protein of orexin (Non-patent Reference 3), furthermore the orexin concentration in cerebrospinal fluid of narcolepsy patients was markedly lowered (Non-Patent Document 4). Thus, it is suggested that narcolepsy can be initiated due to lack of orexin.

In addition, it was reported that there was a mutation of orexin receptor type 2 in a dog suffering from hereditary narcolepsy (Non-Patent Document 5), which suggests that orexin receptor type 2 is involved in sleep-wake function. Furthermore, it was revealed that narcolepsy-like symptom was initiated in a KO mouse of orexin receptor type 2 (Non-Patent Document 6), which strongly suggests that the stimulation on orexin receptor type 2 is involved in sleep-wake function. Thus, an orexin receptor type 2 agonist is expected to be a hopeful therapy for a patient presenting with hypersomnia-like symptom such as narcolepsy.

Recently, a compound with orexin receptor type 2 agonistic effect was reported (Patent Document 1 or Patent Document 2).

### PRIOR ART DOCUMENTS

### PATENT DOCUMENTS

Patent Document 1: WO 2017/135306
Patent Document 2: WO 2021/107023

### NON-PATENT DOCUMENTS

Non-Patent Document 1: Cell, Vol.92, 573-585, 1998
Non-Patent Document 2: Proc. Natl. Acad. Sci. USA, Vol. 101, 4649-4654, 2004
Non-Patent Document 3: Cell, Vol. 98, 437-451, 1999
Non-Patent Document 4: THE LANCET, Vol. 355, 39-40, 2000
Non-Patent Document 5: Cell, Vol. 98, 365-376, 1999
Non-Patent Document 6: Neuron, Vol. 38, 715-730, 2003
Non-Patent Document 7: Brain, Vol. 130, 1577-1585, 2007
Non-Patent Document 8: Neuroscience Letters, Vol. 569, 68-73, 2014

### SUMMARY OF THE INVENTION

### PROBLEMS TO BE SOLVED BY THE INVENTION

An object to the present invention is to provide a medicament for treating or preventing a disease associated with orexin receptor type 2, specifically a disease such as narcolepsy, idiopathic hypersomnia, hypersomnia, sleep apnea syndrome.

### SOLUTIONS TO THE PROBLEMS

The present inventors have extensively studied to reach the above object, and then have found that a compound of the following formula (1) or a pharmaceutically acceptable salt thereof (hereinafter, it may be referred to as "the present compound") has therapeutic and prophylactic effect for a disease associated with orexin receptor type 2, specifically, a disease such as narcolepsy, idiopathic hypersomnia, hypersomnia, sleep apnea syndrome. Based on the new findings, the present invention has been completed.

The present invention can show as follows.

### [Item 1]

A compound of formula (1): 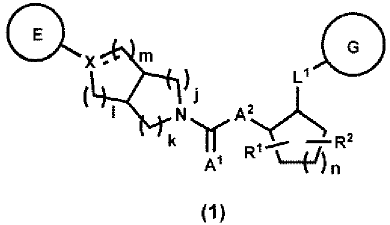 or a pharmaceutically acceptable salt thereof,
wherein
R¹ and R² are each independently hydrogen atom, halogen atom, cyano, - (C=O)NR³R⁴, carboxy group, -(C=O)O-R⁵, optionally-substituted C₁₋₄ alkyl, or optionally-substituted C₁₋₄ alkoxy, and R¹ and R² may bind to the same carbon atom if chemically possible, or when R¹ and R² bind to separate carbon atoms on the ring, they may be taken together via C₁₋₆ alkylene to form a fused ring or a bridged ring;
R³ and R⁴ are each independently hydrogen atom or optionally-substituted C₁₋₄ alkyl;
R⁵ is optionally-substituted C₁₋₄ alkyl;
-L¹-ring G is -ring G, -CH₂-ring G (wherein the CH₂ may optionally be substituted with the same or different one or more C₁₋₆ alkyl groups), -NR⁶-ring G, - C(=O)-ring G, -OC(=O)-ring G, -SO-ring G, -SO₂-ring G, -S-ring G, or -O-ring G;
R⁶ is hydrogen atom or optionally-substituted C₁₋₄ alkyl;
n is an integer of 1 or 2;
ring G is optionally-substituted C₆₋₁₀ aromatic carbocyclyl group, optionally-substituted 5- to 10-membered aromatic heterocyclyl group, optionally-substituted C₃₋₆ saturated carbocyclyl group, or optionally-substituted 4- to 10-membered saturated heterocyclyl group;
A¹ is oxygen atom or sulfur atom;
A² is oxygen atom or -NH-;
j, k, l, and m are each independently an integer of 0, 1, or 2;
dashed bond is single bond or double bond;
X is -CR⁷-, nitrogen atom, or carbon atom;
R⁷ is hydrogen atom, hydroxy group, halogen atom, cyano, or optionally-substituted C₁₋₄ alkyl; and
ring E is the following formula (la-1), (1a-2), or (la-3): 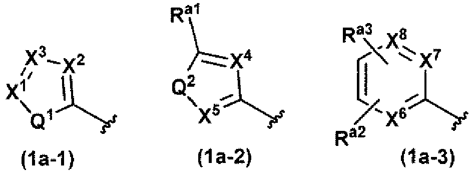 wherein
   X¹ to X⁸ are each independently nitrogen atom or -CR^{a4}-;
   Q¹ and Q² are oxygen atom or -NR^{a5}-:
   R^{a1} to R^{a5} are each independently, (if there are plural CR^{a4}, each R^{a4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are CR^{a4}, two R^{a4} may be combined with the carbon atoms to which they are attached to form a 6-membered carbocyclic ring fused to a 5-membered ring formed by X¹, X² and X³.

### [Item 2]

The compound according to Item 1 or a pharmaceutically acceptable salt thereof, wherein in R¹ to R⁷, the optional substituent of "optionally-substituted C₁₋₄ alkyl" is each independently the same or different one or more substituents selected from halogen atom, hydroxy group, C₁₋₄ alkoxy, or C₃₋₇ cycloalkyl, and the optional substituent of "optionally-substituted C₁₋₄ alkoxy" is each independently the same or different one or more substituents selected from halogen atom, hydroxy group, or C₃₋₇ cycloalkyl,
in ring G, the optional substituent of C₆₋₁₀ aromatic carbocyclyl group, 5- to 10-membered aromatic heterocyclyl group, C₃₋₆ saturated carbocyclyl group, and 4- to 10-membered saturated heterocyclyl group is each independently one or more substituents selected from the group consisting of halogen atom, C₁₋₆ alkyl (wherein the alkyl may be optionally substituted with the same or different one or more substituents selected from halogen atom, hydroxy group, C₁₋₄ alkoxy, or C₃₋₇ cycloalkyl), C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, C₁₋₄ alkoxy, or C₃₋₇ cycloalkyl), C₁₋₄ alkoxy (wherein the alkoxy may be optionally substituted with the same or different one or more substituents selected from halogen atom, hydroxy group, or C₃₋₇ cycloalkyl), C₃₋₇ cycloalkyl (wherein the cycloalkyl may be optionally substituted with the same or different one or more substituents selected from halogen atom, hydroxy group, C₁₋₄ alkyl, C₁₋₄ alkoxy, or C₃₋₇ cycloalkyl), and C₃₋₇ cycloalkoxy (wherein the cycloalkoxy may be optionally substituted with the same or different one or more substituents selected from halogen atom, hydroxy group, C₁₋₄ alkyl, C₁₋₄ alkoxy, or C₃₋₇ cycloalkyl), or when there are plural optional substituents, two of them may be taken together via C₁₋₆ alkylene to form a chemically-possible bicyclic structure selected from a fused ring, a spiro ring, or a bridged ring.

### [Item 3]

The compound according to Item 1 or 2 or a pharmaceutically acceptable salt thereof, wherein in R¹ to R⁷, the optional substituent of "optionally-substituted C₁₋₄ alkyl" is each independently the same or different one or more substituents selected from halogen atom or C₁₋₄ alkoxy, and the optional substituent of "optionally-substituted C₁₋₄ alkoxy" is each independently the same or different one or more halogen atoms,
in ring G, the optional substituent of C₆₋₁₀ aromatic carbocyclyl group, 5- to 10-membered aromatic heterocyclyl group, C₃₋₆ saturated carbocyclyl group, and 4- to 10-membered saturated heterocyclyl group is each independently one or more substituents selected from the group consisting of halogen atom, C₁₋₆ alkyl (wherein the alkyl may be optionally substituted with the same or different one or more substituents selected from halogen atom or C₁₋₄ alkoxy), C₆₋₁₀ aromatic carbocyclyl group (wherein the C₆₋₁₀ aromatic carbocyclyl group may be optionally substituted with the same or different one or more substituents selected from halogen atom, C₁₋₄ alkyl, or C₁₋₄ alkoxy), C₁₋₄ alkoxy (wherein the alkoxy may be optionally substituted with the same or different one or more halogen atoms), C₃₋₇ cycloalkyl (wherein the cycloalkyl may be optionally substituted with the same or different one or more substituents selected from halogen atom, C₁₋₄ alkyl, or C₁₋₄ alkoxy), and C₃₋₇ cycloalkoxy (wherein the cycloalkoxy may be optionally substituted with the same or different one or more substituents selected from halogen atom, C₁₋₄ alkyl, or C₁₋₄ alkoxy), or when there are plural optional substituents, two of them may be taken together via C₁₋₆ alkylene to form a chemically-possible bicyclic structure selected from a fused ring, a spiro ring, or a bridged ring.

### [Item 4]

The compound according to any one of Items 1 to 3 or a pharmaceutically acceptable salt thereof, wherein ring E is the following formula (1a-1-1), (la-2-1), {1a-3-1), (1a-3-2), (1a-3-3), or (1a-3-4): 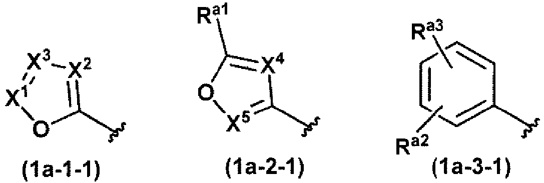 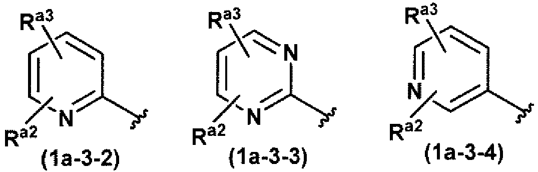 wherein
X¹ to X⁵ are each independently nitrogen atom or -CR^{a4}-; and
R^{a1} to R^{a4} are each independently, (if there are plural CR^{a4}, each R^{a4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a 6-membered carbocyclic ring fused to a 5-membered ring formed by X¹, X² and X³.

### [Item 5]

The compound according to any one of Items 1 to 4 or a pharmaceutically acceptable salt thereof, wherein ring G is the following formula (1b-1) or (1b-2): 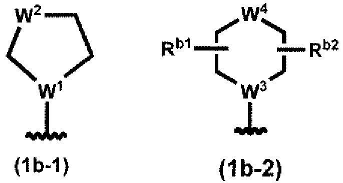 wherein
W¹ and W³ are each independently nitrogen atom or -CR^{b3}-;
W² and W⁴ are each independently -NR^{b4}-, oxygen atom, or -CR^{b5}R^{b6}-;
R^{b1} to R^{b6} are each independently hydrogen atom, -N(R^{b7})R^{b8}, C₁₋₆ alkyl (wherein the alkyl may optionally substituted with the same or different one or more substituents selected from halogen atom, C₃₋₆ cycloalkyl (wherein the cycloalkyl may be optionally substituted with the same or different one or more substituents selected from halogen atom or C₁₋₄ alkyl wherein the C₁₋₄ alkyl may be optionally substituted with halogen atom(s)), or C₁₋₄ alkoxy), C₆₋₁₀ aromatic carbocyclyl group (wherein the C₆₋₁₀ aromatic carbocyclyl group may be optionally substituted with the same or different one or more substituents selected from halogen atom, C₁₋₄ alkyl, or C₁₋₄ alkoxy), C₁₋₄ alkoxy (wherein the alkoxy may be optionally substituted with the same or different one or more halogen atoms), C₃₋₇ cycloalkyl (wherein the cycloalkyl may be optionally substituted with the same or different one or more substituents selected from halogen atom, C₁₋₄ alkyl, or C₁₋₄ alkoxy), or C₃₋₇ cycloalkoxy (wherein the cycloalkoxy may be optionally substituted with the same or different one or more substituents selected from halogen atom, C₁₋₄ alkyl, or C₁₋₄ alkoxy), and R^{b1} and R^{b2} may bind to the same carbon atom if chemically possible;
wherein R^{b1} and R^{b2} may be taken together via C₁₋₆ alkylene to form a chemically-possible bicyclic structure selected from a fused ring, a spiro ring, or a bridged ring;
R^{b7} and R^{b8} are each independently hydrogen atom, C₁₋₆ alkyl (wherein the alkyl may be optionally substituted with the same or different one or more substituents selected from halogen atom, C₁₋₄ alkoxy, C₃₋₇ cycloalkyl (wherein the cycloalkyl may be optionally substituted with one or more substituents selected from halogen atom or C₁₋₄ alkyl wherein the C₁₋₄ alkyl may be optionally substituted with halogen atom(s)), or 5- to 10-membered aromatic heterocyclyl group), 5- to 10-membered aromatic heterocyclyl group (wherein the aromatic heterocyclyl group may be optionally substituted with the same or different one or more substituents selected from halogen atom, C₁₋₄ alkyl, or C₁₋₄ alkoxy), or C₃₋₇ cycloalkyl (wherein cycloalkyl may be optionally substituted with the same or different one or more substituents selected from halogen atom, hydroxy group, C₁₋₄ alkyl, or C₁₋₄ alkoxy),
wherein when R^{b1} to R^{b6} are -N(R^{b7})R^{b8}, R^{b7} and R^{b8} may be combined with the nitrogen atom to which they are attached to form a 3- to 7-membermed nitrogen-containing saturated heterocycle.

### [Item 6]

The compound according to any one of Items 1 to 5 or a pharmaceutically acceptable salt thereof, wherein the formula is formula (2): 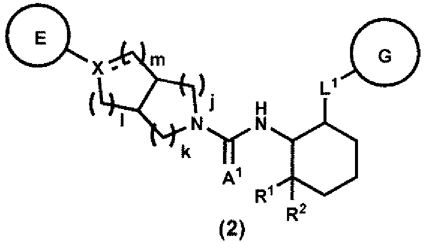 wherein
R¹ and R² are each independently hydrogen atom, halogen atom, C₁₋₄ alkyl (wherein the alkyl may be optionally substituted with the same or different one or more substituents selected from halogen atom, hydroxy group, C₁₋₄ alkoxy, or C₃₋₇ cycloalkyl), or C₁₋₄ alkoxy (wherein the alkoxy may be optionally substituted with the same or different one or more substituents selected from halogen atom, hydroxy group, or C₃₋₇ cycloalkyl);
-L'-ring G is -ring G, -CH₂-ring G (wherein the CH₂ may be optionally substituted with the same or different one or more C₁₋₆ alkyl groups), -NR⁶-ring G, - C(=O)-ring G, -OC(=O)-ring G, -SO-ring G, -SO₂-ring G, -S-ring G, or -O-ring G;
R⁶ is each independently hydrogen atom or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring G is the following formula (1b-1) or (1b-2): 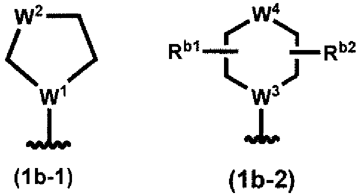 wherein
   W¹ and W³ are each independently nitrogen atom or -CR^{b3}-;
   W² and W⁴ are each independently -NR^{b4}-, oxygen atom, or -CR^{b5}R^{b6}-;
   R^{b1} to R^{b6} are each independently hydrogen atom, -N(R^{b7})R^{b8}, C₁₋₆ alkyl (wherein the alkyl may be optionally substituted with the same or different one or more substituents selected from halogen atom, C₃₋₆ cycloalkyl (wherein the cycloalkyl may be optionally substituted with the same or different one or more substituents selected from halogen atom or C₁₋₄ alkyl wherein the C₁₋₄ alkyl may be optionally substituted with halogen atom(s)), or C₁₋₄ alkoxy), C₆₋₁₀ aromatic carbocyclyl group (wherein the C₆₋₁₀ aromatic carbocyclyl group may be optionally substituted with the same or different one or more substituents selected from halogen atom, C₁₋₄ alkyl, or C₁₋₄ alkoxy), C₁₋₄ alkoxy (wherein the alkoxy may be optionally substituted with the same or different one or more halogen atom(s)), C₃₋₇ cycloalkyl (wherein the cycloalkyl may be optionally substituted with one or more substituents selected from halogen atom, C₁₋₄ alkyl, or C₁₋₄ alkoxy), or C₃₋₇ cycloalkoxy (wherein the cycloalkoxy may be optionally substituted with the same or different one or more substituents selected from halogen atom, C₁₋₄ alkyl, or C₁₋₄ alkoxy), and R^{b1} and R^{b2} may bind to the same carbon atom if chemically possible:
   wherein R^{b1} and R^{b2} may be taken together via C₁₋₆ alkylene to form a chemically-possible bicyclic structure selected from a fused ring, a spiro ring, or a bridged ring;
   R^{b7} and R^{b8} are each independently hydrogen atom, C₁₋₆ alkyl (wherein the alkyl may be optionally substituted with the same or different one or more substituents selected from halogen atom, C₁₋₄ alkoxy, C₃₋₇ cycloalkyl (wherein the cycloalkyl may be optionally substituted with the same or different one or more substituents selected from halogen atom or C₁₋₄ alkyl wherein C₁₋₄ alkyl may be optionally substituted with halogen atom(s)), or 5- to 10-membered aromatic heterocyclyl group), 5- to 10-membered aromatic heterocyclyl group (wherein the aromatic heterocyclyl group may be optionally substituted with the same or different one or more substituents selected from halogen atom, C₁₋₄ alkyl, or C₁₋₄ alkoxy), or C₃₋₇ cycloalkyl (wherein the cycloalkyl may be optionally substituted with the same or different one or more substituents selected from halogen atom, hydroxy group, C₁₋₄ alkyl, or C₁₋₄ alkoxy),
   wherein when R^{b1} to R^{b6} are -N(R^{b7})R^{b8}, R^{b7} and R^{b8} may be combined with the nitrogen atom to which they are attached to form a 3- to 7-membered nitrogen-containing saturated heterocycle;
      A¹ is oxygen atom or sulfur atom;
      j, k, l, and m are each independently an integer of 0, 1, or 2;
      dashed bond is single bond or double bond;
      X is -CR⁷-, nitrogen atom, or carbon atom;
      R⁷ is hydrogen atom, hydroxy group, halogen atom, cyano, or C₁₋₄ alkyl (wherein the alkyl may be optionally substituted with the same or different one or more substituents selected from halogen atom, hydroxy group, C₁₋₄ alkoxy, or C₃₋₇ cycloalkyl);
      ring E is the following formula (1a-1-1), (1a-2-1), (1a-3-1), (la-3-2), (1a-3-3), or (la-3-4): 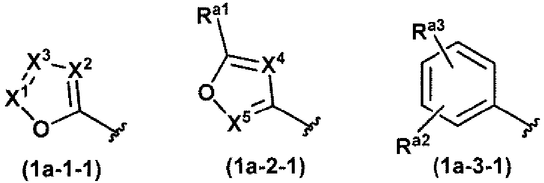 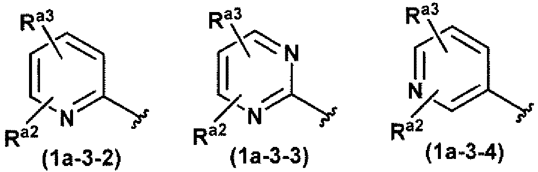
   wherein
      X¹ to X⁵ are each independently nitrogen atom or -CR^{a4}-;
      R^{a1} to R^{a4} are each independently, (if there are plural CR^{a4}, each R^{a4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, C₁₋₄ alkyl, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5-to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a δ-membered carbocyclic ring fused to a 5-membered ring formed by X¹, X² and X³.

### [Item 7]

The compound according to Item 6 or a pharmaceutically acceptable salt thereof, wherein R¹ and R² are each independently hydrogen atom, halogen atom, or C₁₋₄ alkyl (wherein the alkyl may be optionally substituted with the same or different one or more substituents selected from halogen atom, hydroxy group, C₁₋₄ alkoxy, or C₃₋₇ cycloalkyl);
L¹ is single bond or oxygen atom;
ring G is the following formula (lb-1-1), (lb-2-1), (lb-1-2), or (1b-2-2): 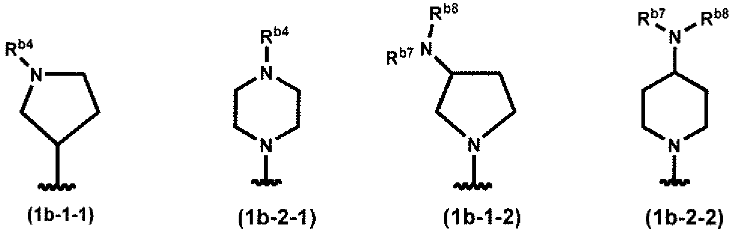
wherein R^{b4}, R^{b7}, and R^{b8} are each independently hydrogen atom or C₁₋₆ alkyl (wherein the alkyl may be optionally substituted with the same or different one or more substituents selected from halogen atom or C₁₋₄ alkoxy).

### [Item 8]

The compound according to any one of Items 1 to 7 or a pharmaceutically acceptable salt thereof, wherein the formula is formula (3): 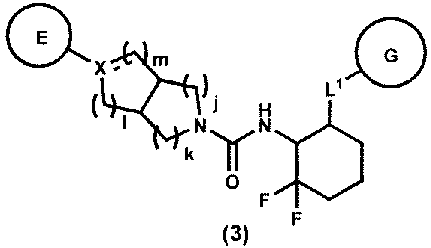 wherein
L¹ is single bond or oxygen atom;
ring G is the following formula (lb-1-1), (lb-2-1), (lb-1-2), or (lb-2-2): 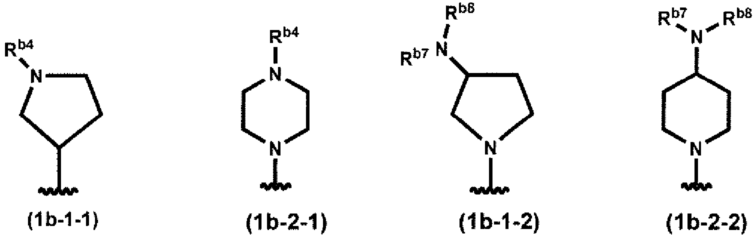 wherein R^{b4}, R^{b7}, and R^{b8} are each independently hydrogen atom or C₁₋₆ alkyl (wherein the alkyl may be optionally substituted with the same or different one or more substituents selected from halogen atom or C₁₋₄ alkoxy),
j, k, l, and m are each independently an integer of 0, 1, or 2;
dashed bond is single bond or double bond;
X is -CR⁷-, nitrogen atom, or carbon atom;
R⁷ is hydrogen atom, hydroxy group, halogen atom, cyano, or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring E is the following formula (1a-1-1), (1a-2-1), (1a-3-1), (1a-3-2), (1a-3-3), or (la-3-4): 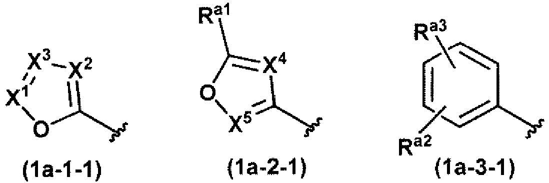 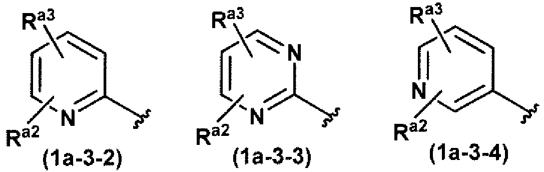 wherein
   X¹ to X⁵ are each independently nitrogen atom or -CR^{a4}-;
   R^{a1} to R^{a4} are each independently, (if there are plural CR^{a4}, each R^{a4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a 6-membered carbocyclic ring fused to a 5-membered ring formed by X¹, X² and X³.

### [Item 9]

The compound according to any one of Items 1 to 8 or a pharmaceutically acceptable salt thereof, wherein the formula is formula (4): 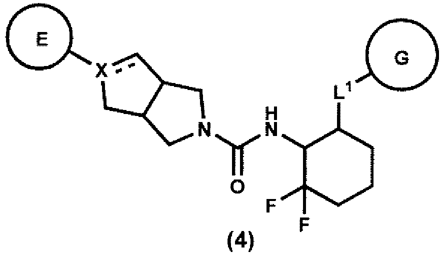 wherein
L¹ is single bond or oxygen atom;
ring G is the following formula (1b-1-1), (1b-2-1), (1b-1-2), or (1b-2-2): 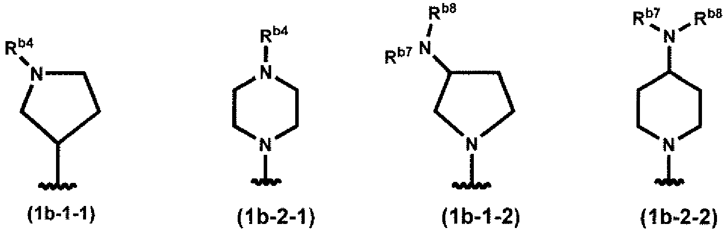 wherein R^{b4}, R^{b7}, and R^{b8} are each independently hydrogen atom or C₁₋₆ alkyl (wherein the alkyl may be optionally substituted with the same or different one or more substituents selected from halogen atom or C₁₋₄ alkoxy);
dashed bond is single bond or double bond;
X is -CR⁷-, nitrogen atom, or carbon atom;
R⁷ is hydrogen atom or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring E is the following formula (1a-1-1), (1a-2-1), (1a-3-1), (1a-3-2), (1a-3-3), or (1a-3-4): 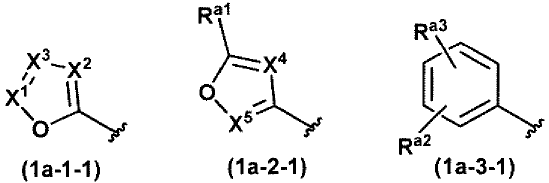 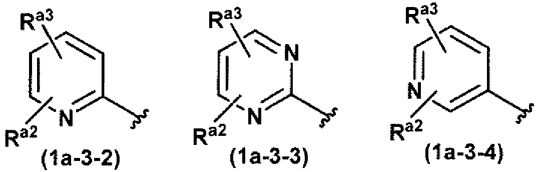 wherein
   X¹ to X⁵ are each independently nitrogen atom or -CR^{a4}-;
   R^{a1} to R^{a4} are each independently, (if there are plural CR^{a4}, each R^{a4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a 6-membered carbocyclic ring fused to a 5-membered ring formed by X¹, X² and X³.

### [Item 10]

The compound according to Item 9 or a pharmaceutically acceptable salt thereof, wherein ring E is the above formula (1a-2-1) or (1a-3-1) wherein X⁴ to X⁵ are each independently nitrogen atom or -CR^{a4}-; and R^{a1} to R^{a4} are each independently, (if there are plural CR^{a4}, each R^{a4} is also independently), hydrogen atom, halogen atom, C₁₋₄ alkyl (wherein the alkyl may be optionally substituted with the same or different one or more substituents selected from halogen atom or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), or C₁₋₄ alkoxy (wherein the alkoxy may be optionally substituted with the same or different one or more halogen atom(s)).

### [Item 11]

The compound according to Item 9 or a pharmaceutically acceptable salt thereof, wherein ring E is the following formula (la-2-2) or (1a-3-1): 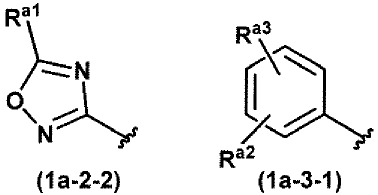 wherein R^{a1} to R^{a3} are each independently hydrogen atom, halogen atom, C₁₋₄ alkyl (wherein the alkyl may be optionally substituted with the same or different one or more substituents selected from halogen atom or C₁₋₄ alkoxy), or C₃₋₇ cycloalkyl (wherein the cycloalkyl may be optionally substituted with the same or different one or more substituents selected from halogen atom, hydroxy group, C₁₋₄ alkyl, or C₁₋₄ alkoxy),

### [Item 12]

The compound according to any one of Items 1 to 11 or a pharmaceutically acceptable salt thereof, wherein the formula is formula (5): 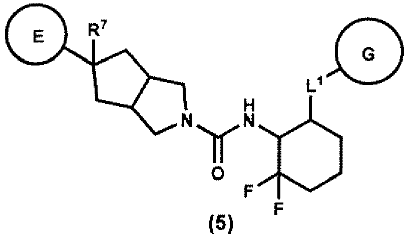 wherein
L¹ is single bond or oxygen atom;
ring G is the following formula (1b-1-1) or (lb-2-1): 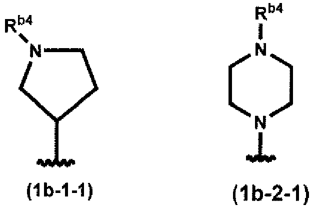 wherein R^{b4} is hydrogen atom or C₁₋₆ alkyl (wherein the alkyl may be optionally substituted with the same or different one or more substituents selected from halogen atom or C₁₋₄ alkoxy);
R⁷ is hydrogen atom or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring E is the following formula (1a-2-2) or (1a-3-1): 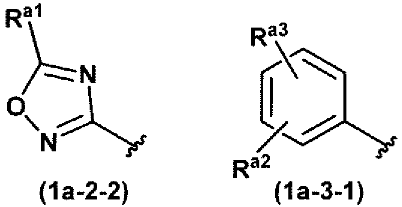 wherein R^{a1} to R^{a3} are each independently hydrogen atom, halogen atom, C₁₋₄ alkyl (wherein the alkyl may be optionally substituted with the same or different one or more substituents selected from halogen atom or C₁₋₄ alkoxy), or C₃₋₇ cycloalkyl (wherein the cycloalkyl may be optionally substituted with the same or different one or more substituents selected from halogen atom, hydroxy group, C₁₋₄ alkyl, or C₁₋₄ alkoxy).

### [Item 13]

The compound according to Item 12 or a pharmaceutically acceptable salt thereof, wherein the ring G is the above formula (1b-1-1) and R^{b4} is C₁₋₄ alkyl which may be optionally substituted with the same or different one or more halogen atom(s).

### [Item 14]

The compound according to Item 12 or a pharmaceutically acceptable salt thereof, wherein the ring G is the above formula (1b-2-1) and R^{b4} is hydrogen atom or C₁₋₄ alkyl wherein the alkyl may be optionally substituted with the same or different one or more substituents selected from halogen atom or C₁₋₄ alkoxy.

### [Item 15]

The compound according to Item 12 or a pharmaceutically acceptable salt thereof, wherein R⁷ is hydrogen atom.

### [Item 16]

The compound according to Item 1 selected from the following compounds:
Example 1: (3aR,5S,6aS)-5-(5-cyclopropyl-1,2,4-oxadiazol-3-yl)-N-{(1R,6S)-2,2-difluoro-6-[4-(propan-2-yl)piperazin-1-yl]cyclohexyl}hexahydropenta[c]pyrrole-2(1H)-carboxamide,
Example 2: (3aR,5S,6aS)-N-{(1R,6S)-2,2-difluoro-6-[4-(propan-2-yl)piperazin-1-yl]cyclohexy}-5-{5-[(1S,2S)-2-fluorocyclopropyl]-1,2,4-oxadiazol-3-yl}hexahydropenta[c]pyrrole-2(1H)-carboxamide,
Example 3: (1R,5S,6R)-6-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[3.1.0]hexane-3-carboxamide,
Example 4: (1R,5S,6S)-6-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[3.1.0]hexane-3-carboxamide,
Example 6: (3aR,5R,6aS)-5-(5-cyclopropyl-1,2,4-oxadiazol-3-yl)-N-{(1R,6S)-2,2-difluoro-6-[4-(propan-2-yl)piperazin-1-yl]cyclohexyl}-5-methylhexahydropenta[c]pyrrole-2(1H)-carboxamide,
Example 7: (3aR,5R,6aS)-N-{(1R,6S)-2,2-difluoro-6-[4-(propan-2-yl)piperazin-1-yl]cyclohexyl}-5-{5-[(1S,2S)-2-fluorocyclopropyl]-1,2,4-oxadiazol-3-yl}-5-methylhexahydropenta[c]pyrrole-2(1H)-carboxamide,
Example 8: (3aR,5R,6aS)-5-(5-cyclopropyl-1,2,4-oxadiazol-3-yl)-N-{(1R,6S)-2,2-difluoro-6-[4-(propan-2-yl)piperazin-1-yl]cyclohexyl}hexahydropenta[c]pyrrole-2(1H)-carboxamide,
Example 9: (3aR,5R,6aS)-N-{(1R,6S)-2,2-difluoro-6-[4-(propan-2-yl)piperazin-1-yl]cyclohexyl}-5-phenylhexahydropenta[c]pyrrole-2(1H)-carboxamide,
Example 11: (3aR,6aS)-N-{(1R,6S)-2,2-difluoro-6-[4-(propan-2-yl)piperazin-1-yl]cyclohexyl }-5-phenylhexahydropyrrolo[3,4-c]pyrrole-2(1H)-carboxamide,
Example 12: (3aR,5S,6aS)-N-{(1R,6S)-2,2-difluoro-6-[4-(propan-2-yl)piperazin-1-yl]cyclohexyl}-5-phenylhexahydropenta[c]pyrrole-2(1H)-carboxamide,
Example 13: (3aR,5R,6aS)-N-{(1R,6S)-2,2-difluoro-6-[4-(propan-2-yl)piperazin-1-yl]cyclohexyl }-5-(4-methylphenyl)hexahydropenta[c]pyrrole-2(1H)-carboxamide,
Example 14: (3aR,5R,6aS)-N-{(1R,6S)-2,2-difluoro-6-[4-(propan-2-yl)piperazin-1-yl]cyclohexyl}-5-(2-methylphenyl)hexahydropenta[c]pyrrole-2(1H)-carboxamide,
Example 15: (3aR,5R,6aS)-N-{(1R,6S)-2,2-difluoro-6-[4-(propan-2-yl)piperazin-1-yl]cyclohexyl}-5-(4-fluorophenyl)hexahydropenta[c]pyrrole-2(1H)-carboxamide,
Example 16: (3aR,5R,6aS)-N-{(1R,6S)-2,2-difluoro-6-[4-(propan-2-yl)piperazin-1-yl]cyclohexyl}-5-(3-fluorophenyl)hexahydropenta[c]pyrrole-2(1H)-carboxamide,
Example 17: (3aR,5R,6aS)-N-{(1R,6S)-2,2-difluoro-6-[4-(propan-2-yl)piperazin-1-yl]cyclohexyl}-5-(2-fluorophenyl)hexahydropenta[c]pyrrole-2(1H)-carboxamide,
Example 5-A: (1S,6R,8S)-8-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[4.2.0]octane-3-carboxamide,
Example 5-B: (1R,6S,8R)-8-(5-cyclopropyl-1,2,4-oxadiazol-3-yl)-N-{(1R,6S)-2,2-difluoro-6-[4-(propan-2-yl)piperazin-1-yl]cyclohexyl)-3-azabicyclo[4.2.0]octane-3-carboxamide,
Example 10-A: (3aS,6aR)-N-{(1R,6S)-2,2-difluoro-6-[4-(propan-2-yl)piperazin-1-yl]cyclohexyl}-5-phenyl-3,3a,4,6a-tetrahydropenta[c]pyrrole-2(1H)-carboxamide,
Example 10-B: (3aR,6aS)-N-{(1R,6S)-2,2-difluoro-6-[4-(propan-2-yl)piperazin-1-yl]cyclohexyl}-5-phenyl-3,3a,4,6a-tetrahydropenta[c]pyrrole-2(1H)-carboxamide,
Example 18: (3aR,6aS)-N-{(1R,6S)-2,2-difluoro-6-[4-(propan-2-yl)piperazin-1-yl]cyclohexyl}-5-(4-fluorophenyl)hexahydropyrrolo[3,4-c]pyrrole-2(1H)-carboxamide,
Example 19: (3aR,6aS)-N- {(1R,6S)-2,2-difluoro-6-[4-(propan-2-yl)piperazin-1-yl]cyclohexyl } -5-(2-fluorophenyl)hexahydropyirrolot3,4-c]pyrrole-2(1H)-carboxamide,
Example 20: (3aR,6aS)-N-{(1R,6S)-2,2-difluoro-6-[4-(propan-2-yl)piperazin-1-yl]cyclohexyl }-5-(pyridin-2-yl)hexahydropyrrolo[3,4-c]pyrrole-2(1H)-carboxamide,
Example 21: (3aR,6aS)-N-{(1R,6S)-2,2-difluoro-6-[4-(propan-2-yl)piperazin-1-yl]cyclohexyl}-5-(pyrimidin-2-yl)hexahydropyrrolo[3,4-c]pyirrole-2(1H)-carboxamide,
Example 22: (3aR,6aS)-N-{(1R,6S)-2,2-difluoro-6-[4-(propan-2-yl)piperazin-1-yl]cyclohexyl}-5-(3-fluorophenyl)hexahydropyrrolo[3,4-c]pyrrole-2(1H)-carboxamide,
Example 23: (3aR,6aS)-N- {(1R,6S)-2,2-difluoro-6-[4-(propan-2-yl)piperazin-1-yl]cyclohexyl}-5-(4-methylphenyl)hexahydropyrrolo[3,4-c]pyrrole-2(1H)-carboxamide,
Example 24: (3aR,5R,6aS)-N-{(1R,6S)-2,2-difluoro-6-[4-(propan-2-yl)piperazin-1-yl]cyclohexyl}-5-(3-methylphenyl)hexahydropenta[c]pyrrole-2(1H)-carboxamide,
Example 25: (3aR,5R,6aS)-N-{(1R,6S)-2,2-difluoro-6-[4-(propan-2-yl)piperazin-1-yl]cyclobexyl} -5-(pyridin-2-yl)hexahydropenta[c]pyrrole-2(1H)-carboxamide,
Example 26: (3aR,5R,6aS)-N-{(1R,6S)-2,2-difluoro-6-[4-(propan-2-yl)piperazin-1-yl]cyclohexyl}-5-(pyrimidin-2-yl)hexahydropenta[c]pyrrole-2(1H)-carboxamide,
Example 27: (3aR,5R,6aS)-N-{(1R,6S)-2,2-difluoro-6-[4-(propan-2-yl)piperazin-1-yl]cyclohexyl}-5-(pyridin-3-yl)hexahydropenta[c]pyrrole-2(1H)-carboxamide,
Example 28: (3aR,5R,6aS)-N-[(1R,6S)-2,2-difluoro-6-{[(3S)-1-(propan-2-yl)pyrrolidin-3-yl]oxy}cyclohexyl]-5-phenylhexahydropenta[c]pyrrole-2(1H)-carboxamide,
Example 29: (3aR,6aS)-N-[(1R,6S)-2,2-difluoro-6-{[(3S)-1-(propan-2-yl)pyrrolidin-3-yl]oxy}cyclohexyl]-5-phenylhexahydropyrrolo[3,4-c]pyrrole-2(1H)-carboxamide,
Example 30: (3aR,5R,6aS)-N-{(1R,6S)-2,2-difluoro-6-[4-(propan-2-yl)piperazin-1-yl]cyclohexy]}-5-(5-methyl-1,2,4-oxadiazol-3-yl)hexahydropenta[c]pyrrole-2(1H)-carboxamide,
Example 31: (3aR,5R,6aS)-N-[(1R,6S)-2,2-difluoro-6-{methyl[(3S)-1-(propan-2-yl)pyrrolidin-3-yl]amino}cyclohexyl]-5-(5-methyl-1,2,4-oxadiazol-3-yl)hexahydropenta[c]pyrrole-2(1H)-carboxamide,
Example 32: (3aR,5R.6aS)-N-[(1R,6S)-2,2-difluoro-6-1[(3S)-I-(propan-2-yl)pyrrolidin-3-yl]oxy}cyclohexyl]-5-(5-methyl-1,2,4-oxadiazol-3-yl)hexahydropenta[c]pyrrole-2(1H)-carboxamide,
Example 33: (3aR,5R,6aS)-N-[(1R,6S)-2,2-difluoro-6-{methyl[(3S)-1-(propan-2-yl)pyrrolidin-3-yl]amino}cyclohexyl]-5-phenylhexahydropenta[c]pyrrole-2(1H)-carboxamide,
Example 34: (3aR,6aS)-N-[(1R,6S)-2,2-difluoro-6-{methyl[(3S)-1-(propan-2-yl)pyrrolidin-3-yl]amino}cyclohexyl]-5-phenylhexahydropyrrolo[3,4-c]pyrrole-2(1H)-carboxamide,
or a pharmaceutically acceptable salt thereof.

### [Item 17]

A pharmaceutical composition comprising the compound according to any one of Items 1 to 16 or a pharmaceutically acceptable salt thereof.

### [Item 18]

A pharmaceutical composition comprising the compound according to any one of Items 1 to 16 or a pharmaceutically acceptable salt thereof for use in the treatment of narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

### [Item 19]

A medicament for treating a disease associated with orexin receptor type 2 comprising the compound according to any one of Items 1 to 16 or a pharmaceutically acceptable salt thereof.

### [Item 20]

A medicament for treating narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body, hypersomnia syndrome involving daytime hypersomnia (for example, Kleine-Levin syndrome, major depression accompanied by hypersomnia, dementia with Lewy body. Parkinson's disease, progressive supranuclear palsy, Prader-Willi syndrome, Moebius syndrome, hypoventilation syndrome, Niemann-Pick disease type C, brain contusion, cerebral infarction, brain tumor, muscular dystrophy, multiple sclerosis, acute disseminated encephalomyelitis, Guillain-Barre syndrome, Rasmussen's encephalitis, Wemicke's encephalopathy, limbic encephalitis, Hashimoto encephalopathy), coma, loss of consciousness, obesity (for example, malignant mast cell, extrinsic obesity, hyperinsulinar obesity, hyperplasmic obesity, hypophysial obesity, hypoplasmic obesity, hypothyroid obesity, hypothalamic obesity, symptomatic obesity, childhood obesity, upper body obesity, alimentary obesity, gonadal obesity, systemic mastocytosis, primary obesity, central obesity), insulin resistance syndrome, Alzheimer, impaired consciousness such as coma, side effect or complication caused by anesthesia, sleep disturbance, sleep problem, insomnia, intermittent sleep, night myoclonus, REM sleep interruption, jet lag, jet lag syndrome, sleep disorder of shift workers, dyssomnia, sleep terror, depression, major depression, sleepwalking, enuresis, sleep disorder, Alzheimer's sundown syndrome, disease associated with circadian rhythm, fibromyalgia, condition resulting from decrease in sleeping quality, bulimia, obsessive eating disorder, obesity-related diseases, hypertension, diabetes, elevated plasma insulin level/insulin resistance, hyperlipemia, hyperlipidaemia, endometrial cancer, breast cancer, prostate cancer, colon cancer, cancer, osteoarthritis, obstructive sleep apnea, cholelithiasis, gallstone, heart disease, abnormal heartbeat, arrhythmia, myocardial infarction, congestive heart failure, coronary heart disease, cardiovascular disease, sudden death, polycystic ovary, craniopharyngioma, Prader-Willi syndrome, Froehlich syndrome, growth hormone deficiency, normal variant short stature, Turner syndrome, children suffering from acute lymphoblastic leukemia, syndrome X, reproductive hormone abnormality, decrease of fecundability, infertility, hypogonadism in men, sexual/reproductive-function dysfunction such as hirsutism in women, fetal defect associated with maternity obesity, gastrointestinal motility disorder such as obesity-related gastroesophageal reflux, obesity hypoventilation syndrome (Pickwickian syndrome), respiratory disease such as respiratory distress, inflammation such as vascular systemic inflammation, arteriosclerosis, hypercholesterolemia, hyperuricemia, low back pain, gallbladder disease, gout, renal cancer, secondary risk of obesity such as risk of left ventricle hypertrophy, migraine, headache, neuropathic pain, Parkinson's disease, psychosis, schizophrenia, facial flushing, night sweat, disease in genitalia/urinary system, disease associated with sexual function or fecundability, dysthymic disorder, bipolar disorder, bipolar I disorder, bipolar II disorder, cyclothymic disorder, acute stress disorder, agoraphobia, generalized anxiety disorder, obsessive-compulsive disorder, panic attack, panic disorder, posttraumatic stress disorder, separation anxiety disorder, social phobia, anxiety disorder, acute neurological and psychiatric disorder such as cerebral deficiency developed after heart bypass surgery or heart transplant, stroke, ischemic stroke, cerebral ischemia, spinal cord trauma, head injury, periparturient hypoxia, cardiac arrest, hypoglycemic nerve injury, Huntington's disease, amyotrophic lateral sclerosis, multiple sclerosis, eye damage, retinopathy, cognitive impairment, muscle spasm, tremor, epilepsy, disorder associated with muscle spasm, delirium, amnestic disorder, age-associated cognitive decline, schizoaffective disorder, paranoia, drug addiction, movement disorder, chronic fatigue syndrome, fatigue, medication-induced parkinsonian syndrome, Gilles de la Tourette syndrome, chorea, myoclonus, tic, restless legs syndrome, dystonia, dyskinesia, attention deficit hyperactivity disorder (ADHD), conduct disorder, urinary incontinence, withdrawal symptom, trigeminal neuralgia, hearing loss, tinnitus, nerve injury, retinopathy, macular degeneration, vomiting, cerebral edema, pain, bone pain, arthralgia, toothache, cataplexy, or traumatic brain injury, comprising the compound according to any one of Items 1 to 16 or a pharmaceutically acceptable salt thereof.

### [Item 21]

A medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, comprising the compound according to any one of Items 1 to 16 or a pharmaceutically acceptable salt thereof.

### [Item 22]

A medicament for treating narcolepsy, comprising the compound according to any one of Items 1 to 16 or a pharmaceutically acceptable salt thereof.

### [Item 23]

A medicament for treating idiopathic hypersomnia, comprising the compound according to any one of Items 1 to 16 or a pharmaceutically acceptable salt thereof.

### [Item 24]

A method of treating a disease associated with orexin receptor type 2, which comprises administering a therapeutically effective amount of the compound according to any one of Items 1 to 16 or a pharmaceutically acceptable salt thereof to a patient in need thereof.

### [Item 25]

A method of treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, which comprises administering a therapeutically effective amount of the compound according to any one of Items 1 to 16 or a pharmaceutically acceptable salt thereof to a patient in need thereof.

### [Item 26]

The compound according to any one of Items 1 to 16 or a pharmaceutically acceptable salt thereof to a patient in need thereof for use in the treatment and/or prophylaxis of a disease associated with orexin receptor type 2.

### [Item 27]

The compound according to any one of Items 1 to 16 or a pharmaceutically acceptable salt thereof for use in the treatment and/or prophylaxis of narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

### [Item 28]

Use of the compound according to any one of Items 1 to 16 or a pharmaceutically acceptable salt thereof, in the manufacture of a medicament for treating a disease associated with orexin receptor type 2.

### [Item 29]

Use of the compound according to any one of Items 1 to 16 or a pharmaceutically acceptable salt thereof, in the manufacture of a medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

### [Item 30]

A combination medicament of a medicament comprising the compound according to any one of Items 1 to 16 or a pharmaceutically acceptable salt thereof and one or more drugs selected from drugs classified as a medicament for treating a disease such as narcolepsy, idiopathic hypersomnia, hypersomnia, sleep apnea syndrome, narcolepsy syndrome involving narcolepsy-like symptom, or a medicament for treating Parkinson's disease or dementia with Lewy body.

### [Item 31]

A medicament comprising the compound according to any one of Items 1 to 16 or a pharmaceutically acceptable salt thereof for treating a disease associated with orexin receptor type 2 in combination with one or more drugs selected from drugs classified as a medicament for treating a disease such as narcolepsy, idiopathic hypersomnia, hypersomnia, sleep apnea syndrome, narcolepsy syndrome involving narcolepsy-like symptom, or a medicament for treating Parkinson's disease or dementia with Lewy body.

### DETAILED DESCRIPTION

Hereinafter, the present invention is explained in more detail. The number of carbon atoms in the definition of "substituent group" as used herein may be expressed as, for example, "C₁₋₆". Specifically, the expression "C₁₋₆ alkyl" means an alkyl group having 1 to 6 carbon atoms. As used herein, a substituent group which is not accompanied with the term "optionally-substituted" or "substituted" means an "unsubstituted" substituent group. For example, "C₁₋₆ alkyl" means "unsubstituted C₁₋₆ alkyl".

The substituent group as used herein may be expressed without the term "group". In case that "optionally-substituted" is used in the definition of substituent group, the number of the substituting group is not limited as long as the substitution is available, *i.e.,* it is one or more. It means that the possible number of substituting groups is the substitution-available number on carbon atoms or carbon and nitrogen atoms in a substituent group which are acceptable for substitution. Unless otherwise specified, the definition of each substituent group also extends over the case that the substituent group is partially included in another substituent group or the case that the substituent group is attached to another substituent group.

Unless otherwise specified, the binding site of substituent groups is not limited as long as the site is chemically available to be bound. Also, in the structural formula as used herein, wedge solid and dashed bonds mean absolute configuration, and thick solid and dashed bonds mean relative configuration.

The "halogen" includes, for example, fluorine, chlorine, bromine, iodine, and the like. Preferably, it is fluorine or chlorine.

The "C₁₋₄ alkyl" means a straight or branched chain saturated hydrocarbon group having 1 to 4 carbon atoms, and the "C₁₋₆ alkyl" means straight or branched chain saturated hydrocarbon group having 1 to 6 carbon atoms. Specific examples of the "C₁₋₄ alkyl" include methyl, ethyl, propyl, isopropyl, butyl, isobutyl, sec-butyl and *tert*-butyl, and specific examples of the "C₁₋₆ alkyl" include pentyl, isopentyl, neopentyl, 1-ethylpropyl, hexyl and a structural isomer thereof, besides the above C₁₋₄ alkyl. The "C₁₋₆ alkyl" or "C₁₋₄ alkyl" is preferably methyl, ethyl, propyl and isopropyl, more preferably methyl and isopropyl.

The "C₁₋₆ alkylene" means a divalent straight or branched chain saturated hydrocarbon group having 1 to 6 carbon atoms. The "C₁₋₄ alkylene" includes preferably "C₁₋₄ alkylene", more preferably "C₁₋₃ alkylene". Specific examples of the "C₁₋₄ alkylene" include methylene, ethylene, propylene, trimethylene, and the like. Specific examples of the "C₁₋₄ alkylene" include butylene, 1,1-dimethylethylene, 1,2-dimethylethylene, 1-methyltrimethylene, 2-methyltrimethylene, and the like, besides the specific examples listed in the above "C₁₋₄ alkylene". Specific examples of the "C₁₋₆ alkylene" include pentylene, 1,1-dimethyltrimethylene, 1,2-dimethyltrimethylene, 1-methylbutylene, 2-methylbutylene, 1-methylpentylene, 2-methylpentylene, 3-methylpentylene, hexylene, and the like, besides the specific examples listed in the above "C₁₋₄ alkylene".

The "C₃₋₇ cycloalkyl" means a non-aromatic cyclic hydrocarbon group (*i.e.,* saturated hydrocarbon group and partially-unsaturated hydrocarbon group) having 3 to 7 carbon atoms. Preferably, it is "C₃₋₆ cycloalkyl". The "C₃₋₇ cycloalkyl" also includes a bridged one. Specific examples of the "C₃₋₇ cycloalkyl" include cyclopropyl, cyclobutyl, cyclopentyl, cyclohexyl, cyclopentenyl, cyclohexenyl, cycloheptyl, and the like.

The "C₃₋₇ cycloalkyl" also includes a bi-cyclic condensed ring in which the "C₃₋₇ cycloalkyl" is fused with benzene ring or a 5- or 6-membered ring having one heteroatom selected from nitrogen, sulfur, or oxygen atom, or the same or different two or more (for example, 2 to 4) heteroatoms thereof (for example, 5- or 6-membered ring in "4- to 10-membered saturated heterocycle" mentioned below),

The "C₁₋₄ alkoxy" means oxy group substituted with the above "C₁₋₄ alkyl", and the "C₁₋₆ alkoxy" means oxy group substituted with the above "C₁₋₆, alkyl". Specific examples of the "C₁₋₄ alkoxy" include methoxy, ethoxy, propoxy, isopropoxy, butoxy, isobutoxy, *sec*-butoxy, *tert*-butoxy, and the like, and specific examples of the "C₁₋₆ alkoxy" include pentyloxy, hexyloxy, and the like. Preferably, the "C₁₋₄ alkoxy" or "C₁₋₆ alkoxy" includes methoxy, ethoxy and isopropoxy.

The "C₃₋₇ cycloalkoxy" means oxy group substituted with the above "C₃₋₇ cycloalkyl". Preferably, it is "C₃₋₆ cycloalkoxy". Specific examples of the "C₃₋₇ cycloalkoxy" include cyclopropyloxy, cyclobutyloxy, cyclopentyloxy, cyclohexyloxy, and the like. Preferably, it is cyclohexyloxy.

The "C₁₋₁₀ aromatic carbocyclyl group" means an aromatic hydrocarbon group having 6 to 10 carbon atoms, which is also referred to as "C₆₋₁₀ aryl". Specific examples of the "C₆₋₁₀ aromatic carbocyclyl group" includes phenyl, 1-naphthyl, 2-naphthyl, and the like. More preferably, it is phenyl.

The "C₆₋₁₀ aromatic carbocyclyl group" also includes a fused ring group in which "phenyl" is fused with a 5- or 6-membered ring having one heteroatom selected from nitrogen, sulfur or oxygen atom, or the same or different two or more (for example, 2 to 4) heteroatoms thereof (for example, "5- or 6-membered monocyclic aromatic heterocycle" mentioned below, and 5- or 6-membered ring in "4- to 10-membered saturated heterocycle" mentioned below), or a 5- to 7-membered cycloalkyl ring (for example, cyclopentane, cyclohexane or cycloheptane).

The "5- to 10-membered aromatic heterocyclyl group" means a mono- or polycyclic 5- to 10-membered aromatic group having one heteroatom selected from nitrogen, sulfur or oxygen atom, or the same or different two or more (for example, 2 to 4) heteroatoms thereof, besides carbon atoms as the ring atoms. Preferably, it is "5- or 6-membered monocyclic aromatic heterocyclyl group". The "5- or 6-membered monocyclic aromatic heterocyclyl group" means a monocyclic 5- or 6-membered aromatic heterocyclyl group within the "5- to 10-membered aromatic heterocyclyl group".

The polycyclic aromatic heterocyclyl group in the above "5- to 10-membered aromatic heterocyclyl group" includes, for example, a fused ring group in which the same or different two monocyclic aromatic heterocycles are fused, and a condensed ring group in which a monocyclic aromatic heterocycle and an aromatic ring (for example, benzene) or a non-aromatic ring (for example, cyclohexane) are fused.

Specific examples of the "5- to 10-membered aromatic heterocyclyl group" include pyrazolyl, imidazolyl, benzofuranyl, pyridyl, pyrimidinyl, pyrazinyl, pyridazinyl, and the like. In another embodiment, it preferably includes benzofuranyl in which the binding site is on the heteroaryl (furan) ring, pyridyl, pyrimidinyl, pyrazinyl and pyridazinyl.

The "C₃₋₆ saturated carbocyclic ring" means a monocyclic saturated or partially-unsaturated hydrocarbon ring having 3 to 6 carbon atoms. Specific examples of the "C₃₋₆ saturated carbocyclic ring" include cyclopropane, cyclobutane, cyclopentane, cyclohexane, cyclopropene, cyclobutene, cyclopentene, cyclohexene, cyclohexadiene, and the like. Preferably, it includes cyclopropane or cyclobutane.

The "4- to 10-membered saturated heterocycle" means a monocyclic or bicyclic saturated heterocycle composed of 4 to 10 atoms, which has the same or different one or more (for example, 2 to 4, preferably 2 to 3, more preferably 2) heteroatoms selected from the group consisting of oxygen atom, nitrogen atom and sulfur atom, besides carbon atoms as the ring atoms. The heterocycle includes a partially-unsaturated heterocycle, a partially-bridged heterocycle and a partially-spiro heterocycle. Preferably, the heterocycle is 5- or 6-membered saturated heterocycle. The bicyclic saturated heterocycle also includes a condensed ring in which a monocyclic saturated heterocycle and benzene or a monocyclic 5- to 6-membered aromatic heterocycle are fused. Also, the saturated heterocycle may further comprise one or two carbonyl, thiocarbonyl, sulfinyl or sulfonyl, that is, the saturated heterocycle includes, for example, a cyclic group such as lactam, thiolactam, lactone, thiolactone, cyclic imide, cyclic carbamate, and cyclic thiocarbamate, which the number of atoms composing 4- to 10-membered ring (*i.e.*, ring size) or the number of heteroatoms composing heterocycle does not count the oxygen atom in carbonyl, sulfinyl and sulfonyl, and the sulfur atom in thiocarbonyl. The "4- to 10-membered saturated heterocycle" includes preferably monocyclic or bicyclic "4- to 8-membered saturated heterocycle", more preferably monocyclic "4- to 6-membered saturated heterocycle", and furthermore preferably monocyclic "5- or 6-membered saturated heterocycle". Preferably, the heterocycle is 5- or 6-membered saturated heterocycle. The bicyclic saturated heterocycle also includes a condensed ring in which a monocyclic saturated heterocycle and benzene or a monocyclic 5- to 6-membered aromatic heterocycle are fused. Also, the saturated heterocycle may further comprise one or two carbonyl, thiocarbonyl, sulfinyl or sulfonyl, that is, the saturated heterocycle includes, for example, a cyclic group such as lactam, thiolactam, lactone, thiolactone, cyclic imide, cyclic carbamate, and cyclic thiocarbamate, which the number of atoms composing 4- to 10-membered ring (*i.e*., ring size) or the number of heteroatoms composing heterocycle does not count the oxygen atom in carbonyl, sulfinyl and sulfonyl, and the sulfur atom in thiocarbonyl. Specific examples of the "4- to 10-membered saturated heterocycle" include piperazine, oxetane, azetidine, pyrrolidine, pyrazolidine, imidazolidine, piperidine, morpholine, homopiperidine, thiomorpholine, dioxothiomorpholine, hexamethyleneimine, oxazolidine, thiazolidine, oxoimidazolidine, dioxoimidazolidine, oxooxazolidine, dioxooxazolidine, dioxothiazolidine, tetrahydrofuran, tetrahydropyran, tetrahydropyridine, and the like. Preferably, the 4- to 10-membered saturated heterocycle is pyrrolidine, piperidine, piperazine, and morpholine. Specific examples of the bicyclic saturated heterocycle include indoline, isoindoline, dihydropurine, dihydrothiazolopyrimidine, dihydrobenzodioxane, dihydroindazole, dihydropyrrolopyridine, tetrahydroquinoline, decahydroquinoline, tetrahydroisoquinoline, decahydroisoquinoline, tetrahydronapthyridine, tetrahydropyridoazepine, and the like.

The "4- to 6-membered saturated heterocyclyl group" means a monovalent substituent derived from "4- to 6-membered saturated heterocycle" which belongs to the above "4- to 10-membered saturated heterocycle". Preferably, it includes azetidinyl, pyrrolidinyl, piperidinyl, piperazinyl, morpholinyl, oxetanyl, tetrahydrofuranyl, tetrahydropyranyl, and the like.

The present compound includes various hydrates, solvates, and crystal polymorphs thereof.

The present compound may include one or more isotope atoms such as D, ³H, ¹¹C, ¹³C, ¹⁴C, ¹³N, ¹⁵N, ¹⁵O, ³⁵S, ¹⁸F and ¹²⁵I by substitution, and such isotope-substituted compound is also included in the present compound.

The present compound also includes all tautomers, all existing stereoisomers, and mixtures thereof.

For example, optically-active products of the present compound can be prepared from an optically-active starting material or by the optical resolution of the racemate of a final product. The optical resolution method includes physical separation methods with optically-active column, and chemical separation methods such as a fractional crystallization method.

The "pharmaceutically acceptable salt" as used herein means a pharmaceutically usable acid addition salt and a pharmaceutically usable base addition salt. Examples of the "pharmaceutically acceptable salt" include an acid addition salt such as acetate, propionate, butyrate, formate, trifluoroacetate, maleate, fumarate, tartrate, citrate, stearate, succinate, ethylsuccinate, malonate, lactobionate, gluconate, glucoheptonate, benzoate, methanesulfonate, benzenesulfonate, p-toluenesulfonate (tosylate), laurylsulfate, malate, ascorbate, mandelate, saccharate, xinafoate, pamoate, cinnamate, adipate, cysteine, N-acetylcysteine, hydrochloride, hydrobromide, phosphate, sulfate, hydroiodide, nicotinate, oxalate, picrate, thiocyanate, undecanoate, polyacrylate and carboxy vinyl polymer; an inorganic base addition salt such as lithium salt, sodium salt, potassium salt and calcium salt; an organic base addition salt such as morpholine and piperidine; and an amino acid addition salt such as aspartate and glutamate, but are not limited thereto.

The present compound may be orally or parenterally administered directly or as a suitable formulation such as drug product, medicament, and pharmaceutical composition. Specific examples of the formulation thereof may include tablet, capsule, powder, granule, liquid, suspension, injection, patch and gel patch, but are not limited thereto. The formulation can be prepared with pharmaceutically acceptable additives in known means.

The additives may be chosen for any purpose, including an excipient, a disintegrant, a binder, a fluidizer, a lubricant, a coating agent, a solubilizer, a solubilizing agent, a thickener, a dispersant, a stabilizing agent, a sweetening agent, a flavor, and the like. Specific examples thereof include lactose, mannitol, microcrystalline cellulose, low-substituted hydroxypropylcellulose, corn starch, partially-pregelatinized starch, carmellose calcium, croscarmellose sodium, hydroxypropylcellulose, hydroxypropyl methylcellulose, polyvinyl alcohol, magnesium stearate, sodium stearyl fumarate, polyethylene glycol, propylene glycol, titanium oxide, talc, and the like.

The dose of the present compound should be suitably determined depending on subject animal for administration, administration route, target disease, and age, body weight, and condition of patients. For example, in the case of oral administration, about 0.01 mg as minimum to 10000 mg as maximum may be administered a day for an adult in one to several portions.

The present compound is a compound with agonist activity on orexin receptor type 2. Hence, the present compound may be a medicament useful for treating or preventing a disease associated with orexin receptor type 2. Specific examples of the disease include narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body, hypersomnia syndrome involving daytime hypersomnia (for example, Kleine-Levin syndrome, major depression accompanied by hypersomnia, dementia with Lewy body, Parkinson's disease, progressive supranuclear palsy, Prader-Willi syndrome, Moebius syndrome, hypoventilation syndrome, Niemann-Pick disease type C, brain contusion, cerebral infarction, brain tumor, muscular dystrophy, multiple sclerosis, acute disseminated encephalomyelitis, Guillain-Barre syndrome, Rasmussen's encephalitis, Wernicke's encephalopathy, limbic encephalitis, Hashimoto encephalopathy), coma, loss of consciousness, obesity (for example, malignant mast cell, extrinsic obesity, hyperinsulinar obesity, hyperplasmic obesity, hypophysial obesity, hypoplasmic obesity, hypothyroid obesity, hypothalamic obesity, symptomatic obesity, childhood obesity, upper body obesity, alimentary obesity, gonadal obesity, systemic mastocytosis, primary obesity, central obesity), insulin resistance syndrome, Alzheimer, impaired consciousness such as coma, side effect or complication caused by anesthesia, sleep disturbance, sleep problem, insomnia, intermittent sleep, night myoclonus, REM sleep interruption, jet lag, jet lag syndrome, sleep disorder of shift workers, dyssomnia, sleep terror, depression, major depression, sleepwalking, enuresis, sleep disorder, Alzheimer's sundown syndrome, disease associated with circadian rhythm, fibromyalgia, condition resulting from decrease in sleeping quality, bulimia, obsessive eating disorder, obesity-related diseases, hypertension, diabetes, elevated plasma insulin level/insulin resistance, hyperlipemia, hyperlipidaemia, endometrial cancer, breast cancer, prostate cancer, colon cancer, cancer, osteoarthritis, obstructive sleep apnea, cholelithiasis, gallstone, heart disease, abnormal heartbeat, arrhythmia, myocardial infarction, congestive heart failure, coronary heart disease, cardiovascular disease, sudden death, polycystic ovary, craniopharyngioma, Prader-Willi syndrome, Froehlich syndrome, growth hormone deficiency, normal variant short stature, Turner syndrome, children suffering from acute lymphoblastic leukemia, syndrome X, reproductive hormone abnormality, decrease of fecundability, infertility, hypogonadism in men, sexual/reproductive-function dysfunction such as hirsutism in women, fetal defect associated with maternity obesity, gastrointestinal motility disorder such as obesity-related gastroesophageal reflux, obesity hypoventilation syndrome (Pickwickian syndrome), respiratory disease such as respiratory distress, inflammation such as vascular systemic inflammation, arteriosclerosis, hypercholesterolemia, hyperuricemia, low back pain, gallbladder disease, gout, renal cancer, secondary risk of obesity such as risk of left ventricle hypertrophy, migraine, headache, neuropathic pain, Parkinson's disease, psychosis, schizophrenia, facial flushing, night sweat, disease in genitalia/urinary system, disease associated with sexual function or fecundability, dysthymic disorder, bipolar disorder, bipolar I disorder, bipolar II disorder, cyclothymic disorder, acute stress disorder, agoraphobia, generalized anxiety disorder, obsessive-compulsive disorder, panic attack, panic disorder, posttraumatic stress disorder, separation anxiety disorder, social phobia, anxiety disorder, acute neurological and psychiatric disorder such as cerebral deficiency developed after heart bypass surgery or heart transplant, stroke, ischemic stroke, cerebral ischemia, spinal cord trauma, head injury, periparturient hypoxia, cardiac arrest, hypoglycemic nerve injury, Huntington's disease, amyotrophic lateral sclerosis, multiple sclerosis, eye damage, retinopathy, cognitive impairment, muscle spasm, tremor, epilepsy, disorder associated with muscle spasm, delirium, amnestic disorder, age-associated cognitive decline, schizoaffective disorder, paranoia, drug addiction, movement disorder, chronic fatigue syndrome, fatigue, medication-induced parkinsonian syndrome, Gilles de la Tourette syndrome, chorea, myoclonus, tic, restless legs syndrome, dystonia, dyskinesia, attention deficit hyperactivity disorder (ADHD), conduct disorder, urinary incontinence, withdrawal symptom, trigeminal neuralgia, hearing loss, tinnitus, nerve injury, retinopathy, macular degeneration, vomiting, cerebral edema, pain, bone pain, arthralgia, toothache, cataplexy, or traumatic brain injury. Preferably, the disease includes narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body.

The present compound is a compound with agonist activity on orexin receptor type 2. Hence, the present compound may be a medicament useful for treating or preventing a disease associated with orexin receptor type 2. The present compound is particularly effective in sleep-wake disorders. Specific examples of the disorders include insomnia disorder, hypersomnolence disorder, narcolepsy, narcolepsy without cataplexy but with orexin (hypocretin) deficiency, narcolepsy with cataplexy but without orexin (hypocretin) deficiency, autosomal dominant cerebellar ataxia, deafness, and narcolepsy, autosomal dominant narcolepsy, obesity, and type 2 diabetes, narcolepsy secondary to another medical condition, breathing-related sleep disorders, circadian rhythm sleep-wake disorders, parasomnias, restless legs syndrome (restless legs syndrome), substance/medication-induced sleep disorder, other specified insomnia disorder, unspecified insomnia disorder, other specified hypersomnolence disorder, unspecified hypersomnolence disorder, other specified sleep-wake disorder, unspecified sleep-wake disorder.

The "prophylaxis" as used herein means the administration of the present compound to a healthy subject who does not develop a disease. For example, the prophylaxis is intended to prevent the development of a disease. The "treatment" as used herein means the administration of the present compound to a person diagnosed with the development of a disease by a doctor (*i.e.,* a patient). For example, the treatment is intended to alleviate a disease or symptom thereof or improve the condition of a patient to the previous condition before a disease is developed. Also, even if the present compound is administered for the purpose of preventing the worsening of a disease or symptom thereof, the administration is referred to as "treatment" when the subject to be administered is a patient.

The present compound may be used in combination with a medicament for treating a disease such as narcolepsy, idiopathic hypersomnia, hypersomnia, sleep apnea syndrome, narcolepsy syndrome involving narcolepsy-like symptom, or a medicament for treating Parkinson's disease or dementia with Lewy body as a combined medicine. Examples of the medicament for treating a disease such as narcolepsy, idiopathic hypersomnia, hypersomnia, sleep apnea syndrome, narcolepsy syndrome involving narcolepsy-like symptom include methylphenidate, modafinil, armodafinil, sodium oxybate, pitolisant, solriamfetol, and the like. Examples of the medicament for treating Parkinson's disease or dementia with Lewy body include amphetamine include levodopa, carbidopa, entacapone MAOB inhibitors (for example, zonisamide, selegiline, rasagiline, safmamide, and the like), dopamine receptor agonists (for example, bromocriptine, pergolide, talipexole, cabergoline, pramipexole, ropinirole, rotigotine, apomorphine, and the like), amantadine, droxidopa, istradefylline, anticholinergic agents (for example, trihexyphenidyl, biperiden, and the like), and the like.

The administration timing of the present compound and the combined medicine is not necessarily limited, and they may be administered simultaneously or administered with time-interval to a subject. In addition, the present compound and the combined medicine may be used in the form of a combination drug. The dosage of the combined medicine may be optionally selected based on the dosage in the clinical use. Also, the mixing ratio of the present compound and the combined medicine may be optionally determined depending on the subject to be administered, the administration route, the disease to be treated, the symptom, and a combination thereof.

Hereinafter, the processes for preparing the compound of formula (1) of the present invention are exemplified along with examples, but the processes of the present invention should not be limited to the examples.

### Preparation Process

The present compound may be synthesized according to each Preparation Process shown below or its combination with a known synthetic process.

Each compound in the following schemes may exist as a salt thereof, wherein the salt includes, for example, the "pharmaceutically acceptable salt" mentioned above. The following schemes are disclosed as just examples, and thus it is also possible to optionally prepare the present compound by a different process based on the knowledge of a skilled person in synthetic organic chemistry field.

In each Preparation Process described below, protecting groups can be used as necessary, even if the use of protecting groups is not explicitly stated. And, the protecting groups can be deprotected after a reaction is completed or a series of reactions have been carried out to obtain the desired compound.

As such protecting groups, for example, general protecting groups described in T. W. Greene, and P. G. M. Wuts, "Protective Groups in Organic Synthesis ", 3rd Ed., John Wiley & Sons, Inc., New York (1999), and the like may be used. Examples of amino-protecting group include *tert*-butoxycarbonyl, benzyloxycarbonyl, p-toluenesulfonyl, o-nitrobenzenesulfonyl, tetrahydropyranyl, and the like; examples of hydroxy-protecting group include trialkylsilyl, acetyl, benzyl, tetrahydropyranyl, methoxymethyl, and the like; examples of aldehyde-protecting group include dialkylacetal, cyclic alkylacetal, and the like; and examples of carboxyl-protecting group include *tert*-butyl ester, orthoester, amide, and the like.

The introduction and elimination of protecting groups can be carried out by a method commonly used in synthetic organic chemistry (for example, *see* T. W. Greene, and P. G. M. Wuts, "Protective Groups in Organic Synthesis ", 3rd Ed., John Wiley & Sons, Inc., New York (1999*)*), or a similar method.

### Preparation Process 1:

A compound of formula (1) or a pharmaceutically acceptable salt thereof can be prepared, for example, by the following process: 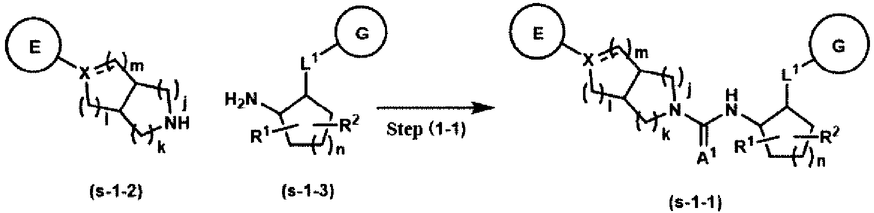 wherein R¹, R², L¹, j, k, l, m, n, ring G, A¹, dashed bond, X, and ring E are as defined in Item 1.

### Step (1-1):

Compound (s-1-1) can be prepared by reacting compound (s-1-2) with compound (s-1-3) in a suitable inert solvent under a commonly-used reaction condition of urea-binding formulation. The present reaction condition includes, for example, the reaction condition using triphosgene, 4-nitrophenyl chloroformate, 1,1'-carbonyldiimidazole or thiophosgene. A base is used in the present reaction, and the base used herein includes triethylamine, diisopropylethylamine, and the like. The inert solvent includes a halogenated carbon solvent such as chloroform and dichloromethane; an ether solvent such as diethyl ether, THF and 1,4-dioxane; an aromatic hydrocarbon solvent such as benzene, toluene and xylene; and an ester solvent such as ethyl acetate and methyl acetate. The reaction time is generally about 1 hour to 24 hours, and the reaction temperature is -20°C to boiling point of a solvent used herein.

In Step (1-1), an intermediate such as isocyanate compound may be isolated for subsequent conversion.

### Preparation Process 2:

In the compound of formula (s-1-2), a compound of formula (s-2-1) can be prepared, for example, by the following process: 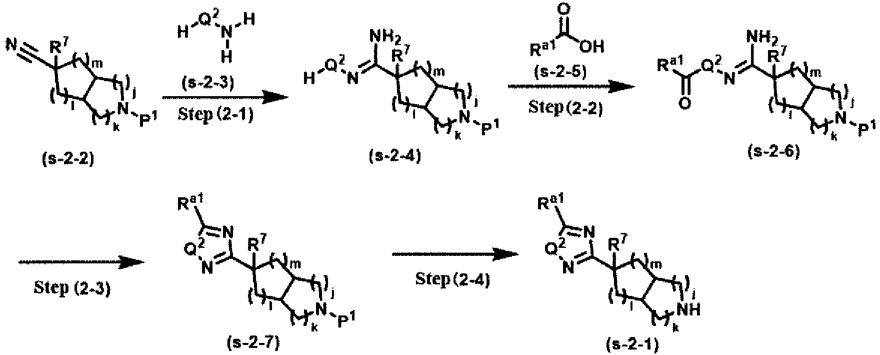 wherein R⁷, R^{a1}, Q², j, k, l and m are as defined in Item l, and P¹ is a suitable protecting group and is applied even when it is not explained below.

### Step (2-1):

Compound (s-2-4) can be prepared by reacting compound (s-2-2) with compound (s-2-3) in a suitable inert solvent under a commonly-used addition reaction condition. The inert solvent includes a halogenated carbon solvent such as chloroform and dichloromethane; an ether solvent such as diethyl ether, THF and 1,4-dioxane; an aromatic hydrocarbon solvent such as benzene, toluene and xylene; an alcohol solvent such as methanol and ethanol; and water. The reaction time is generally about 1 hour to 24 hours, and the reaction temperature is -20°C to boiling point of a solvent used herein.

### Step (2-2):

Compound (s-2-6) can be prepared by reacting compound (s-2-4) with compound (s-2-5) in a suitable inert solvent under a commonly-used condensation reaction condition. The present reaction condition includes, for example, the condition using HATU, DCC or CDI. A base may be used in the present reaction, and the base used herein includes triethylamine, diisopropylethylamine, and the like. The solvent includes, for example, a halogenated carbon solvent such as chloroform and dichloromethane; an ether solvent such as diethyl ether, THF and 1,4-dioxane; an aromatic hydrocarbon solvent such as benzene, toluene and xylene; and an ester solvent such as ethyl acetate and methyl acetate. The reaction time is generally about 1 hour to 24 hours, and the reaction temperature is -20°C to boiling point of a solvent used herein.

### Step (2-3):

Compound (s-2-7) can be prepared by reacting compound (s-2-6) in a suitable inert solvent under a commonly-used dehydration reaction condition. In the present reaction, a base such as triethylamine and DBU may be used. The inert solvent includes, for example, a halogenated carbon solvent such as chloroform and dichloromethane; an ether solvent such as diethyl ether, THF and 1,4-dioxane; an aromatic hydrocarbon solvent such as benzene, toluene and xylene; and an ester solvent such as ethyl acetate and methyl acetate. The reaction time is generally about 1 hour to 24 hours, and the reaction temperature is -20°C to boiling point of a solvent used herein.

### Step (2-4):

Compound (s-2-1) is prepared by eliminating the protecting group in compound (s-2-7) in the same manner as a known method, for example, the method described in Protective Groups in Organic Synthesis 3rd Edition (John Wiley & Sons, Inc.), Comprehensive Organic Transformation, R. C. Larock, VCH publisher Inc., 1989, and the like.

### Preparation Process 3:

In the compound of formula (s-1-2), a compound of formula (s-3-1) can be prepared, for example, by the following process: 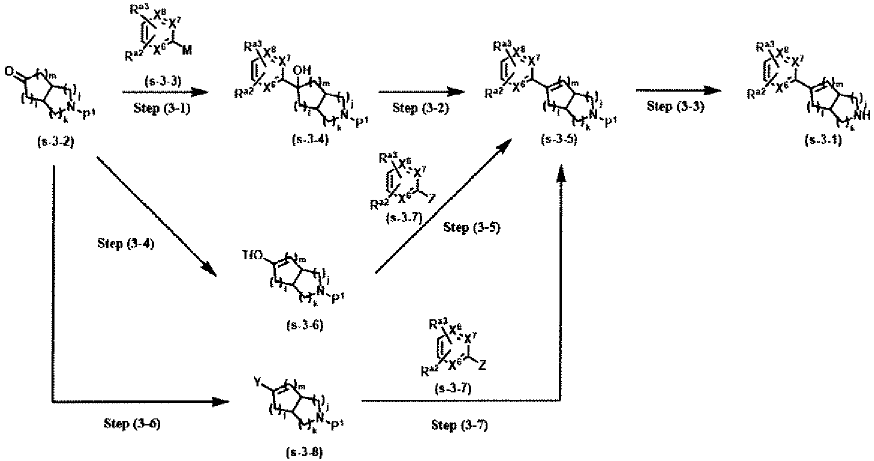 wherein R^{a2}, R^{a3}, X⁶, X⁷, X⁸, j, k, l and m are as defined in Item 1, M is a metal such as lithium and magnesium halide, Z is boronic acid, boronate ester, BF₃K, BF₃Na, trialkyl tin, zinc halide or hydrogen atom, and Y is halogen.

Compound (s-3-1) can be synthesized from compound (s-3-2) via Step (3-1) to Step (3-3).

### Step (3-1):

Compound (s-3-4) can be prepared by reacting compound (s-3-2) with compound (s-3-3) in a suitable inert solvent under a commonly-used reaction condition of addition to ketones in the absence of an additive or in the presence of a Lewis acid. The Lewis acid used herein includes, for example, zinc (II) chloride, scandium (III) triflate, copper (I) chloride, boron trifluoride, boronic acid, boronate ester, and the like. The inert solvent includes, for example, a halogenated carbon solvent such as chloroform and dichloromethane; an ether solvent such as diethyl ether, THF and 1,4-dioxane; and an aromatic hydrocarbon solvent such as benzene, toluene and xylene. The reaction time is generally about 1 hour to 24 hours, and the reaction temperature is -78°C to boiling point of a solvent used herein.

### Step (3-2):

Compound (s-3-5) can be prepared by reacting compound (s-3-4) in a suitable inert solvent under a commonly-used dehydration reaction condition. The dehydration reaction condition is the condition using an acid and a dehydrating agent such as Burgess reagent and the condition such as Mitsunobu reaction condition, or the condition in which hydroxyl group in the compound is converted to a leaving group (via methanesulfonylation, p-toluenesulfonylation or halogen substitution) and then the leaving group is eliminated using a reagent such as a base. The acid used herein includes, for example, a protic acid such as hydrochloric acid, sulfuric acid, phosphoric acid, methanesulfonic acid, benzenesulfonic acid and p-toluenesulfonic acid; and a Lewis acid such as zinc (II) chloride, scandium (III) triflate, copper (I) chloride, boron trifluoride, boronic acid and boronate ester. Also, the base used herein includes, for example, an organic base such as triethylamine, diisopropylethylamine and DBU; an inorganic base such as sodium hydrogen carbonate, sodium carbonate and potassium carbonate; a metal alkoxide such as potassium *tert*-butoxide; an organometallic reagent such as n-butyllithium and isopropylmagnesium chloride; and a metal amide reagent such as LDA and LHMDS. The inert solvent includes, for example, a halogenated carbon solvent such as chloroform and dichloromethane; an ether solvent such as diethyl ether, THF and 1,4-dioxane; an aromatic hydrocarbon solvent such as benzene, toluene and xylene; and an ester solvent such as ethyl acetate and methyl acetate. The reaction time is generally about 1 hour to 24 hours, and the reaction temperature is -78°C to boiling point of a solvent used herein.

### Step (3-3):

Compound (s-3-1) can be prepared from compound (s-3-5) in the same condition as Step (2-4).

Compound (s-3-5) can also be synthesized from compound (s-3-2) via Step (3-4) and Step (3-5).

### Step (3-4):

Compound (s-3-6) can be prepared by reacting a commonly-used inflating agent with compound (s-3-2) in a suitable inert solvent in the absence of an additive or in the presence of an acid or a base (or in the presence of both an acid and a base). The triflating agent used herein includes, for example, trifluoromethanesulfonic anhydride, N-phenylbis(trifluoromethanesulfonimide), and the like. The acid used herein includes, for example, a Lewis acid such as zinc (II) chloride, scandium (III) triflate, copper (I) chloride, boron trifluoride, boronic acid and boronate ester, and the like. Also, the base used herein includes, for example, an organic base such as triethylamine, diisopropylethylamine and DBU; an inorganic base such as sodium hydrogen carbonate, sodium carbonate and potassium carbonate; a metal alkoxide such as potassium *tert-*butoxide; an organometallic reagent such as n-butyllithium and isopropylmagnesium chloride; and a metal amide reagent such as LDA and LHMDS. The inert solvent includes, for example, a halogenated carbon solvent such as chloroform and dichloromethane; an ether solvent such as diethyl ether, THF and 1,4-dioxane; an aromatic hydrocarbon solvent such as benzene, toluene and xylene; and an ester solvent such as ethyl acetate and methyl acetate. The reaction time is generally about 1 hour to 24 hours, and the reaction temperature is -78°C to boiling point of a solvent used herein.

### Step (3-5):

Compound (s-3-5) can be prepared by reacting compound (s-3-6) with compound (s-3-7) wherein Z is boronic acid, boronate ester, BF₃K, BF₃Na, trialkyl tin or zinc halide in a suitable inert solvent in the presence of palladium catalyst and phosphine ligand, and, as necessary, a base. Compound (s-3-7) may be purchased as a commercial product or synthesized according to a known method or a similar method thereto. The palladium catalyst includes, for example, tetrakis(triphenylphosphine)palladium (0), bis(dibenzylideneacetone)palladium (0), tris(dibenzylideneacetone)dipalladium (0), bis(tri-*tert*-butylphosphine)palladium (0), palladium acetate (0), [1,1'-bis(diphenylphosphino)ferrocene]palladium (II) dichloride, bis(di-*tert*-butyl(4-dimethylaminophenyl)phosphine)dichloropalladium (II), and the like.

The phosphine ligand includes, for example, o-tolylphosphine, 2-dicyclohexylphosphino-2',6'-dimethoxybiphenyl (S-Phos), 2-dicyclohexylphosphino-2',4',6'-triisopropylbiphenyl (X-Phos), 1,1'-bis(diphenylphosphino)ferrocene (DPPF), 1,2-bis(diphenylphosphino)ethane (DPPE), 1,3-bis(diphenylphosphino)propane (DPPP), 1,4-bis(diphenylphosphino)butane (DPPB), 2,2'-bis(diphenylphosphino)-1,1'-binaphthyl (BINAP), 4,5-bis(diphenylphosphino)-9,9-dimethylxanthene (XANT-Phos), bis[2-(diphenylphosphino)phenyl]ether (DPE-Phos), and the like. The base includes, for example, sodium carbonate, potassium carbonate, cesium carbonate, potassium phosphate, sodium hydroxide, potassium hydroxide, and the like. The inert solvent includes, for example, 1,4-dioxane, THF, 1,2-dimethoxyethane, acetonitrile, water and a mixture thereof. The reaction time is generally about 1 hour to 24 hours, and the reaction temperature is -20°C to boiling point of a solvent used herein.

Compound (s-3-5) can also be synthesized from compound (s-3-2) via Step (3-6) and Step (3-7).

### Step (3-6):

Compound (s-3-8) can be prepared by reacting the compound in a suitable inert solvent under a commonly-used reaction condition of vinyl halide formation. The reaction condition of vinyl halide formation used herein includes, for example, the condition for reacting with hydrazine and then reacting with tetramethylguanidine and iodine, the condition for reacting with phosphorous acid and bromine in the presence of triethylamine, or the condition for reacting with a chlorinating reagent such as phosphorus pentachloride and phosphorus trichloride. The inert solvent includes, for example, a halogenated carbon solvent such as chloroform and dichloromethane; an ether solvent such as diethyl ether, THF and 1,4-dioxane; an aromatic hydrocarbon solvent such as benzene, toluene and xylene; and an ester solvent such as ethyl acetate and methyl acetate. The reaction time is generally about 1 hour to 24 hours, and the reaction temperature is - 78°C to boiling point of a solvent used herein.

### Step (3-7):

Compound (s-3-5) can be prepared from compound (s-3-8) in the same condition as Step (3-5).

### Preparation Process 4:

In the compound of formula (s-1-2), a compound of formula (s-4-1) can be prepared, for example, by the following process: 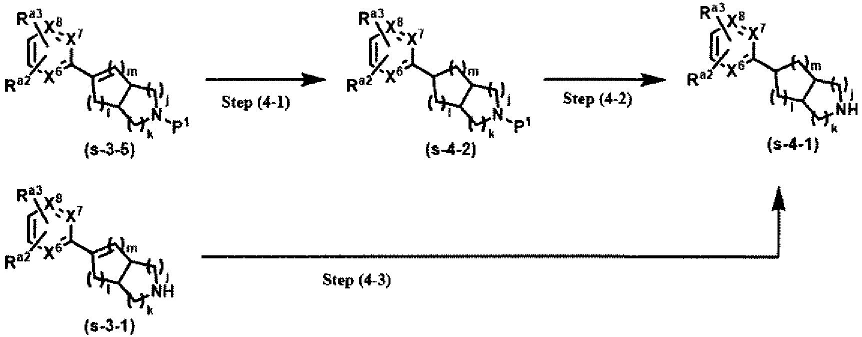 wherein R^{a2}, R^{a3}, X⁶, X⁷, X⁸, j, k, l and m are as defined in Item 1.

Compound (s-4-1) can be synthesized from compound (s-3-5) via Step (4-1) and Step (4-2) or from compound (s-3-1) via Step (4-3).

### Step (4-1):

Compound (s-4-2) can be prepared by reacting compound (s-3-5) in a suitable inert solvent and, as necessary, under hydrogen atmosphere in the absence of an additive or in the presence of an acid under a commonly-used reduction reaction condition of double bonds. The present reaction condition includes, for example, the condition using palladium carbon, palladium (II) hydroxide or Raney nickel and the condition using a hydride reducing agent such as triethylsilane. The acid used herein includes, for example, a protic acid such as hydrochloric acid, sulfuric acid, phosphoric acid, methanesulfonic acid, benzenesulfonic acid and p-toluenesulfonic acid. The inert solvent includes, for example, a halogenated carbon solvent such as chloroform and dichloromethane; an ether solvent such as diethyl ether, THF and 1,4-dioxane; an aromatic hydrocarbon solvent such as benzene, toluene and xylene; and an alcohol solvent such as methanol and ethanol. The reaction time is generally about 1 hour to 24 hours, and the reaction temperature is -20°C to boiling point of a solvent used herein.

### Step (4-2):

Compound (s-4-1) can be prepared from compound (s-4-2) in the same condition as Step (2-4).

### Step (4-3):

Compound (s-4-1) can be prepared from compound (s-3-1) in the same condition as Step (4-1).

### Preparation Process 5:

In the compound of formula (s-1-2), a compound of formula (s-5-1) can be prepared, for example, by the following process: 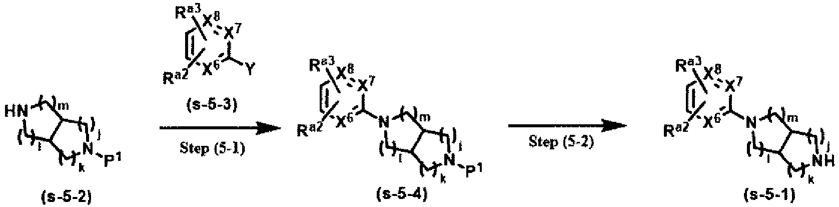 wherein R^{a2}, R^{a3}, X⁶, X⁷, X⁸, j, k, l and m are as defined in Item 1, and Y is halogen.

Compound (s-5-1) can be synthesized from compound (s-5-2) via Step (5-1) and Step (5-2).

### Step (5-1):

Compound (s-5-4) is prepared by reacting compound (s-5-2) with compound (s-5-3) in a suitable inert solvent in the presence of palladium catalyst, phosphine ligand and a base. Compound (s-5-3) may be purchased as a commercial product or synthesized according to a known method or a similar method thereto. The palladium catalyst includes, for example, tetrakis(triphenylphosphine)palladium (0), bis(dibenzylideneacetone)palladium (0), tris(dibenzylideneacetone)dipalladium (0), bis(tri-*tert*-butylphosphine)palladium (0), palladium acetate (0), [1,1'-bis(diphenylphosphino)ferrocene]palladium (II) dichloride, bis(di-*tert*-butyl(4-dimethylaminophenyl)phosphine)dichloropalladium (II), and the like.

The phosphine ligand includes, for example, o-tolylphosphine, 2-dicyclohexylphosphino-2',6'-dimethoxybiphenyl (S-Phos), 2-dicyclohexylphosphino-2',4',6'-triisopropylbiphenyl (X-Phos), 1,1'-bis(diphenylphosphino)ferrocene (DPPF), 1,2-bis(diphenylphosphino)ethane (DPPE), 1,3-bis(diphenylphosphino)propane (DPPP), 1,4-bis(diphenylphosphino)butane (DPPB), 2,2'-bis(diphenylphosphino)-1,1'-binaphthyl (BINAP), 4,5-bis(diphenylphosphino)-9,9-dimethylxanthene (XANT-Phos), bis[2-(diphenylphosphino)phenyl]ether (DPE-Phos), and the like. The base includes, for example, sodium carbonate, potassium carbonate, cesium carbonate, potassium phosphate, sodium hydroxide, potassium hydroxide, and the like. The inert solvent includes, for example, 1,4-dioxane, THF, 1,2-dimethoxyethane, acetonitrile, water and a mixture thereof. The reaction time is generally about 1 hour to 24 hours, and the reaction temperature is -20°C to boiling point of a solvent used herein.

In the case that in Step (5-1), compound (s-5-3) undergoes nucleophilic aromatic substitution reaction, compound (s-5-4) can also be synthesized by reacting compound (s-5-2) and compound (s-5-3) under the reaction condition.

### Step (5-2):

Compound (s-5-1) can be prepared from compound (s-5-4) in the same condition as Step (2-4).

### EXAMPLES

The present invention is explained in more detail in the following by referring to Reference Examples, Examples and Test Examples, but is not limited thereto. It should be understood that the names of compounds used in the following Reference Examples and Examples do not necessarily follow the IUPAC nomenclature.

In the present specification, the abbreviations shown below may be used.
n-: normal-
tert-: tertiary-
p-: para-
CDCl₃: deuterochloroform
Rt: retention time
min: minute
HATU: O-(7-aza-1H-benzotriazol-1-yl)-N,N,N',N'-tetramethyluronium hexafluorophosphate
DCC: N,N'-dicyclohexylcarbodiimide
CDI: carbonyldiimidazole
THF: tetrahydrofuran
TFA: trifluoroacetic acid
DMSO: dimethyl sulfoxide
Boc: *tert*-butoxycarbonyl
DBU: diazabicycloundecene
LDA: lithium diisopropylamide
LHMDS: lithium bis(trimethylsilyl)amide

In the column chromatography and amino chromatography used in Reference Examples and Examples, silica gel column and amino column made by YAMAZEN CORPORATION were used. The TLC (silica gel plate) used in the TLC purification was Silica gel 60F254 (Merck), and the TLC (NH silica gel plate) used therein was TLC plate NH (FujiSilysia).

In Reference Examples and Examples, the reactors shown below were used. The physicochemical data described in Reference Examples and Examples were obtained with the apparatuses below.

Microwave reactor: Biotage AB Initiator
¹H-NMR: JEOL JNM-AL400; JEOL JNM-ECS400 ; Brucker AVANCE 400 Spectrometer

The symbols used in NMR are defined as follows, s: singlet, d: doublet, dd: doublet of doublets, ddd: doublet of doublets of doublets, dddd: doublet of doublets of doublets of doublets, t: triplet, td: triplet of doublets, q: quartet, m: multiplet, br: broad singlet or multiplet, and J: coupling constant.

The LC/MS data of each compound in Examples and Reference Examples were obtained with any one of the apparatuses below.

### Method A

Detection apparatus: ACQUITY^{™} SQ detector (Waters Corporation)
HPLC: ACQUITY UPLC^{™} SYSTEM
Column: Waters ACQUITY UPLC^{™} BEH C18 (1.7 µm, 2.1 mm × 30 mm)

### Method B

Detection apparatus: Shimadzu LCMS-2020
Column: Phenomenex Kinetex (C18, 1.7 µm, 2.1 mm x 50 mm)

High-performance liquid chromatograph mass spectrometer; the measurement conditions of LC/MS are as follows, wherein the observed value [MS (m/z)] is denoted by [M+H]⁺ and the retention time is denoted by Rt (min).

### Method A

Solvent: A solution; 0.06% formic acid/H₂O, B solution; 0.06% formic acid/acetonitrile
Gradient condition: 0.0-1.3 min (linear gradient from B 2% to B 96%)
Flow rate: 0.8 mL/min; Detective UV: 220 nm and 254 nm; Temperature: 40°C

Hereinafter, the LC-MS data shown below were measured by Method A, unless otherwise indicated.

### Method B

Solvent: A solution; 0.05% TFA/H₂O, B solution; acetonitrile
Gradient condition: 0.0-1.7 min (linear gradient from B 10% to B 99%), 1.7-1.9 min (B 99%), 1.9-3.0 min (B 10%)
Flow rate: 0.5 mL/min; Detective UV: 254 nm; Temperature: 40°C

### Example 1:

### (3aR,5S,6aS)-5-(5-Cyclopropyl-1,2,4-oxadiazol-3-yl)-N-{(1R,6S)-2,2-difluoro-6-[4-(propan-2-yl)piperazin-1-yl]cyclohexy}hexahydropenta[c]pyrrole-2(1H)-carboxamide

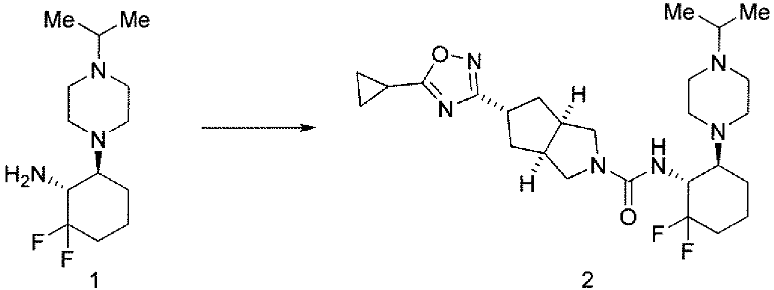

To a solution of triphosgene (38.6 mg) in chloroform (0.4 mL) were added a solution of literature-known compound 1 (100 mg) (Starting material A) in chloroform (1.4 mL) and a solution of triethylamine (160 µL) in chloroform (0.4 mL) at -20°C and the mixture was stirred at the temperature for 40 minutes. The reaction mixture was added to a solution of the compound of Reference Example 4 (92 mg) (Starting material B) and triethylamine (107 µL) in chloroform (0.5 mL) at 0°C and the mixture was stirred at room temperature for 1 hour. To the reaction mixture was added water and the mixture was extracted with chloroform. The organic layer was dried over anhydrous sodium sulfate and concentrated *in vacuo.* The resulting residue was then purified by silica gel column chromatography (Eluate: chloroform/methanol) to give the title compound 2 (168 mg).
¹H-NMR (CDCl₃) δ: 1.01 (6H, d, J = 6.0 Hz), 1.17-1.23 (4H, m), 1.23-1.47 (2H, m), 1.60-1.87 (2H, m), 1.89-2.02 (3H, m), 2.10-2.24 (4H, m), 2.33-2.52 (7H, m), 2.69 (1H, sep, J = 6.4 Hz), 2.69-2.81 (2H, m), 2.87-3.00 (2H, m), 3.26 (2H, ddd, J = 20.8, 10.4, 4.0 Hz), 3.40-3.48 (1H, m), 3.62-3.71 (2H, m), 4.04-4.20 (1H, m), 4.28 (1H, d, J = 8.4 Hz).

### Examples 2 to 17:

The compounds of Examples 2 to 17 shown in the table below were synthesized in the same manner as Example 1 by using each literacture-known product or compound of Reference Example shown in the table below as the compounds corresponding to Starting material A and Starting material B in Example 1.

**[Table 1]**

| Example | Structural Formula | Starting material A | Starting material B |
|---|---|---|---|
| | Spectral Data | | |
| 2 | 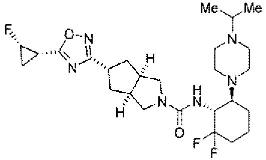 | Literature-known product | Reference Example 5 |
| | | 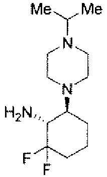 | 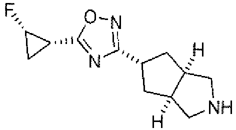 |
| | ¹H-NMR (CDCl₃) δ: 1.01 (6H, d, J = 6.0 Hz), 1.21-1.53 (3H, m), 1.60-1.86 (3H, m), 1.88-2.05 (4H, m), 2.10-2.27 (3H, m), 2.29-2.53 (7H, m), 2.57 (1H, sep, J = 6.4 Hz), 2.70-2.80 (2H, m), 2.88-3.02 (2H, m), 3.27 (2H, ddd, J = 21.2, 10.4, 4.0 Hz), 3.50 (1H, dq, J = 8.0, 7.2 Hz), 3.67 (2H, dd, J = 10.0, 7.6 Hz), 4.05-4.20 (1H, m), 4.28 (1H, d, J = 7.6 Hz), 4.91 (1H, ddt, J = 63.2, 6.0, 3.6 Hz). | | |
| 3 | 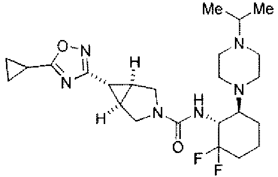 | Literature-known product | Reference Example 6 |
| | | 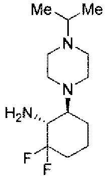 | 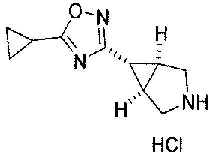 |
| | ¹H-NMR (CDCl₃) δ: 4.26 (1H, d, J = 8.5 Hz), 4.17-4.03 (1H, m), 3.76 (2H, dd, J = 17.1, 9.8 Hz), 3.62-3.53 (2H, m), 2.78-2.69 (2H, m), 2.65-2.55 (1H, m), 2.55-2.31 (7H, m), 2.22-2.08 (4H, m), 1.98-1.88 (2H, m), 1.85-1.61 (2H, m), 1.46-1.13 (6H, m), 1.03 (6H, d, J = 6.7 Hz). | | |
| 4 | | Literature-known product | Reference Example 7 |
| | 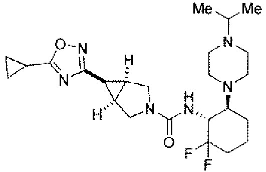 | 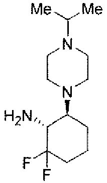 | 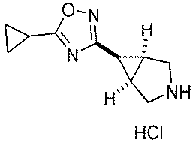 |
| | ¹H-NMR (CDCl₃) δ: 4.11 (1H, d, J = 6.7 Hz), 4.03-3.88 (1H, m), 3.80-3.66 (1H, m), 3.62-3.46 (3H, m), 3.32-2.47 (8H, m), 2.46-2.35 (1H, m), 2.20-2.01 (5H, m), 2.01-1.53 (4H, m), 1.43-1.20 (8H, m), 1.20-1.09 (4H, m). | | |

**[Table 2]**

| | | | |
|---|---|---|---|
| 5 | 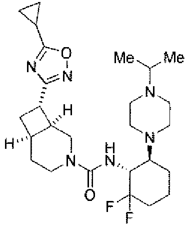 | Literature-known product | Reference Example 8 |
| | | 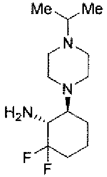 | 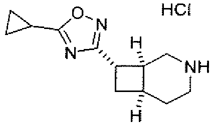 |
| | mixtures of two diastereomers : ¹H-NMR (CDCl₃) δ: 4.77 (1H, d, J = 7.4 Hz), 4.54 (1H, d, J = 7.4 Hz), 4.34-4.09 (3H, m), 3.86-3.69 (3H, m), 3.66-3.57 (1H, m), 3.54-3.45 (1H, m), 3.33-3.23 (3H, m), 2.94-2.83 (2H, m), 2.83-2.69 (4H, m), 2.68-2.09 (27H, m), 2.07-1.87 (6H, m), 1.86-1.60 (3H, m), 1.46-1.22 (5H, m), 1.22-1.14 (8H, m), 1.04-0.94 (12H, m). | | |
| 6 | 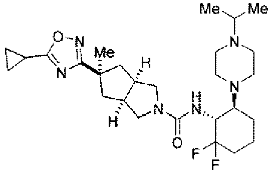 | Literature-known product | Reference Example 9 |
| | | 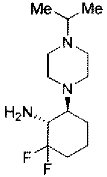 | 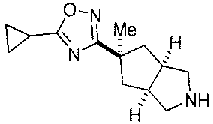 |
| | ¹H-NMR (CDCl₃) δ: 0.98 (6H, d, J = 5.6 Hz), 1.12-1.34 (5H, m), 1.34-1.45 (4H, m), 1.61-1.86 (2H, m), 1.87-1.97 (1H, m), 1.97-2.11 (4H, m), 2.11-2.22 (2H, m), 2.31-2.50 (7H, m), 2.54 (1H, sep, J = 6.8 Hz), 2.69-2.80 (2H, m), 2.87-2.99 (2H, m), 3.35 (2H, dt, J = 10.0, 3.6 Hz), 3.60 (2H, dd, J = 10.0, 7.6 Hz), 4.04-4.20 (1H, m), 4.26 (1H, d, J = 8.4 Hz). | | |
| 7 | 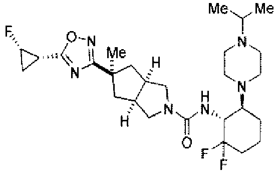 | Literature-known product | Reference Example 10 |
| | | 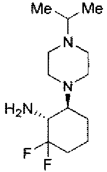 | 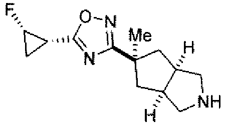 |
| | ¹H-NMR (CDCl₃) δ: 0.98 (6H, d, J = 6.4 Hz), 1.20-1.51 (7H, m), 1.61-1.85 (2H, m), 1.87-2.22 (7H, m), 2.29-2.50 (7H, m), 2.54 (1H, sep, J = 6.8 Hz), 2.69-2.79 (2H, m), 2.86-3.00 (2H, m), 3.54 (2H, ddd, J = 14.0, 10.4, 3.6 Hz), 3.61 (2H, ddd, J = 10.4, 8.0, 3.2 Hz), 4.03-4.19 (1H, m), 4.26 (1H, d, J = 8.4 Hz), 4.90 (1H, ddt, J = 64.0, 6.0, 4.0 Hz). | | |

**[Table 3]**

| | | | |
|---|---|---|---|
| 8 | 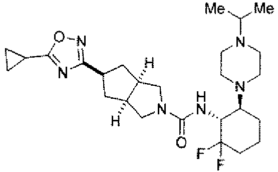 | Literature-known product | Reference Example 11 |
| | | 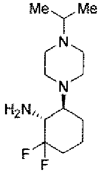 | 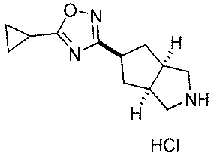 |
| | 1H-NMR (CDCl₃) δ: 4.25 (1H, d, J = 8.6 Hz), 4.18-4.04 (1H, m), 3.64-3.56 (2H, m), 3.37 (2H, ddd, J = 14.7, 10.4, 3.7 Hz), 3.32-3.23 (1H, m), 2.83-2.70 (4H, m), 2.60-2.24 (10H, m), 2.19-2.10 (2H, m), 1.96-1.88 (1H, m), 1.86-1.61 (4H, m), 1.46-1.12 (6H, m), 0.98 (6H, d, J = 6.1 Hz). | | |
| 9 | 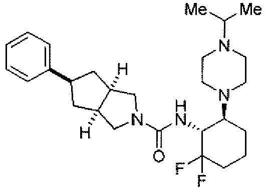 | Literature-known product | Literature-known product |
| | | 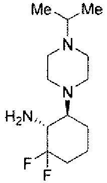 | 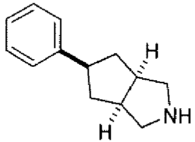 |
| | ¹H-NMR (CDCl₃) δ: 0.94 (6H, s), 1.17-1.38 (2H, m), 1.47-1.76 (5H, m), 1.87 (1H, m), 2.10 (1H, m), 2.25-2.44 (9H, m), 2.71-2.78 (4H, m), 3.09 (1H, m), 3.27-3.35 (2H, m), 3.51-3.56 (2H, m), 4.06 (1H, m), 4.23 (1H, d, J = 8 Hz), 7.10-7.23 (6H, m). | | |

**[Table 4]**

| | | | |
|---|---|---|---|
| 10 | 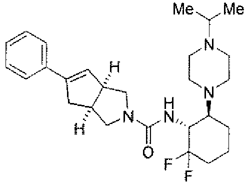 | Literature-known product | Literature-known product |
| | | 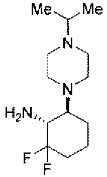 | 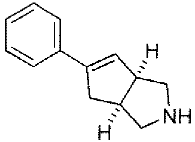 |
| | Mixtures of two diastereomers : ¹H-NMR (CDCl₃) δ: 0.94 (6H, m), 1.15-1.73 (5H, m), 1.84 (1H, d, J = 13.2 Hz), 2.08 (1H, m), 2.29-2.40 (6H, m), 2.56 (1H, d, J = 13.2 Hz), 2.62-2.69 (2H, m), 2.91 (1H, m), 2.98-3.11 (2H, m), 3.43-3.54 (2H, m), 3.60 (1H, m), 3.72 (1H,m), 4.02 (1H, m), 4.17 (1H, d, J = 8.8 Hz), 5.97 (1H, s), 7.15-7.18 (2H, m), 7.22-7.26 (2H, m), 7.30-7.35 (2H, m). | | |
| 11 | 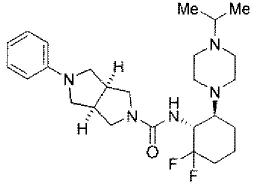 | Literature-known product | Literature-known product |
| | | 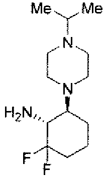 | 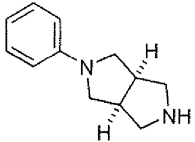 |
| | ¹H-NMR (CDCl₃) δ: 7.25-7.21 (2H, m), 6.70 (1H, dd, J = 7.3, 3.7 Hz), 6.56-6.54 (2H, m), 4.25 (1H, d, J = 8.5 Hz), 3.76-3.73 (2H, m), 3.58-3.56 (2H, m), 3.45-3.42 (1H, m), 3.37-3.35 (1H, m), 3.28-3.23 (2H, m), 3.10-3.07 (2H, m), 2.75-2.74 (2H, m), 2.49-2.41 (6H, m), 1.55 (8H, s), 0.98 (6H, d, J = 6.1 Hz). (1H, d, J = 8.5 Hz), 7.91 (1H, dd, J = 8.5, 2.4 Hz), 8.04 (1H, d, J = 1.2 Hz), 8.41 (1H, s). | | |
| 12 | | Literature-known product | Literature-known product |
| | 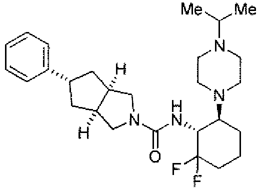 | 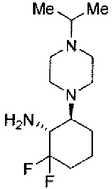 | 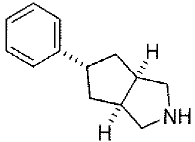 |
| | ¹H-NMR (CDCl₃) δ: 1.00 (5H, d, J = 6.1 Hz), 1.34 (2H, dt, J = 10.0, 5.0 Hz), 1.57 (4H, s), 1.81-1.78 (1H, m), 2.00-1.95 (3H, m), 2.19-2.16 (1H, m), 2.54-2.45 (8H, m), 2.77-2.75 (2H, m), 2.98-2.95 (2H, m), 3.30-3.26 (2H, m), 3.74-3.72 (2H, m), 4.16-4.12 (1H, m), 4.31 (1H, d, J = 8.5 Hz), 7.31-7.17 (5H,m). | | |

**[Table 5]**

| | | | |
|---|---|---|---|
| 13 | 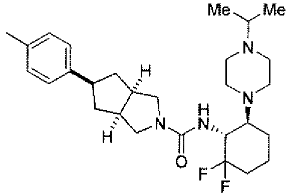 | Literature-known product | Reference Example 12 |
| | | 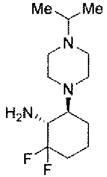 | 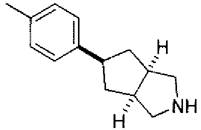 |
| | ¹H-NMR (CDCl₃) δ: 0.98 (6H, d, J = 6.1 Hz), 1.39-1.27 (2H, m), 1.59-1.55 (6H, m), 1.80-1.78 (1H, m), 1.95-1.92 (1H, m), 2.18-2.16 (1H, m), 2.32 (1H, s), 2.50-2.40 (8H, m), 2.81-2.75 (4H, m), 3.15-3.09 (1H, m), 3.40-3.36 (2H, m), 3.64-3.58 (2H, m), 4.17-4.12 (1H, m), 4.30 (1H, d, J = 7.9 Hz), 7.11 (4H, dd, J = 13.1, 8.2 Hz). | | |
| 14 | 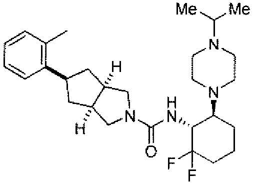 | Literature-known product | Reference Example 13 |
| | | 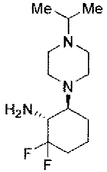 | 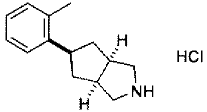 |
| | ¹H-NMR (CDCl₃) δ: 1.00 (6H, d, J = 6.4 Hz), 1.46-1.21 (2H, m), 1.63-1.58 (4H, m), 1.81-1.80 (1H, m), 1.96-1.92 (1H, m), 2.17-2.16 (1H, m), 2.32-2.25 (2H, m), 2.35 (3H, s), 2.59-2.40 (8H, m), 2.86-2.67 (3H, m), 3.44-3.25 (2H, m), 3.64-3.61 (2H, m), 4.20-4.09 (1H, m), 4.31 (1H, d, J = 7.9 Hz), 7.31-7.07 (4H, m). | | |
| 15 | 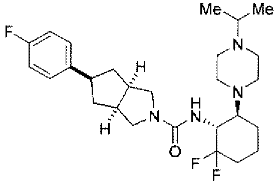 | Literature-known product | Reference Example 14 |
| | | 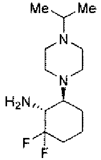 | 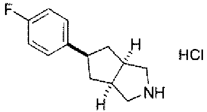 |
| | ¹H-NMR (CDCl₃) δ: 0.97 (6H, d, J = 6.7 Hz), 1.35 (2H, m), 1.58-1.48 (1H, m), 1.64 (2H, m), 1.79 (1H. m), 1.93 (1H, m), 2.17 (1H, m), 2.42 (9H, m), 2.78 (4H, m), 3.17-3.08 (1H, m), 3.39 (2H, dm), 3.62-3.52 (2H, m), 4.18-4.08 (1H, m), 4.30 (1H, d, J = 7.9 Hz), 7.03-6.91 (2H, m), 7.22-7.16 (2H, m). | | |

**[Table 6]**

| | | | |
|---|---|---|---|
| 16 | 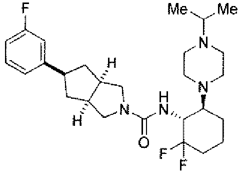 | Literature-known product | Reference Example 15 |
| | | 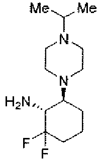 | 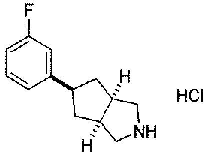 |
| | ¹H-NMR (CDCl₃) δ: 0.96 (6H, d, J = 6.7 Hz), 1.45-1.23 (2H, m), 1.58-1.55 (2H, m), 1.82-1.79 (1H, m), 1.82-1.79 (1H, m), 1.95-1.92 (1H,m), 2.18-2.16 (1H, m), 2.55-2.33 (9H, m), 2.84-2.78 (4H, m), 3.17-3.14 (1H, m), 3.41-3.36 (2H, m), 3.63-3.58 (2H, m), 4.19-4.08 (1H, m), 4.30 (1H, d, J = 7.9 Hz), 6.91-6.88 (2H, m), 7.01-6.99 (1H, m), 7.25-7.21 (1H, m). | | |
| 17 | 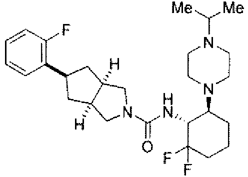 | Literature-known product | Reference Example 16 |
| | | 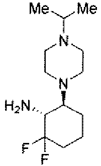 | 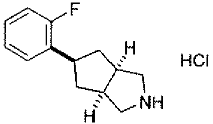 |
| | ¹H-NMR (CDCl₃) δ: 0.97 (6H, d, J = 6.7 Hz), 1.39-1.30 (2H, m), 1.58 (2H, s), 1.65-1.62 (1H, m), 1.81-1.80 (1H, m), 1.95-1.92 (1H, m), 2.18-2.16 (1H, m), 2.55-2.31 (9H, m), 2.83-2.75 (4H, m), 3.44-3.34 (3H, m), 3.64-3.59 (2H, m), 4.19-4.08 (1H, m), 4.30 (1H, d, J = 7.9 Hz), 7.20-6.97 (4H, m). | | |

The chemical names of Examples 2 to 4, Examples 6 to 9 and Examples 11 to 17 are described below.
Example 2: (3aR,5S,6aS)-N-{(1R,6S)-2,2-difluoro-6-[4-(propan-2-yl)piperazin-1 - yl]cyclohexyl}-5-{ 5-[(1S,2S)-2-fluorocyclopropyl]-1,2,4-oxadiazol-3-yl}hexahydropenta[c]pyrrole-2(1H)-carboxamide
Example 3: (1R,5S,6R)-6-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[3.1.0]hexane-3-carboxamide
Example 4: (1R,5S,6S)-6-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[3.1.0]hexane-3-carboxamide
Example 6: (3aR,5R,6aS)-5-(5-cyclopropyl-1,2,4-oxadiazol-3-yl)-N-{(1R.6S)-2,2-difluoro-6-[4-(propan-2-yl)piperazin-1-yl]cyclohexyl}1-5-methylhexahydropenta[c]pyrrole-2(1H)-carboxamide
Example 7: (3aR,5R,6aS)-N-{(1R,6S)-2,2-difluoro-6-[4-(propan-2-yl)piperazin-1-yl]cyclohexyl}-5-{5-[(1S,2S)-2-fluorocyclopropyl]-1,2,4-oxadiazol-3-yl}-5-methylhexahydropenta[c]pyrrole-2(1H)-carboxamide
Example 8: (3aR,5R,6aS)-5-(5-cyclopropyl-1,2,4-oxadiazol-3-yl)-N-{(1R,6S)-2,2-difluoro-6-[4-(propan-2-yl)piperazin-1-yl]cyclohexyl}hexahydropenta[c]pyrrole-2(1H)-carboxamide
Example 9: (3aR,5R,6aS)-N-{(1R,6S)-2,2-difluoro-6-[4-(propan-2-yl)piperazin-1-yl]cyclohexyl}-5-phenylhexahydropenta[c]pyrrole-2(1H)-carboxamide
Example 11:(3aR,6aS)-N-{(1R,6S)-2,2-difluoro-6-[4-(propan-2-yl)piperazin-1-yl]cyclohexyl}-5-phenylhexahydropyrrolo[3,4-c]pyrrole-2(1H)-carboxamide
Example 12: (3aR,5S,6aS)-N-{(1R,6S)-2,2-difluoro-6-[4-(propan-2-yl)piperazin-1-yl]cyclohexyl)-5-phenylhexahydropenta[c]pyrrole-2(1H)-carboxamide
Example 13: (3aR,5R.6aS)-N-{(1R,6S)-2,2-difluoro-6-[4-(propan-2-yl)piperazin-1-yl)cyclohexyl}-5-(4-methylphenyl)hexahydropenta[c]pyrrole-2(1H)-carboxamide
Example 14: (3aR,5R,6aS)-N-{(1R,6S)-2,2-difluoro-6-[4-(propan-2-yl)piperazin-1-yl]cyclohexyl}-5-(2-methylphenyl)hexahydropenta[c]pyrrole-2(1H)-carboxamide
Example 15: (3aR,5R,6aS)-N-{(1R,6S)-2,2-difluoro-6-[4-(propan-2-yl)piperazin-1-yl]cyclohexyl}-5-(4-fluorophenyl)hexahydropenta[c]pyrrole-2(1H)-carboxamide
Example 16: (3aR,5R,6aS)-N-{(1R,6S)-2,2-difluoro-6-[4-(propan-2-yl)piperazin-1-yl]cyclohexyl}-5-(3-iluorophenyl)hexahydropenta[c]pyrrole-2(1H)-carboxamide
Example 17: (3aR,5R,6aS)-N-{(1R,6S)-2,2-difluoro-6-[4-(propan-2-yl)piperazin-1-yl]cyclohexyl}-5-(2-fluorophenyl)hexahydropenta[c]pyrrole-2(1H)-carboxamide

The compounds of Example 5 and Example 10 are diastereomeric mixtures having two different diastereomers, respectively. They can be asymmetrically synthesized and fractionated by chiral chromatography. In other words, each of such two diastereomers can be said to have been substantially synthesized.

**[Table 7]**

| Example | Chemical structure of diastereomer | Chemical name |
|---|---|---|
| 5-A | 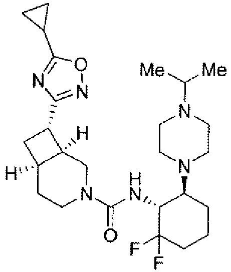 | (1S,6R,8S)-8-(5-cyclopropyl-1,2,4-oxadiazol-3-yl)-N-{(1R,6S)-2,2-difluoro-6-[4-(propan-2-yljpiperazin-1-yl]cyclohexyl}-3-azabicyclo[4.2.0]octane-3-carboxamide |
| 5-B | 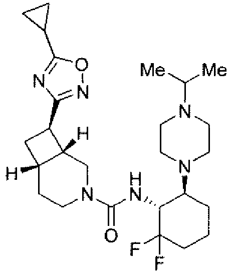 | (1R,6S,8R)-8-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[4.2.0]octane-3-carboxamide |
| 10-A | 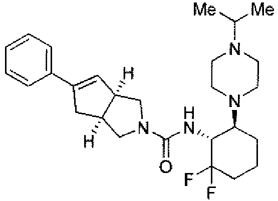 | (3aS,6aR)-N-{(1R,6S)-2,2-difluoro-6-[4-(propan-2-yl)piperazin-1-yl)cyclohexyl} -5-phenyl-3,3a,4,6a-tetrahydropenta[c]pyrrole-2(1H)-carboxamide |
| 10-B | 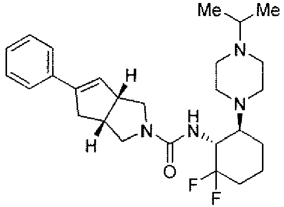 | (3aR,6aS)-N-{(1R,6S)-2,2-difluoro-6-[4-(propan-2-yl)piperazin-1-yl]cyclohexyl}-5-phenyl-3,3a,4,6a-tetrahydropenta[c]pyrrole-2(1H)-carboxamide |

### Examples 18 to 34:

The compounds of Examples 18 to 34 shown in the table below were synthesized in the same manner as Example 1 by using each literature-known product or compound of Reference Example shown in the table below as the compounds corresponding to Starting material A and Starting material B in Example 1.

**[Table 8]**

| Example | Structural Formula | Starting material A | Starting material B |
|---|---|---|---|
| | Spectral Data | | |
| 18 | 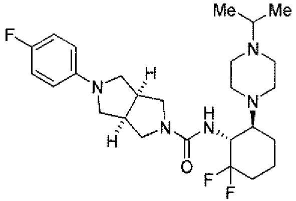 | Literature-known product | Literature-known product |
| | | 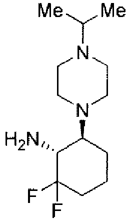 | 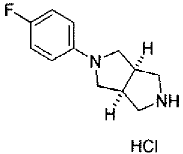 |
| | ¹H-NMR (CDCl₃) δ: 0.97 (6H, d, J = 18.9 Hz), 1.44-1.23 (2H, m), 1.76-1.64 (1H, m), 1.81-1.78 (1H, m), 1.94-1.91 (1H, m), 2.17-2.15 (1H, m), 2.55-2.37 (8H, m), 2.75-2.73 (2H, m), 3.11-3.06 (2H, m), 3.23-3.19 (2H, m), 3.44-3.36 (2H, m), 3.54-3.49 (2H, m), 3.76-3.71 (2H, m), 4.17-4.05 (1H, m), 4.27 (1H, d, J = 7.9 Hz), 6.46-6.45 (2H, m), 6.97-6.90 (2H, m). | | |
| 19 | 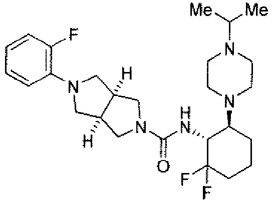 | Literature-known product | Reference Example 17 |
| | | 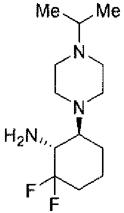 | 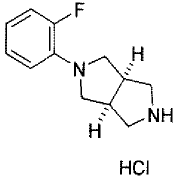 |
| | ¹H-NMR (CDCl₃) δ: 0.95 (6H, d, J = 58.5 Hz), 1.50-1.19 (2H, m), 1.86-1.59 (2H, m), 1.99-1.86 (1H, m). 2.25-2.08 (1H, m), 2.55-2.27 (8H, m), 2.79-2.65 (2H, m), 3.13-2.89 (2H, m), 3.39-3.25 (3H, m), 3.51-3.39 (1H, m), 3.66-3.54 (2H, m), 3.79-3.66 (2H, m), 4.20-4.01 (1H, m), 4.26 (1H, d, J = 23.8 Hz), 6.79-6.57 (2H, m), 7.05-6.84 (2H, m). | | |
| 20 | | Literature-known product | Literature-known product |
| | 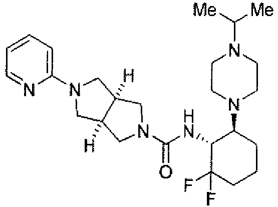 | 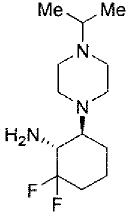 | 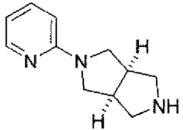 |
| | ¹H-NMR (CDCl₃) δ: 0.95 (6H, d, J = 39.0 Hz), 1.47-1.20 (2H, m), 1.86-1.65 (2H, m), 1.98-1.87 (1H, m), 2.22-2.10 (1H, m), 2.54-2.26 (8H, m), 2.81-2.65 (2H, m), 3.16-3.00 (2H, m), 3.48-3.32 (4H, m), 3.80-3.68 (4H, m), 4.19-4.03 (1H, m), 4.27 (1H, d, J = 18.3 Hz), 6.37-6.29 (1H, m), 6.61-6.51 (1H, m), 7.48-7.39 (1H, m), 8.18-8.11 (1H, m). | | |

**[Table 9]**

| | | | |
|---|---|---|---|
| 21 | 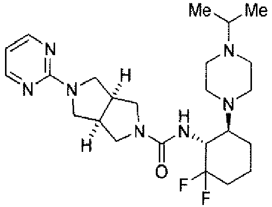 | Literature-known product | Literature-known product |
| | | 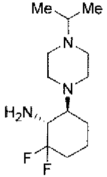 | 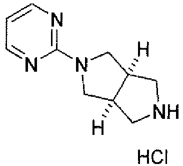 |
| | ¹H-NMR (CDCl₃) δ: 0.96 (6H, d, J = 33.5 Hz), 1.48-1.20 (2H, m), 1.86-1.68 (2H, m), 2.00-1.86 (1H, m), 2.22-2.09 (1H, m), 2.57-2.30 (8H, m), 2.78-2.67 (2H, m), 3.15-3.00 (2H, m), 3.46-3.32 (2H, m), 3.61-3.51 (2H, m), 3.82-3.69 (2H, m), 3.91-3.82 (2H, m), 4.20-4.03 (1H, m), 4.27 (1H, d, J = 21.3 Hz), 6.50 (1H, t, J = 20.7 Hz), 8.31 (2H, d, J = 14.6 Hz). | | |
| 22 | 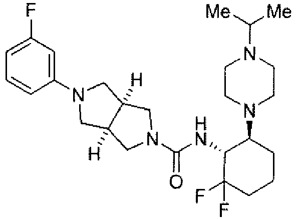 | Literature-known product | Reference Example 18 |
| | | 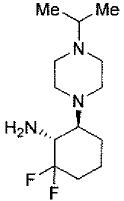 | 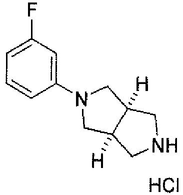 |
| | ¹H-NMR (CDCl₃) δ: 0.97 (6H, d, J = 20.1 Hz), 1.47-1.21 (2H, m), 1.75-1.63 (1H, m), 1.86-1.75 (1H, m), 1.97-1.88 (1H, m), 2.22-2.11 (1H, m), 2.55-2.32 (8H, m), 2.78-2.68 (2H, m), 3.14-3.01 (2H, m), 3.27-3.19 (2H, m), 3.39-3.32 (1H, m), 3.46-3.41 (1H, m), 3.59-3.53 (2H, m), 3.79-3.70 (2H, m), 4.19-4.04 (1H, m), 4.26 (1H, d, J = 18.9 Hz), 6.24-6.18 (1H, m), 6.32-6.26 (1H, m), 6.42-6.34 (1H, m), 7.18-7.10 (1H, m). | | |
| 23 | | Literature-known product | Literature-known product |
| | 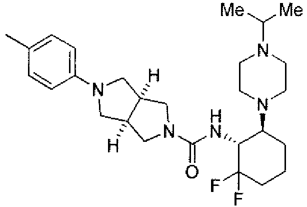 | 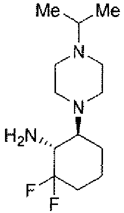 | 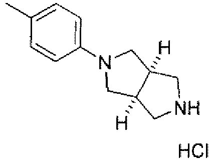 |
| | ¹H-NMR (CDCl₃) δ: 0.98 (6H, d, J = 21.3 Hz), 1.47-1.24 (2H, m), 1.86-1.62 (2H, m), 1.97-1.87 (1H, m), 2.22-2.09 (1H, m), 2.26 (3H, s), 2.53-2.33 (8H, m), 2.78-2.68 (2H, m), 3.12-2.99 (2H, m), 3.27-3.19 (2H, m), 3.39-3.30 (1H, m), 3.46-3.39 (1H, m), 3.58-3.50 (2H, m), 3.77-3.69 (2H, m), 4.19-4.04 (1H, m), 4.25 (1H, d, J = 17.7 Hz), 6.47 (2H, d, J = 40.2 Hz), 7.03 (2H, d, J = 23.8 Hz). | | |

**[Table 10]**

| | | | |
|---|---|---|---|
| 24 | 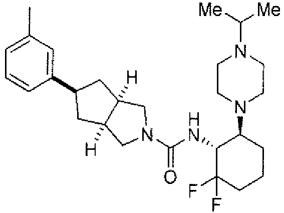 | Literature-known product | Reference Example 19 |
| | | 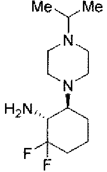 | 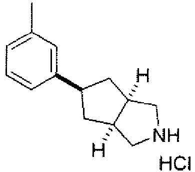 |
| | ¹H-NMR (CDCl₃) δ: 0.89 (6H, d, J = 24.4 Hz), 1.42-1.16 (2H, m), 1.57-1.46 (2H, m), 1.79-1.57 (2H, m), 1.92-1.81 (1H, m), 2.16-2.04 (1H, m), 2.53-2.16 (13H, m), 2.78-2.64 (4H, m), 3.11-2.99 (1H, m), 3.36-3.27 (2H, m), 3.60-3.50 (2H, m), 4.14-4.00 (1H, m), 4.23 (1H, d, J = 18.9 Hz), 7.01-6.91 (3H, m), 7.14-7.07 (1H, m). | | |
| 25 | 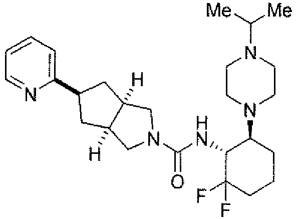 | Literature-known product | Reference Example 20 |
| | | 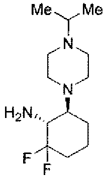 | 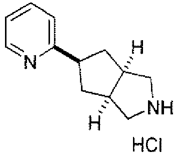 |
| | ¹H-NMR (CDCl₃) δ: 1.19-0.81 (6H, m), 1.48-1.23 (2H, m), 1.64-1.48 (2H, m), 1.90-1.64 (4H, m), 2.03-1.88 (1H, m), 2.25-2.12 (1H, m), 2.69-2.29 (9H, m), 2.92-2.74 (4H, m), 3.49-3.27 (3H, m), 3.66-3.54 (1H, m), 4.22-4.03 (1H, m), 4.29 (1H, d, J = 22.5 Hz), 7.14-7.06 (1H, m), 7.20-7.14 (1H, m), 7.62-7.55 (1H, m), 8.56-8.47 (1H, m). | | |
| 26 | 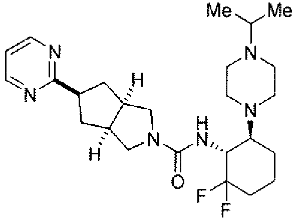 | Literature-known product | Reference Example 21 |
| | | 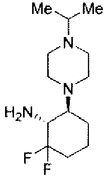 | 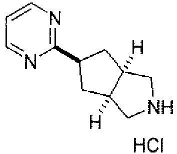 |
| | | | ¹H-NMR (CDCl₃) δ: 1.18-0.93 (6H, m), 1.48-1.23 (2H, m), 1.87-1.48 (3H, m), 2.00-1.87 (1H, m), 2.10-2.00 (1H, m), 2.23-2.10 (1H, m), 2.34-2.23 (2H, m), 2.93-2.34 (8H, m), 3.07-2.93 (2H, m), 3.35-3.22 (2H, m), 3.47-3.38 (1H, m), 3.58-3.49 (1H, m), 3.74-3.58 (3H, m), 4.20-4.06 (1H, m), 4.33-4.24 (1H, m), 7.14-7.09 (1H, m), 8.68-8.63 (2H, m). |

**[Table 11]**

| | | | |
|---|---|---|---|
| 27 | 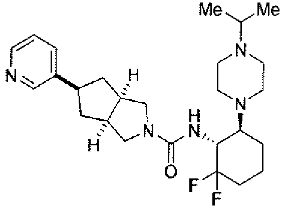 | Literature-known product | Reference Example 22 |
| | | 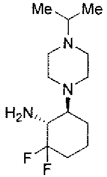 | 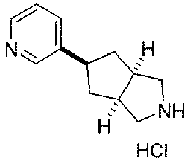 |
| | ¹H-NMR (CDCl₃) δ: 1.12-0.88 (6H, m), 1.48-1.21 (2H, m), 1.88-1.51 (5H, m), 1.99-1.88 (1H, m), 2.24-2.10 (1H, m), 2.68-2.32 (9H, m), 2.92-2.71 (4H, m), 3.24-3.09 (1H, m), 3.46-3.35 (2H, m), 3.68-3.53 (2H, m), 4.22-4.05 (1H, m), 4.31 (1H, d, J = 23.2 Hz), 7.25-7.17 (1H, m), 7.59-7.48 (1H, m), 8.52-8.40 (2H, m). | | |
| 28 | 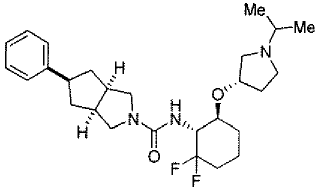 | Literature-known product | Literature-known product |
| | | 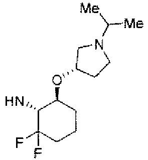 | 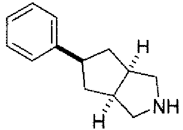 |
| | ¹H-NMR (CDCl₃) δ: 1.01 (3H, d, J = 6.0 Hz), 1.03 (3H, d, J = 6.0 Hz), 1.32-1.49 (2H, m), 1.49-1.61 (2H, m), 1.64-1.78 (3H, m), 1.91-2.03 (1H, m), 2.02-2.19 (2H, m), 2.28-2.38 (4H, m), 2.41-2.50 (1H, m), 2.59-2.66 (1H, m), 2.73-2.85 (2H, m), 2.98-3.04 (1H, m), 3.07-3.23 (2H, m), 3.32 (1H, dd, J = 9.8, 3.1 Hz), 3.39 (1H, dd, J = 10.1, 3.4 Hz), 3.51-3.62 (2H, m), 4.07-4.25 (2H, m), 4.31 (1H, d, J = 9.2 Hz), 7.14-7.22 (3H, m), 7.24-7.30 (2H, m). | | |
| 29 | 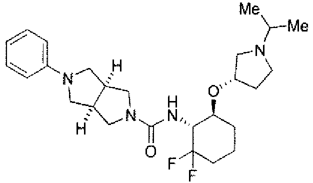 | Literature-known product | Literature-known product |
| | | 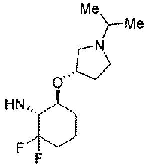 | 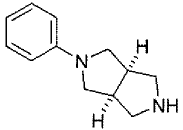 |
| | ¹H-NMR (CDCl₃) δ: 1.01 (3H, d, J = 6.1 Hz), 1.02 (3H, d, J = 6.1 Hz), 1.30-1.47 (2H, m), 1.60-1.82 (3H, m), 1.87-1.98 (1H, m), 2.00-2.18 (2H, m), 2.26-2.37 (2H, m), 2.37-2.45 (1H, m), 2.55-2.63 (1H, m), 2.96-3.11 (3H, m), 3.13-3.26 (3H, m), 3.32 (1H, dd, J = 9.8, 4.3 Hz), 3.42 (1H, dd, J = 10.4, 4.3 Hz), 3.49-3.57 (2H, m), 3.64-3.74 (2H, m), 4.05-4.22 (2H, m), 4.25 (1H, d, J = 9.2 Hz), 6.53 (2H, d, J = 7.3 Hz), 6.68 (1H, dd, J = 7.6, 7.3 Hz), 7.20 (2H, dd, J = 8.6, 7.6 Hz). | | |

**[Table 12]**

| | | | |
|---|---|---|---|
| 30 | 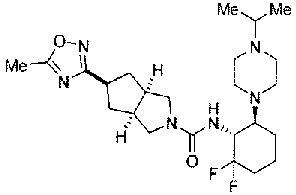 | Literature-known product | Reference Example 23 |
| | | 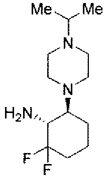 | 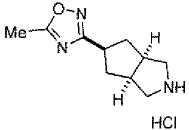 |
| | ¹H-NMR (CDCl₃) δ: 0.96 (6H, d, J = 6.4 Hz), 1.20-1.44 (2H, m), 1.60-1.86 (4H, m), 1.86-1.95 (1H, m), 2.08-2.20 (1H, m), 2.26-2.58 (10H, m), 2.52 (3H, s), 2.66-2.85 (4H, m), 3.26-3.41 (3H, m), 3.55-3.64 (2H, m), 4.02-4.17 (1H, m), 4.24 (1H, d, J = 8.6 Hz). | | |
| 31 | 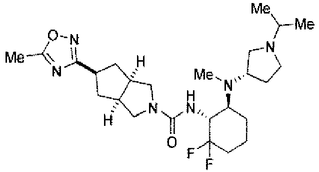 | Literature-known product | Reference Example 23 |
| | | 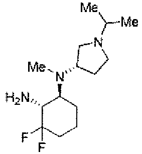 | 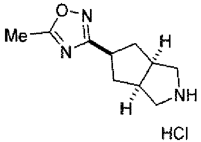 |
| | ¹H-NMR (CDCl₃) δ: 1.08 (3H, d, J = 6.1 Hz), 1.10 (3H, d, J = 6.1 Hz), 1.21-1.46 (3H, m), 1.50-1.60 (2H, m), 1.63-1.86 (2H, m), 1.89-2.00 (1H, m), 2.09-2.23 (5H, m), 2.25-2.65 (6H, m), 2.68-2.98 (4H, m), 3.13-3.41 (4H, m), 3.50-3.62 (2H, m), 4.03-4.19 (1H, m), 4.26 (1H, d, J = 8.5 Hz). | | |
| 32 | 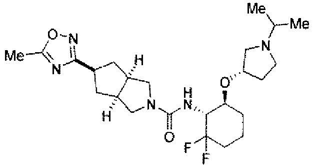 | Literature-known product | Reference Example 23 |
| | | 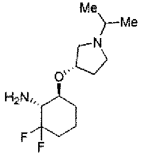 | 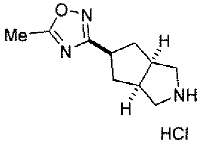 |
| | ¹H-NMR (CDCl₃) δ: 1.02 (3H, d, J = 6.7 Hz), 1.04 (3H, d, J = 6.7 Hz), 1.31-1.48 (2H, m), 1.63-1.84 (5H, m), 1.91-2.02 (1H, m), 2.02-2.19 (2H, m), 2.27-2.40 (4H, m), 2.40-2.50 (1H, m), 2.52 (3H, s), 2.62-2.72 (1H, m), 2.74-2.85 (2H, m), 3.00-3.08 (1H, m), 3.12-3.22 (1H, m), 3.27-3.40 (3H, m), 3.52-3.62 (2H, m), 4.06-4.23 (2H, m), 4.28 (1H, d, J = 9.2 Hz). | | |

**[Table 13]**

| | | | |
|---|---|---|---|
| 33 | 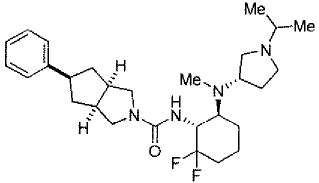 | Literature-known product | Literature-known product |
| | | 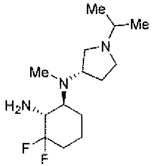 | 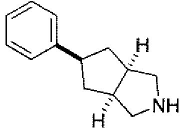 |
| | ¹H-NMR (CDCl₃) δ: 1.08 (3H, d, J = 6.1 Hz), 1.10 (3H, d, J = 6.1 Hz), 1.21-1.46 (3H, m), 1.50-1.60 (2H, m), 1.63-1.86 (2H, m), 1.89-2.00 (1H, m), 2.09-2.23 (5H, m), 2.25-2.65 (6H, m), 2.70-2.90 (4H, m), 3.06-3.19 (1H, m), 3.23-3.40 (3H, m), 3.50-3.62 (2H, m), 4.05-4.22 (1H, m), 4.29 (1H, d, J = 7.9 Hz), 7.12-7.31 (5H, m). | | |
| 34 | 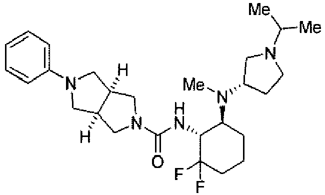 | Literature-known product | Literature-known product |
| | | 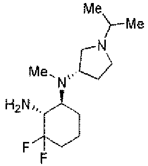 | 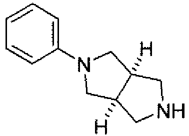 |
| | ¹H-NMR (CDCl₃) δ: 1.00 (3H, d, J = 6.4 Hz), 1.02 (3H, d, J = 6.4 Hz), 1.14-1.46 (3H, m), 1.49-1.95 (4H, m), 2.05-2.32 (6H, m), 2.36-2.45 (1H, m), 2.55-2.65 (1H, m), 2.68-2.77 (1H, m), 2.82-2.90 (1H, m), 3.00-3.08 (2H, m), 3.14-3.26 (3H, m), 3.29-3.56 (4H, m), 3.65-3.73 (2H, m), 4.04-4.16 (1H, m), 4.23 (1H, d, J = 7.9 Hz), 6.53 (2H, d, J = 7.9 Hz), 6.68 (1H, dd, J = 7.9, 7.3 Hz), 7.16-7.23 (2H, m). | | |

The chemical names of Examples 18 to 34 are described below.
Example 18: (3aR,6aS)-N-{(1R,6S)-2,2-difluoro-6-[4-(propan-2-yl)piperazin-1-yl]cyclohexyl}-5-(4-fluorophenyl)hexahydropyrrolo[3,4-c]pyrrole-2(1H)-carboxamide
Example 19: (3aR,6aS)-N- {(1R,6S)-2.2-difluoro-6-[4-(propan-2-yl)piperazin-1-yl]cyclohexyl}-5-(2-fluorophenyl)hexahydropyrrolo[3,4-c]pyrrole-2(1H)-carboxamide
Example 20: (3aR,6aS)-N-{(1R,6S)-2,2-difluoro-6-[4-(propan-2-yl)piperazin-1-yl]cyclohexyl}-5-(pyridin-2-yl)hexahydropyrrolo[3,4-c]pyrrole-2(1H)-carboxamide
Example 21: (3aR,6aS)-N-{(1R,6S)-2,2-difluoro-6-[4-(propan-2-yl)piperazin-1-yl]cyclohexyl}-5-(pyrimidin-2-yl)hexahydropyrrolo[3,4-c]pyrrole-2(1H)-carboxamide
Example 22: (3aR,6aS)-N-{(1R,6S)-2,2-difluoro-6-[4-(propan-2-yl)piperazin-1-yl]cyclohexyl}-5-(3-fluorophenyl)hexahydropyrrolo[3,4-c]pyrrole-2(1H)-carboxamide
Example 23: (3aR,6aS)-N-{(1R,6S)-2,2-difluoro-6-[4-(propan-2-yl)piperazin-I - yl]cyclohexyl}-5-(4-methylphenyl)hexahydropyrrolo[3,4-c]pyrrole-2(1H)-carboxamide
Example 24: (3aR,5R,6aS)-N-{(1R,6S)-2,2-difluoro-6-[4-(propan-2-yl)piperazin-1-yl]cyclohexyl}-5-(3-methylphenyl)hexahydropenta[c]pyrrole-2(1H)-carboxamide
Example 25: (3aR,5R,6aS)-N-{(1R,6S)-2,2-difluoro-6-[4-(propan-2-yl)piperazin-1-yl]cyclohexyl}-5-(pyridin-2-yl)hexahydropenta[c]pyrrole-2(1H)-carboxamide
Example 26: (3aR,5R,6aS)-N-{(1R,6S)-2,2-difluoro-6-[4-(propan-2-yl)piperazin-1-yl]cyclohexyl}-5-(pyrimidin-2-yl)hexahydropenta[c]pyrrole-2(1H)-carboxamide
Example 27: (3aR,5R,6aS)-N-{(1R,6S)-2,2-difluoro-6-[4-(propan-2-yl)piperazin-1-yl]cyclohexyl}-5-(pyridin-3-yl)hexahydropenta[c]pyrrole-2(1H)-carboxamide
Example 28: (3aR,5R,6aS)-N-[(1R,6S)-2,2-difluoro-6-{[(3S)-1-(propan-2-yl)pyrrolidin-3-yl]oxy}cyclohexyl]-5-phenylhexahydropenta[c]pyrrole-2(1H)-carboxamide
Example 29: (3aR,6aS)-N-[(1R,6S)-2,2-difluoro-6-{[(3S)-1-(propan-2-yl)pyrrolidin-3-yl]oxy}cyclohexyl]-5-phenylhexahydropyrrolo[3,4-c]pyrrole-2(1H)-carboxamide
Example 30: (3aR,5R,6aS)-N-{(1R,6S)-2,2-difluoro-6-[4-(propan-2-yl)piperazin-1-yl]cyclohexyl}-5-(5-methyl-1,2,4-oxadiazol-3-yl)hexahydropenta[c]pyrrole-2(1H)-carboxamide
Example 31: (3aR,5R,6aS)-N-[(1R,6S)-2,2-difluoro-6-{methyl[(3S)-1-(propan-2-yl)pyrrolidin-3-yl]amino} cyclohexy 1]-5-(5-methy 1-1 ,2,4-oxadiazol-3-yl)hexahydropenta[c]pyrrole-2(1H)-carboxamide
Example 32: (3aR,5R,6aS)-N-[(1R,6S)-2,2-difluoro-6-{[(3S)-1-(propan-2-yl)pyrrolidin-3-yl]oxylcyclohexyl]-5-(5-methyl-1,2,4-oxadiazol-3-yl)hexahydropenta[c]pyrrole-2(1H)-carboxamide
Example 33: (3aR,5R,6aS)-N-[(1R,6S)-2,2-difluoro-6-{methyl[(3S)-1-(propan-2-yl)pyrrolidin-3-yl]amino)cyclohexyl]-5-phenylhexahydropenta[c]pyrrole-2(1H)-carboxamide
Example 34: (3aR,6aS)-N-[(1R,6S)-2.2-difluoro-6-{methyl[(3S)-1-(propan-2-yl)pyrrolidin-3-yl]amino}cyclohexyl]-5-phenylhexahydropyrrolo[3,4-c]pyrrole-2(1H)-carboxamide

### Reference Example 1:

### tert-Butyl (3aR,5s,6aS)-5-cyanohexahydropenta[c]pyrrole-2(1H)-carboxylate (Compound 2) and tert-butyl (3aR,5r,6aS)-5-cyanohexahydropenta[c]pyrrole-2(1 H)-carboxylate (Compound 3)

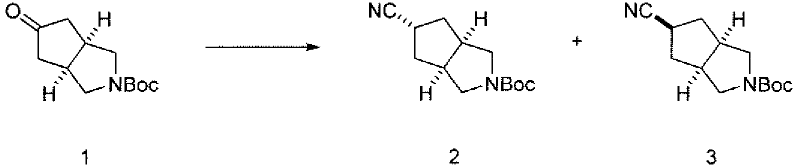

To a solution of Compound 1 (331 mg) in 1,2-dimethoxyethane (6.7 mL)/ethanol (0.67 mL) were added p-toluenesulfonylmethyl isocyanide (373 mg) and *tert-*butoxypotassium (396 mg) at 0°C and the mixture was stirred at 40°C for 4 hours. The mixture was cooled to room temperature, the reaction solution was filtrated, and the filtrate was concentrated *in vacuo.* The resulting residue was then purified by silica gel column chromatography (Eluate: hexane/ethyl acetate) to give the title compound 2 (80 mg) and the title compound 3 (59.4 mg).
Compound 2: LCMS: [M+H]⁺/Rt(min): 237/0.89
Compound 3: LCMS: [M+H]⁺/Rt(min): 237/0.88

### Reference Example 2:

### tert-Butyl (1S,6R,8S)-8-cyano-3-azabicyclo[4.2.0]octane-3-carboxylate

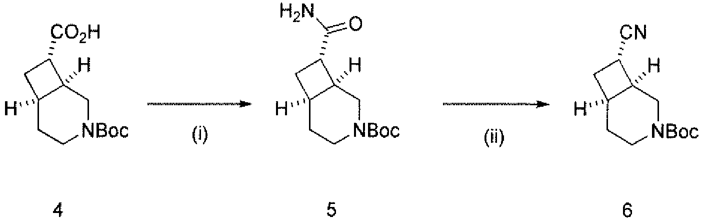

### Step (i):

To a solution of Compound 4 (600 mg) in chloroform (7.8 mL) were added isobutyl chloroformate (352 µL) and triethylamine (393 µL) at 0°C and the mixture was stirred for 2 hours. Ammonia solution was then added to the reaction solution at 0°C, and the mixture was warmed to room temperature and stirred for 1 hour. Water was then added to the reaction mixture and the mixture was extracted with chloroform. The organic layer was dried over anhydrous sodium sulfate and concentrated *in vacuo.* The resulting residue was then purified by silica gel column chromatography (Eluate: hexane/ethyl acetate) to give Compound 5 (372 mg).
LCMS: [M+H]⁺/Rt(min): 255/0.72

### Step (ii):

To a solution of Compound 5 (364 mg) and diisopropylethylamine (0.63 mL) in chloroform (6 mL) was added trifluoromethanesulfonic anhydride (0.27 mL) at 0°C and the mixture was stirred for 1 hour. Water was then added to the reaction mixture and the mixture was extracted with chloroform. The organic layer was dried over anhydrous sodium sulfate and concentrated *in vacuo.* The resulting residue was then purified by silica gel column chromatography (Eluate: hexane/ethyl acetate) to give Compound 6 (320 mg).
LCMS: [M+H]⁺/Rt(min): 237/0.93

### Reference Example 3:

### tert-Butyl (3aR,5r,6aS)-5-cyano-5-methylhexahydropenta[c]pyrrole-2(1H)-carboxylate

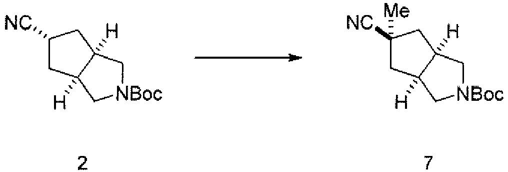

A solution of Compound 2 (2.05 g) in THF (8 mL) was added slowly dropwise to a solution of lithium diisopropylamide (1.09 M n-hexane solution, 16 mL) in THF (8 mL) at -78°C and then the mixture was stirred for 1.5 hours. A solution of iodomethane (2.7 mL) in THF (8 mL) was then added thereto at -78°C, and the mixture was warmed to 0°C and stirred for 2 hours. 1M aqueous potassium hydrogen sulfate solution was added to the reaction solution, and the mixture was warmed to room temperature and extracted with hexane. The organic layer was washed with water, dried over anhydrous sodium sulfate and concentrated *in vacuo.* The resulting residue was then purified by silica gel column chromatography (Eluate: hexane/ethyl acetate) to give the title compound 7 (0.8 g).
LCMS: [M+H]⁺/Rt(min): 251/0.93

### Reference Example 4:

### (3aR,5s,6aS)-5-(5-Cyclopropyl-1,2,4-oxadiazol-3-yl)octahydrocyclopenta[c]pyrrole

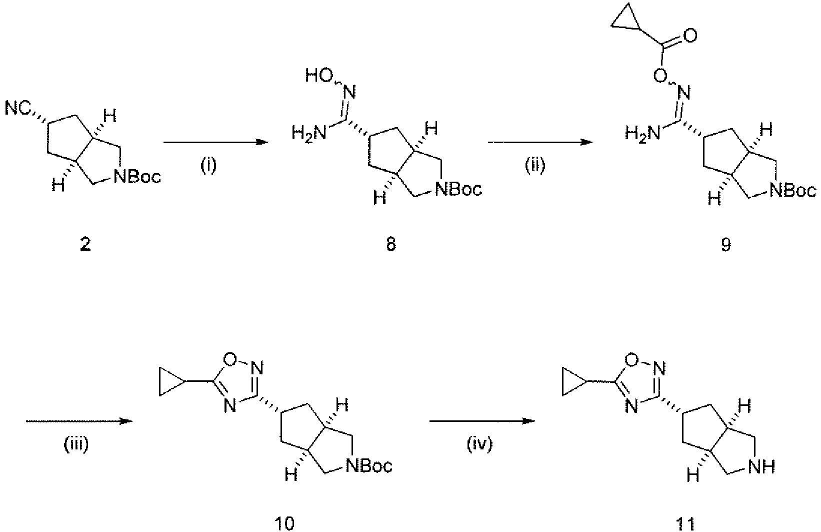

### Step (i):

To a solution of Compound 2 (7.3 g) in ethanol (62 mL) was added 50% aqueous hydroxylamine solution (20 mL) and the mixture was stirred at 70°C for 11 hours. The mixture was cooled to room temperature, water (120 mL) was added to the reaction mixture, and the mixture was extracted with toluene. The organic layer was dried over anhydrous sodium sulfate and concentrated *in vacuo* to give Compound 8 (4.6 g). LCMS: [M+H]⁺/Rt(min): 270/0.52

### Step (ii):

To a mixture of Compound 8 (1.47 g), cyclopropanecarboxylic acid (0.493 g), HATU (2.28 g) and THF (11 mL) was added slowly dropwise triethylamine (3.80 mL) in ice bath and the mixture was stirred at room temperature for 12 hours. Ethyl acetate (11 mL) was added to the reaction mixture and the mixture was washed with water (11 mL) and brine (11 mL). The organic layer was dried over anhydrous sodium sulfate and concentrated *in vacuo.* The resulting residue was then purified by silica gel column chromatography (Eluate: hexane/ethyl acetate) to give Compound 9 (3.71 g).
LCMS: [M+H]⁺/Rt(min): 338/0.82

### Step (iii):

A mixture of Compound 9 (400 mg), DBU (177 µL), toluene (2.2 mL) and acetonitrile (0.741 mL) was stirred with heating under reflux for 1 hour. The mixture was cooled to room temperature and washed with water, and the organic layer was concentrated *in vacuo.* The resulting residue was then purified by silica gel column chromatography (Eluate: hexane/ethyl acetate) to give Compound 10 (106 mg).
LCMS: [M+H]⁺/Rt(min): 320/1.04

### Step (iv):

To a solution of Compound 10 (880 mg) in toluene (2.8 mL) was added trifluoroacetic acid (1.6 mL) and the mixture was stirred overnight. The reaction solution was then concentrated *in vacuo,* toluene (2.76 mL)/acetonitrile (0.9 mL) and sodium carbonate (2.92 g) were added thereto, and the mixture was stirred for 1 hour. The reaction solution was filtrated and the filtrate was concentrated *in vacuo* to give the title compound 11 (690 mg).
LCMS: [M+H]⁺/Rt(min): 220/0.39

### Reference Examples 5 to 11:

The compound of Reference 5 shown in the table below was synthesized according to the process in the above Reference Example 4 by using the carboxylic acid B described in the table below instead of cyclopropanecarboxylic acid at Step (ii) in Reference Example 4, and the compounds of Reference Examples 6 to 11 shown in the table below were synthesized according to the process in the above Reference Example 4 by using each corresponding starting material A and carboxylic acid B instead of the material (Compound 2) at Step (i) and cyclopropanecarboxylic acid at Step (ii) in Reference Example 4.

**[Table 14]**

| Reference Example | Starting material A | Carboxylic acid B | Chemical Structural Formula | Instrumental Analysis Data |
|---|---|---|---|---|
| 5 | Reference Example 1 | Commercial Product | 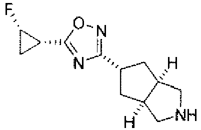 | LCMS: [M+H]⁺/Rt (min): 238/0.39 |
| | 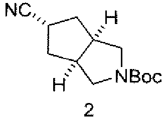 | 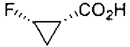 | | |
| 6 | Literature-known product | Commercial Product | 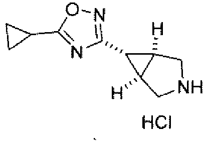 | LCMS: [M+H]⁺/Rt (min): 192/0.45 |
| | 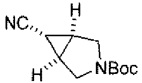 | 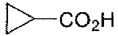 | | |
| 7 | Literature-known product | Commercial Product | 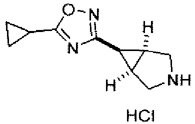 | LCMS: [M+H]⁺/Rt (min): 192/0.34 |
| | 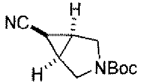 | 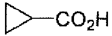 | | |
| 8 | Reference Example 2 | Commercial Product | 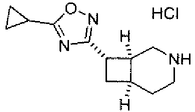 | LCMS: [M+H]⁺/Rt (min): 220/0.49 |
| | 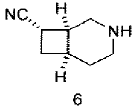 | 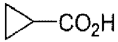 | | |
| 9 | Reference Example 3 | Commercial Product | 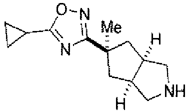 | LCMS: [M+H]⁺/Rt (min): 234/0.56 |
| | 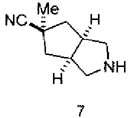 | 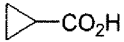 | | |
| 10 | Reference Example 3 | Commercial Product | | LCMS: [M+H]⁺/Rt |
| | 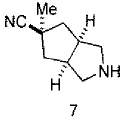 | 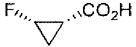 | 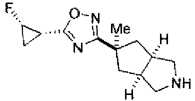 | (min): 252/0.54 |
| 11 | Reference Example 1 | Commercial Product | 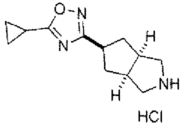 | LCMS: [M+H]⁺/Rt (min): 220/0.48 |
| | 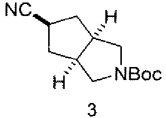 | 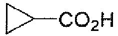 | | |

The chemical names of Reference Examples 5 to 11 are described below.
Reference Example 5: (3aR,5s,6aS)-5-{5-[(1S,2S)-2-fluorocyclopropyl]-1,2,4-oxadiazol-3-yl}octahydrocyclopenta[c]pyrrole
Reference Example 6: (1R,5S,6r)-6-(5-cyclopropyl-1,2,4-oxadiazol-3-yl)-3-azabicyclo[3.1.0]hexane monohydrochloride
Reference Example 7: (1R,5S,6s)-6-(5-cyclopropyl-1,2,4-oxadiazol-3-yl)-3-azabicyclo[3.1.0]hexane monohydrochloride
Reference Example 8: (1S,6R,8S)-8-(5-cyclopropyl-1,2,4-oxadiazol-3-yl)-3-azabicyclo[4.2.0]octane monohydrochloride
Reference Example 9: (3aR,5r,6aS)-5-(5-cyclopropyl-1,2,4-oxadiazol-3-yl)-5-methyloctahydrocyclopenta[c]pyrrole
Reference Example 10: (3aR,5r,6aS)-5-{5-[{1S,2S}-2-fluorocyclopropyl)-1,2,4-oxadiazol-3-yl}-5-methyloctahydrocyclopenta[c]pyrrole
Reference Example 11: (3aR,5r,6aS)-5-(5-cyclopropyl-1,2,4-oxadiazol-3-yl)octahydrocyclopenta[c]pyrrole

### Reference Example 12:

### (3aR,5r,6aS)-5-(4-Methylphenyl)octahydrocyclopenta[c]pyrrole

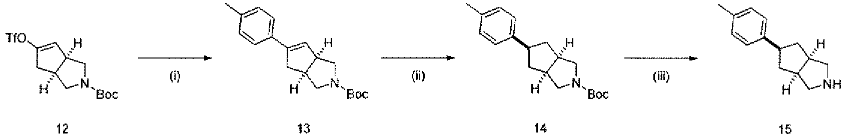

### Step (i):

To a solution of Compound 12 (326 mg) in 1,2-dimethoxyethane (3.2 mL) were added 4-methylphenylboronic acid (149 mg), aqeuous potassium carbonate solution (0.456 ml, 2M), and tetrakistriphenylphosphinepalladium (211 mg) at room temperature, and the mixture was stirred at 80°C for 11 hours. The mixture was cooled to room temperature, water (10 mL) was added to the reaction mixture, and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give Compound 13 (139 mg).
LCMS: [M+H]⁺/Rt(min): 300/2.313 (Method B)

### Step (ii):

To a solution of Compound 13 (139 mg) in methanol (3.9 mL) was added palladium hydroxide-activated carbon (26.1 mg), and the mixture was stirred at room temperature for 2 hours. After the reaction is finished, the reaction solution was filtrated with Celite and the filtrate was concentrated *in vacuo* to give Compound 14 (98 mg).
LCMS: [M+H]⁺/Rt(min): 302/2.346 (Method B)

### Step(iii):

To a solution of Compound 14 (98 mg) in chloroform (0.33 mL) was added TFA (0.125 mL), and the mixture was stirred at room temperature for 1 hour. After the reaction is finished, 3M aqueous sodium hydroxide solution was added thereto and the mixture was extracted with chloroform. The organic layer was dried over anhydrous sodium sulfate and concentrated *in vacuo* to give the title compound 15 (29.5 mg).
LCMS: [M+H]⁺/Rt(min): 202/1.481 (Method B)

### Reference Examples 13 to 16:

The compounds of References 13 to 16 shown in the table below were synthesized according to the process in the above Reference Example 12 by using each corresponding Starting material C instead of 4-methylphenylboronic acid at Step (i) in Reference Example 12.

**[Table 15]**

| Reference Example | Starting material C | Chemical Structural Formula | Instrumental Analysis Data |
|---|---|---|---|
| 13 | 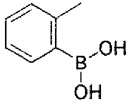 | 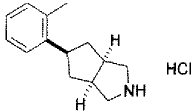 | LCMS: [M+H]+/Rt (min): 202/1.409 (Method B) |
| 14 | 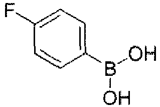 | 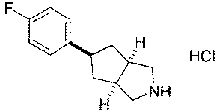 | LCMS: [M+H]+/Rt (min): 206f 1.423 (Method B) |
| 15 | 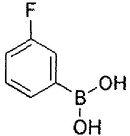 | 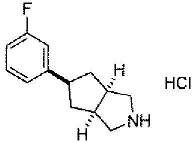 | LCMS: [M+H]+/Rt (min): 206/1.448 (Method B) |
| 16 | 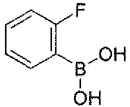 | 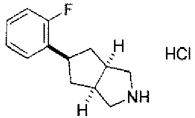 | LCMS: [M+H]+/Rt (min): 206/1.454 (Method B) |

Reference Example 13: (3aR,5r,6aS)-5-(2-methylphenyl)octahydrocyclopenta[c]pyrrole monohydrochloride
Reference Example 14: (3aR,5r,6aS)-5-(4-fluorophenyl)octahydrocyclopenta[c]pyrrole monohydrochloride
Reference Example 15: (3aR,5r,6aS)-5-(3-fluorophenyl)octahydrocyclopenta[c]pyrrole monohydrochloride
Reference Example 16: (3aR,5r,6aS)-5-(2-fluorophenyl)octahydrocyclopenta[c]pyrrole monohydrochloride

### Reference Example 17:

### (3aR,6aS)-2-(2-Fluorophenyl)octahydropyrrolo[3,4-c]pyrrolemonohydrochloride

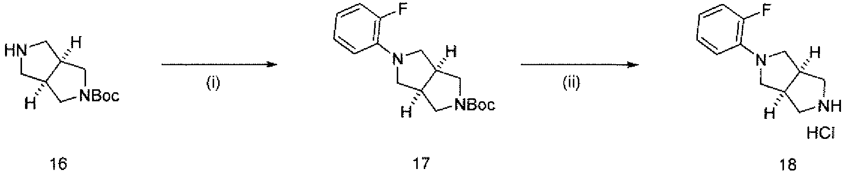

### Step (i):

To a solution of Compound 16 (413 mg) in toluene (6.8 mL) were added 1-bromo-2-fluorobenzene (309 mg), *tert*-butoxysodium (424 mg), tris(dibenzylideneacetone)dipalladium (0) (129 mg) and di-*tert*-butyl 2-biphenylphosphine (42.2 mg) at room temperature, and the mixture was stirred at 90°C for 30 minutes. The mixture was cooled to room temperature, water was added to the reaction mixture, and the mixture was extracted with ethyl acetate. The organic layer was dried over anhydrous sodium sulfate and concentrated *in vacuo.* The resulting residue was then purified by silica gel column chromatography (Eluate: hexane/ethyl acetate) to give Compound 17 (270 mg).
LCMS: [M+H]⁺/Rt(min): 307/2.15 (Method B)

### Step (ii):

To a solution of Compound 17 (305 mg) in chloroform (4 ml) was added hydrochloric acid (4M solution in cyclopentylmethyl, 1.2 ml) and the mixture was stirred at room temperature for 2.5 hours. The reaction solution was then concentrated to give the title compound 18 (287 mg).
LCMS: [M+H]⁺/Rt(min): 207/1.28 (Method B)

### Reference Example 18:

### (3aR,6aS)-2-(3-Fluorophenyl)octahydropyrrolo[3,4-c]pyrrole monohydrochloride

The compound of Reference Example 18 shown in the table below was synthesized according to the process in the above Reference Example 17 by using 1-bromo-3-fluorobenzene instead of 1-bromo-2-fluorobenzene at Step (i) in Reference Example 17.

**[Table 16]**

| Reference Example | Chemical Structural Formula | Instrumental Analysis Data |
|---|---|---|
| 18 | 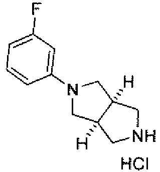 | LCMS: [M+H]⁺/Rt(min): 207/1.34 (Method B) |

### Reference Example 19:

### (3aR,5r,6aS)-5-(3-Methylphenyl)octahydrocyclopenta[c]pyrrole monohydrochloride

The compound of Reference Example 19 shown in the table below was synthesized according to the process in the above Reference Example 12 by using 3-methylphenylboronic acid instead of 4-methylphenylboronic acid at Step (i) in Reference

### Example 12

**[Table 17]**

| Reference Example | Chemical Structural Formula | Instrumental Analysis Data |
|---|---|---|
| 19 | 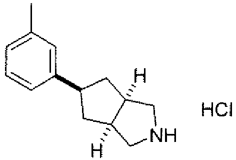 | LCMS: [M+H]⁺/Rt (min): 202/1.57 (Method B) |

### Reference Example 20:

### (3aR,5r,6aS)-5-(Pyridin-2-yl)octahydrocyclopenta[c]pyrrolemonohydrochloride

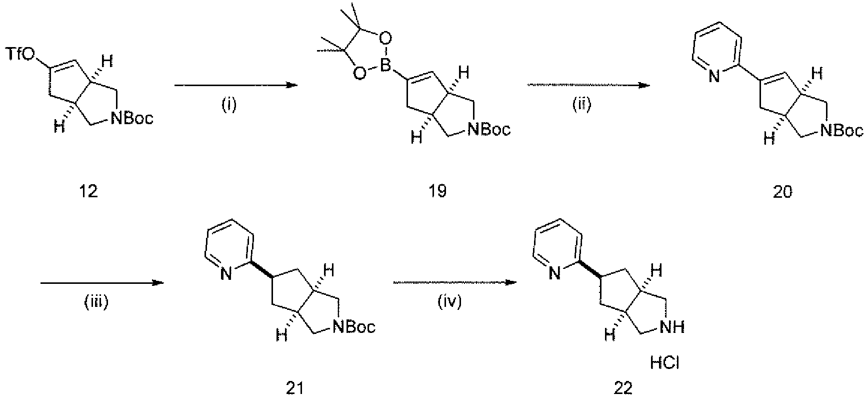

### Step (i):

To a solution of Compound 12 (3.04 g) in 1,4-dioxane (28.4 mL) were added bis(pinacolato)diboron (2.59 g), potassium acetate (2.51 g), [1,1'-bis(diphenylphosphino)ferrocene]dichloropalladium (II) (278 mg) and 1,1'-bis(diphenylphosphino)ferrocene (189 mg) at room temperature, and the mixture was stirred at 80°C for 8 hours. The mixture was cooled to room temperature, and then the reaction mixture was filtrated with Celite and washed with ethyl acetate. Thr filtrate was concentrated *in vacuo.* The resulting residue was then purified by silica gel column chromatography (Eluate: hexane/ethyl acetate) to give Compound 19 (2.82 g).
LCMS: [M+H]⁺/Rt(min): 672/1.158

### Step (ii):

To a solution of Compound 19 (293 mg) in 1,2-dimethoxyethane (3.6 mL) were added 2-bromopyridine (115 mg), aqueous potassium carbonate solution (0.728 ml, 2M) and tetrakistriphenylphosphinepalladium (168 mg) at room temperature, and the mixture was stirred at 80°C for 2 hours. The mixture was cooled to room temperature and the reaction mixture was pufiried by silica gel column chromatography (Eluate: hexane/ethyl acetate) to give Compound 20 (130 mg).
LCMS: [M+H]⁺/Rt(min): 287/1.46 (Method B)

### Step(iii):

Compound 21 was prepared from Compound 20 in the same manner as Step (iii) in Reference Example 12.
LCMS: [M+H]⁺/Rt(min): 289/1.49 (Method B)

### Step (iv):

Compound 22 was prepared from Compound 21 in the same manner as Step (ii) in Reference Example 17.
LCMS: [M+H]⁺/Rt(min): 189/0.778 (Method B)

### Reference Examples 21 and 22:

The compounds of Reference Examples 21 and 22 shown in the table below were synthesized according to the process in the above Reference Example 19 by using each corresponding Starting material D instead of 2-bromopyridine at Step (ii) in Reference Example 19.

**[Table 18]**

| Reference Example | Starting material D | Chemical Structural Formula | Instrumental Analysis Data |
|---|---|---|---|
| 21 | 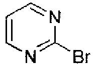 | 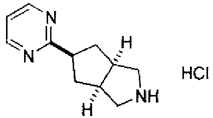 | LCMS: [M+H]+/Rt (min): 190/0.495 (Method B) |
| 22 | 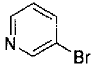 | 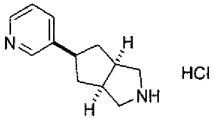 | LCMS: [M+H]+/Rt (min): 189/0.344 (Method B) |

The chemical names of Reference Examples 21 and 22 are described below.
Reference Example 21: (3aR,5r,6aS)-5-(pyrimidin-2-yl)octahydrocyclopenta[c]pyrrole monohydrochloride
Reference Example 22: (3aR,5r,6aS)-5-(pyridin-3-yl)octahydrocyclopenta[c]pyrrole monohydrochloride

### Reference Example 23:

### (3aR,5r,6aS)-5-(5-Methyl-1,2,4-oxadiazol-3-yt)octahydrocyclopenta[c]pyrrole monohydrochloride

The compound of Reference Example 23 shown in the table below was synthesized according to the process in the above Reference Example 4 by using the compound of Reference Example 1 instead of the starting material (Compound 2) at Step (i) and acetyl chloride instead of cyclopropanecarboxylic acid and HATU at Step (ii) in Reference Example 4.

**[Table 19]**

| Reference Example | Chemical Structural Formula | Instrumental Analysis Data |
|---|---|---|
| 23 | 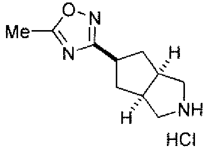 | LCMS: [M+H]+/Rt(min): 19410.499 |

### Test Example 1: Measurement of agonist activity for orexin receptor type 2

Human orexin receptor type 2 and apoaequorin were transiently expressed in CHO cells, and the agonist activity was evaluated based on intracellular calcium mobilization with ligand stimulation. The CHO cells transiently-expressed human orexin receptor type 2 and apoaequorin were seeded on a 384-well plate by 2,000 cells/well, and then incubated for 16 - 22 hours. After the plate was returned to room temperature, Coelenterazine hep (final concentration: 1 µM) was added to the plate, and the plate was allowed to stand at room temperature for 2 hours. And then, Orexin A or each test compound was added to the plate, and the luminescence of the cells was measured with FDSS7000 (Hamamatsu Photonics K.K.), wherein Orexin A and each test compound were dissolved in DMSO (final concentration: 0.1 %), and diluted with a buffer (Hanks, 20 mM HEPES, 0.1 % BSA). The agonist activity of each test compound for orexin receptor type 2 was calculated as relative percentage of luminescence for the luminescence (100 %) of Orexin A (100 pM).

### Test Result:

The results that each compound obtained in Examples was evaluated about the agonist activity for orexin receptor type 2 showed that the present compounds had agonist activity for orexin receptor type 2. Each agonist activity of the compounds obtained in Examples (10 µM) for orexin receptor type 2 is shown in the table below as relative percentage of luminescence for the luminescence (100 %) of Orexin A (100 pM).

**[Table 20]**

| Example | Agonist Activity (%) | Example | Agonist Activity (%) |
|---|---|---|---|
| 1 | 375 | 12 | 492 |
| 2 | 275 | 13 | 466 |
| 3 | 217 | 14 | 446 |
| 4 | 15 | 15 | 511 |
| 5 | 79 | 16 | 491 |
| 6 | 282 | 17 | 315 |
| 7 | 328 | | |
| 8 | 423 | | |
| 9 | 424 | | |
| 10 | 450 | | |
| 11 | 477 | | |

### Test Example 2: Measurement of agonist activity for orexin receptor type 2

Human orexin receptor type 2 and apoaequorin were transiently expressed in CHO cells, and the agonist activity was evaluated based on intracellular calcium mobilization with ligand stimulation. The CHO cells transiently-expressed human orexin receptor type 2 and apoaequorin were seeded on a 384-well plate by 2,000 cells/well, and then incubated for 16 - 22 hours. After the plate was returned to room temperature, Coelenterazine hcp (final concentration: 1 µM) was added to the plate, and the plate was allowed to stand at room temperature for 2 hours. And then, Orexin A (PEPTIDE INSTITUTE, INC., Lot. 711101) or each test compound was added to the plate, and the luminescence of the cells was measured with FDSS7000 (Hamamatsu Photonics K.K.), wherein Orexin A and each test compound were dissolved in DMSO (final concentration: 0.1 %), and diluted with a buffer (Hanks, 20 mM HEPES, 0.1 % BSA). The agonist activity of each test compound for orexin receptor type 2 was calculated as relative percentage of luminescence for the luminescence (100 %) of Orexin A (100 pM).

### Test Result:

The results that each compound obtained in Examples was evaluated about the agonist activity for orexin receptor type 2 showed that the present compounds had agonist activity for orexin receptor type 2. Each agonist activity of the compounds obtained in Examples (10 µM) for orexin receptor type 2 is shown in the table below as relative percentage of luminescence for the luminescence (100 %) of Orexin A (100 pM).

**[Table 21]**

| Example | Agonist Activity (%) | Example | Agonist Activity (%) |
|---|---|---|---|
| 18 | 374 | 27 | 348 |
| 19 | 390 | 28 | 400 |
| 20 | 345 | 29 | 393 |
| 21 | 368 | 30 | 374 |
| 22 | 399 | 31 | 252 |
| 23 | 404 | 32 | 345 |
| 24 | 414 | 33 | 343 |
| 25 | 396 | 34 | 341 |
| 26 | 345 | | |

### INDUSTRIAL APPLICABILITY

The compounds of the present invention exhibit a potent agonist activity for orexin receptor, thereby they are useful as a medicament for treating or preventing a disease such as narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body.

## Claims

1. A compound of formula (1): 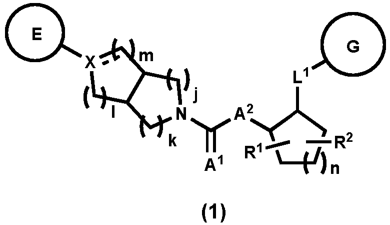 or a pharmaceutically acceptable salt thereof,
wherein
R¹ and R² are each independently hydrogen atom, halogen atom, cyano, - (C=O)NR³R⁴, carboxy group, -(C=O)O-R⁵, optionally-substituted C₁₋₄ alkyl, or optionally-substituted C₁₋₄ alkoxy, and R¹ and R² may bind to the same carbon atom if chemically possible, or when R¹ and R² bind to separate carbon atoms on the ring, they may be taken together via C₁-₆ alkylene to form a fused ring or a bridged ring;
R³ and R⁴ are each independently hydrogen atom or optionally-substituted C₁₋₄ alkyl;
R⁵ is optionally-substituted C₁₋₄ alkyl;
-L¹-ring G is -ring G, -CH₂-ring G (wherein the CH₂ may optionally be substituted with the same or different one or more C₁₋₆ alkyl groups), -NR⁶-ring G, - C(=O)-ring G, -OC(=O)-ring G, -SO-ring G, -SO₂-ring G, -S-ring G, or -O-ring G;
R⁶ is hydrogen atom or optionally-substituted C₁₋₄ alkyl;
n is an integer of 1 or 2;
ring G is optionally-substituted C₆₋₁₀ aromatic carbocyclyl group, optionally-substituted 5- to 10-membered aromatic heterocyclyl group, optionally-substituted C₃₋₆ saturated carbocyclyl group, or optionally-substituted 4- to 10-membered saturated heterocyclyl group;
A¹ is oxygen atom or sulfur atom;
A² is oxygen atom or -NH-;
j, k, l, and m are each independently an integer of 0, 1, or 2;
dashed bond is single bond or double bond;
X is -CR⁷-, nitrogen atom, or carbon atom;
R⁷ is hydrogen atom, hydroxy group, halogen atom, cyano, or optionally-substituted C₁₋₄ alkyl; and
ring E is the following formula (1a-1), (1a-2), or (1a-3): 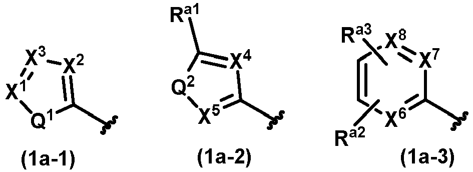 wherein
X¹ to X⁸ are each independently nitrogen atom or -CR^{a4}-;
Q¹ and Q² are oxygen atom or -NR^{a5}-;
R^{a1} to R^{a5} are each independently, (if there are plural CR^{a 4}, each R^{a 4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are CR^{a4}, two R^{a4} may be combined with the carbon atoms to which they are attached to form a 6-membered carbocyclic ring fused to a 5-membered ring formed by X¹, X² and X³.

2. The compound according to claim 1 or a pharmaceutically acceptable salt thereof, wherein in R¹ to R⁷, the optional substituent of "optionally-substituted C₁₋₄ alkyl" is each independently the same or different one or more substituents selected from halogen atom, hydroxy group, C₁₋₄ alkoxy, or C₃₋₇ cycloalkyl, and the optional substituent of "optionally-substituted C₁₋₄ alkoxy" is each independently the same or different one or more substituents selected from halogen atom, hydroxy group, or C₃₋₇ cycloalkyl,
in ring G, the optional substituent of C₆₋₁₀ aromatic carbocyclyl group, 5- to 10-membered aromatic heterocyclyl group, C₃₋₆ saturated carbocyclyl group, and 4- to 10-membered saturated heterocyclyl group is each independently one or more substituents selected from the group consisting of halogen atom, C₁₋₆ alkyl (wherein the alkyl may be optionally substituted with the same or different one or more substituents selected from halogen atom, hydroxy group, C₁₋₄ alkoxy, or C₃₋₇ cycloalkyl), C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, C₁₋₄ alkoxy, or C₃₋₇ cycloalkyl), C₁₋₄ alkoxy (wherein the alkoxy may be optionally substituted with the same or different one or more substituents selected from halogen atom, hydroxy group, or C₃₋₇ cycloalkyl), C₃₋₇ cycloalkyl (wherein the cycloalkyl may be optionally substituted with the same or different one or more substituents selected from halogen atom, hydroxy group, C₁₋₄ alkyl, C₁₋₄ alkoxy, or C₃₋₇ cycloalkyl), and C₃₋₇ cycloalkoxy (wherein the cycloalkoxy may be optionally substituted with the same or different one or more substituents selected from halogen atom, hydroxy group, C₁₋₄ alkyl, C₁₋₄ alkoxy, or C₃₋₇ cycloalkyl), or when there are plural optional substituents, two of them may be taken together via C₁₋₆ alkylene to form a chemically-possible bicyclic structure selected from a fused ring, a spiro ring, or a bridged ring.

3. The compound according to claim 1 or 2 or a pharmaceutically acceptable salt thereof, wherein in R¹ to R⁷, the optional substituent of "optionally-substituted C₁₋₄ alkyl" is each independently the same or different one or more substituents selected from halogen atom or C₁₋₄ alkoxy, and the optional substituent of "optionally-substituted C₁₋₄ alkoxy" is each independently the same or different one or more halogen atoms,
in ring G, the optional substituent of C₆₋₁₀ aromatic carbocyclyl group, 5- to 10-membered aromatic heterocyclyl group, C₃₋₆ saturated carbocyclyl group, and 4- to 10-membered saturated heterocyclyl group is each independently one or more substituents selected from the group consisting of halogen atom, C₁₋₆ alkyl (wherein the alkyl may be optionally substituted with the same or different one or more substituents selected from halogen atom or C₁₋₄ alkoxy), C₆₋₁₀ aromatic carbocyclyl group (wherein the C₆₋₁₀ aromatic carbocyclyl group may be optionally substituted with the same or different one or more substituents selected from halogen atom, C₁₋₄ alkyl, or C₁₋₄ alkoxy), C₁₋₄ alkoxy (wherein the alkoxy may be optionally substituted with the same or different one or more halogen atoms), C₃₋₇ cycloalkyl (wherein the cycloalkyl may be optionally substituted with the same or different one or more substituents selected from halogen atom, C₁₋₄ alkyl, or C₁₋₄ alkoxy), and C₃₋₇ cycloalkoxy (wherein the cycloalkoxy may be optionally substituted with the same or different one or more substituents selected from halogen atom, C₁₋₄ alkyl, or C₁₋₄ alkoxy), or when there are plural optional substituents, two of them may be taken together via C₁₋₆ alkylene to form a chemically-possible bicyclic structure selected from a fused ring, a spiro ring, or a bridged ring.

4. The compound according to any one of claims 1 to 3 or a pharmaceutically acceptable salt thereof, wherein ring E is the following formula (1a-1-1), (1a-2-1), (1a-3-1), (1a-3-2), (1a-3-3), or (1a-3-4): 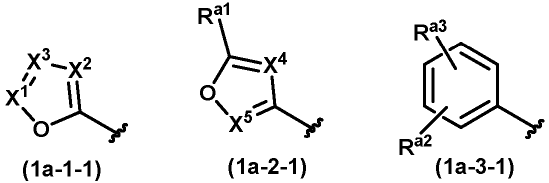 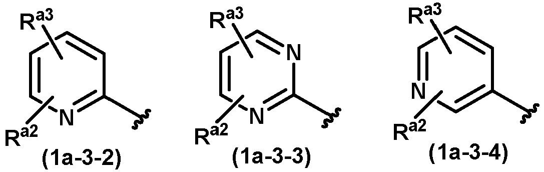 wherein
X¹ to X⁵ are each independently nitrogen atom or -CR^{a4}-; and
R^{a1} to R^{a4} are each independently, (if there are plural CR^{a 4}, each R^{a 4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a 6-membered carbocyclic ring fused to a 5-membered ring formed by X¹, X² and X³.

5. The compound according to any one of claims 1 to 4 or a pharmaceutically acceptable salt thereof, wherein ring G is the following formula (1b-1) or (1b-2): 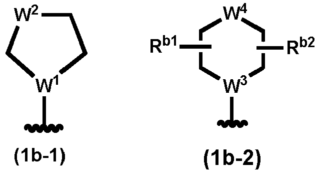 wherein
W¹ and W³ are each independently nitrogen atom or -CR^{b3}-;
W² and W⁴ are each independently -NR^{b4}-, oxygen atom, or -CR^{b5}R^{b6}-;
R^{b1} to R^{b6} are each independently hydrogen atom, -N(R^{b7})R^{b8}, C₁₋₆ alkyl (wherein the alkyl may optionally substituted with the same or different one or more substituents selected from halogen atom, C₃₋₆ cycloalkyl (wherein the cycloalkyl may be optionally substituted with the same or different one or more substituents selected from halogen atom or C₁₋₄ alkyl wherein the C₁₋₄ alkyl may be optionally substituted with halogen atom(s)), or C₁₋₄ alkoxy), C₆₋₁₀ aromatic carbocyclyl group (wherein the C₆₋₁₀ aromatic carbocyclyl group may be optionally substituted with the same or different one or more substituents selected from halogen atom, C₁₋₄ alkyl, or C₁₋₄ alkoxy), C₁₋₄ alkoxy (wherein the alkoxy may be optionally substituted with the same or different one or more halogen atoms), C₃₋₇ cycloalkyl (wherein the cycloalkyl may be optionally substituted with the same or different one or more substituents selected from halogen atom, C₁₋₄ alkyl, or C₁₋₄ alkoxy), or C₃₋₇ cycloalkoxy (wherein the cycloalkoxy may be optionally substituted with the same or different one or more substituents selected from halogen atom, C₁₋₄ alkyl, or C₁₋₄ alkoxy), and R^{b1} and R^{b2} may bind to the same carbon atom if chemically possible;
wherein R^{b1} and R^{b2} may be taken together via C₁-₆ alkylene to form a chemically-possible bicyclic structure selected from a fused ring, a spiro ring, or a bridged ring;
R^{b7} and R^{b8} are each independently hydrogen atom, C₁₋₆ alkyl (wherein the alkyl may be optionally substituted with the same or different one or more substituents selected from halogen atom, C₁₋₄ alkoxy, C₃₋₇ cycloalkyl (wherein the cycloalkyl may be optionally substituted with one or more substituents selected from halogen atom or C₁₋₄ alkyl wherein the C₁₋₄ alkyl may be optionally substituted with halogen atom(s)), or 5- to 10-membered aromatic heterocyclyl group), 5- to 10-membered aromatic heterocyclyl group (wherein the aromatic heterocyclyl group may be optionally substituted with the same or different one or more substituents selected from halogen atom, C₁₋₄ alkyl, or C₁₋₄ alkoxy), or C₃₋₇ cycloalkyl (wherein cycloalkyl may be optionally substituted with the same or different one or more substituents selected from halogen atom, hydroxy group, C₁₋₄ alkyl, or C₁₋₄ alkoxy),
wherein when R^{b1} to R^{b6} are -N(R^{b7})R^{b8}, R^{b7} and R^{b8} may be combined with the nitrogen atom to which they are attached to form a 3- to 7-membermed nitrogen-containing saturated heterocycle.

6. The compound according to any one of claims 1 to 5 or a pharmaceutically acceptable salt thereof, wherein the formula is formula (2): 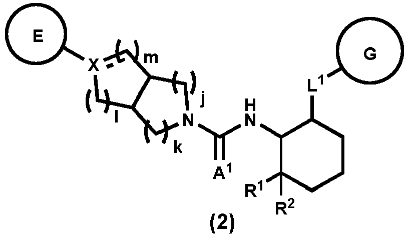 wherein
R¹ and R² are each independently hydrogen atom, halogen atom, C₁₋₄ alkyl (wherein the alkyl may be optionally substituted with the same or different one or more substituents selected from halogen atom, hydroxy group, C₁₋₄ alkoxy, or C₃₋₇ cycloalkyl), or C₁₋₄ alkoxy (wherein the alkoxy may be optionally substituted with the same or different one or more substituents selected from halogen atom, hydroxy group, or C₃₋₇ cycloalkyl);
-L¹-ring G is -ring G, -CH₂-ring G (wherein the CH₂ may be optionally substituted with the same or different one or more C₁₋₆ alkyl groups), -NR⁶-ring G, - C(=O)-ring G, -OC(=O)-ring G, -SO-ring G, -SO₂-ring G, -S-ring G, or -O-ring G;
R⁶ is each independently hydrogen atom or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring G is the following formula (1b-1) or (1b-2): 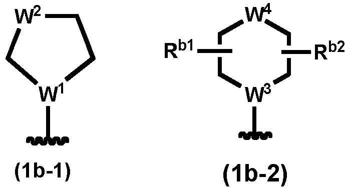 wherein
W¹ and W³ are each independently nitrogen atom or -CR^{b3}-;
W² and W⁴ are each independently -NR^{b4}-, oxygen atom, or -CR^{b5}R^{b6}-;
R^{b1} to R^{b6} are each independently hydrogen atom, -N(R^{b7})R^{b8}, C₁₋₆ alkyl (wherein the alkyl may be optionally substituted with the same or different one or more substituents selected from halogen atom, C₃₋₆ cycloalkyl (wherein the cycloalkyl may be optionally substituted with the same or different one or more substituents selected from halogen atom or C₁₋₄ alkyl wherein the C₁₋₄ alkyl may be optionally substituted with halogen atom(s)), or C₁₋₄ alkoxy), C₆₋₁₀ aromatic carbocyclyl group (wherein the C₆₋₁₀ aromatic carbocyclyl group may be optionally substituted with the same or different one or more substituents selected from halogen atom, C₁₋₄ alkyl, or C₁₋₄ alkoxy), C₁₋₄ alkoxy (wherein the alkoxy may be optionally substituted with the same or different one or more halogen atom(s)), C₃₋₇ cycloalkyl (wherein the cycloalkyl may be optionally substituted with one or more substituents selected from halogen atom, C₁₋₄ alkyl, or C₁₋₄ alkoxy), or C₃₋₇ cycloalkoxy (wherein the cycloalkoxy may be optionally substituted with the same or different one or more substituents selected from halogen atom, C₁₋₄ alkyl, or C₁₋₄ alkoxy), and R^{b1} and R^{b2} may bind to the same carbon atom if chemically possible;
wherein R^{b1} and R^{b2} may be taken together via C₁₋₆ alkylene to form a chemically-possible bicyclic structure selected from a fused ring, a spiro ring, or a bridged ring;
R^{b7} and R^{b8} are each independently hydrogen atom, C₁₋₆ alkyl (wherein the alkyl may be optionally substituted with the same or different one or more substituents selected from halogen atom, C₁₋₄ alkoxy, C₃₋₇ cycloalkyl (wherein the cycloalkyl may be optionally substituted with the same or different one or more substituents selected from halogen atom or C₁₋₄ alkyl wherein C₁₋₄ alkyl may be optionally substituted with halogen atom(s)), or 5- to 10-membered aromatic heterocyclyl group), 5- to 10-membered aromatic heterocyclyl group (wherein the aromatic heterocyclyl group may be optionally substituted with the same or different one or more substituents selected from halogen atom, C₁₋₄ alkyl, or C₁₋₄ alkoxy), or C₃₋₇ cycloalkyl (wherein the cycloalkyl may be optionally substituted with the same or different one or more substituents selected from halogen atom, hydroxy group, C₁₋₄ alkyl, or C₁₋₄ alkoxy),
wherein when R^{b1} to R^{b6} are -N(R^{b7})R^{b8}, R^{b7} and R^{b8} may be combined with the nitrogen atom to which they are attached to form a 3- to 7-membered nitrogen-containing saturated heterocycle;
A¹ is oxygen atom or sulfur atom;
j, k, l, and m are each independently an integer of 0, 1, or 2;
dashed bond is single bond or double bond;
X is -CR⁷-, nitrogen atom, or carbon atom;
R⁷ is hydrogen atom, hydroxy group, halogen atom, cyano, or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring E is the following formula (1a-1-1), (1a-2-1), (1a-3-1), (1a-3-2), (1a-3-3), or (la-3-4): 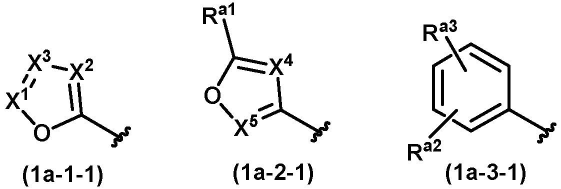 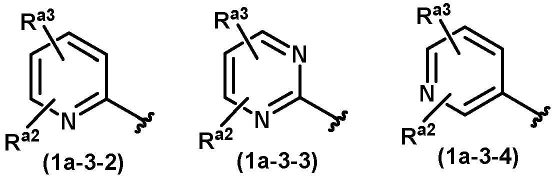 wherein
X¹ to X⁵ are each independently nitrogen atom or -CR^{a4}-;
R^{a1} to R^{a4} are each independently, (if there are plural CR^{a 4}, each R^{a 4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, C₁₋₄ alkyl, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5-to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a 6-membered carbocyclic ring fused to a 5-membered ring formed by X¹, X² and X³.

7. The compound according to claim 6 or a pharmaceutically acceptable salt thereof, wherein R¹ and R² are each independently hydrogen atom, halogen atom, or C₁₋₄ alkyl (wherein the alkyl may be optionally substituted with the same or different one or more substituents selected from halogen atom, hydroxy group, C₁₋₄ alkoxy, or C₃₋₇ cycloalkyl);
L¹ is single bond or oxygen atom;
ring G is the following formula (1b-1-1), (1b-2-1), (1b-1-2), or (1b-2-2): 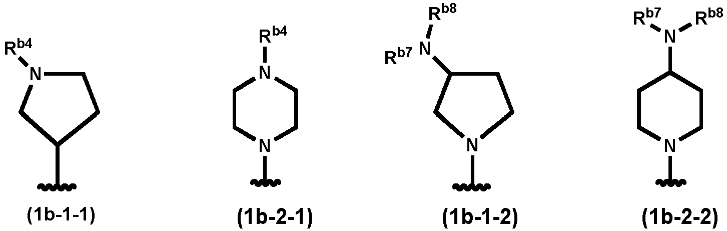
wherein R^{b4}, R^{b7}, and R^{b8} are each independently hydrogen atom or C₁₋₆ alkyl (wherein the alkyl may be optionally substituted with the same or different one or more substituents selected from halogen atom or C₁₋₄ alkoxy).

8. The compound according to any one of claims 1 to 7 or a pharmaceutically acceptable salt thereof, wherein the formula is formula (3): 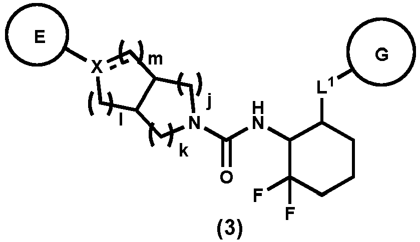 wherein
L¹ is single bond or oxygen atom;
ring G is the following formula (1b-1-1), (1b-2-1), (1b-1-2), or (1b-2-2): 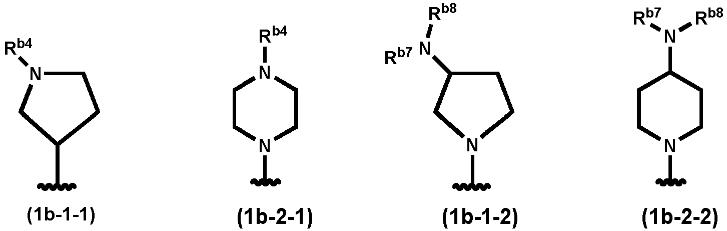 wherein R^{b4}, R^{b7}, and R^{b8} are each independently hydrogen atom or C₁₋₆ alkyl (wherein the alkyl may be optionally substituted with the same or different one or more substituents selected from halogen atom or C₁₋₄ alkoxy);
j, k, l, and m are each independently an integer of 0, 1, or 2;
dashed bond is single bond or double bond;
X is -CR⁷-, nitrogen atom, or carbon atom;
R⁷ is hydrogen atom, hydroxy group, halogen atom, cyano, or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring E is the following formula (1a-1-1), (1a-2-1), (1a-3-1), (1a-3-2), (1a-3-3), or (la-3-4): 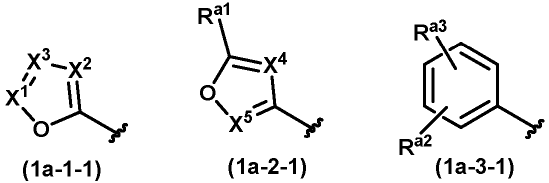 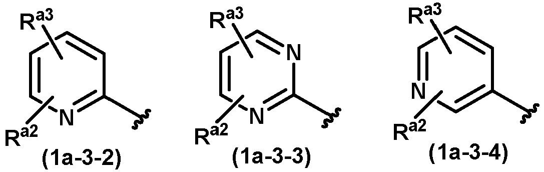 wherein
X¹ to X⁵ are each independently nitrogen atom or -CR^{a4}-;
R^{a1} to R^{a4} are each independently, (if there are plural CR^{a 4}, each R^{a 4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a 6-membered carbocyclic ring fused to a 5-membered ring formed by X¹, X² and X³.

9. The compound according to any one of claims 1 to 8 or a pharmaceutically acceptable salt thereof, wherein the formula is formula (4): 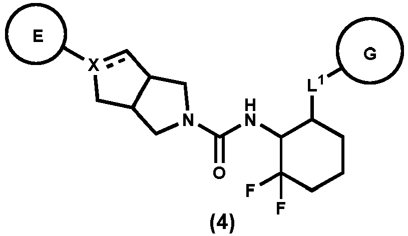 wherein
L¹ is single bond or oxygen atom;
ring G is the following formula (1b-1-1), (1b-2-1), (1b-1-2), or (1b-2-2): 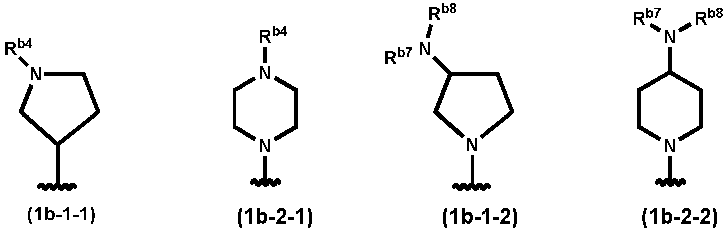 wherein R^{b4}, R^{b7}, and R^{b8} are each independently hydrogen atom or C₁₋₆ alkyl (wherein the alkyl may be optionally substituted with the same or different one or more substituents selected from halogen atom or C₁₋₄ alkoxy);
dashed bond is single bond or double bond;
X is -CR⁷-, nitrogen atom, or carbon atom;
R⁷ is hydrogen atom or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring E is the following formula (1a-1-1), (1a-2-1), (1a-3-1), (1a-3-2), (1a-3-3), or (1a-3-4): 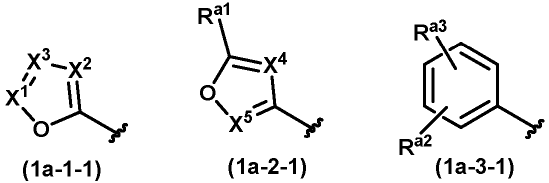 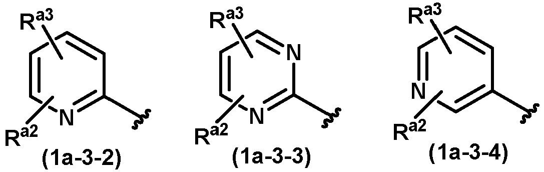 wherein
X¹ to X⁵ are each independently nitrogen atom or -CR^{a4}-;
R^{a1} to R^{a4} are each independently, (if there are plural CR^{a 4}, each R^{a 4} is also independently), hydrogen atom, halogen atom, C₆₋₁₀ aromatic carbocyclyl group (wherein the C₆₋₁₀ aromatic carbocyclyl group may be optionally substituted with the same or different one or more substituents selected from halogen atom, hydroxy group, C₁₋₄ alkyl, or C₁₋₄ alkoxy), C₁₋₄ alkyl (wherein the alkyl may be optionally substituted with the same or different one or more substituents selected from halogen atom, hydroxy group, or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), cyano, C₁₋₄ alkoxy (wherein the alkoxy may be optionally substituted with the same or different one or more substituents selected from halogen atom, hydroxy group, or C₁₋₄ alkoxy), C₃₋₇ cycloalkoxy (wherein the cycloalkoxy may be optionally substituted with the same or different one or more substituents selected from halogen atom, hydroxy group, C₁₋₄ alkyl, or C₁₋₄ alkoxy), or 5- to 10-membered aromatic heterocyclyl group, and R^{a2} and R^{a3} may bind to the same carbon atom if chemically possible; provided that when both X¹ and X³ are -CR^{a4}-, two R^{a4} may be combined with the carbon atoms to which they are attached to form a 6-membered carbocyclic ring fused to a 5-membered ring formed by X¹, X² and X³.

10. The compound according to claim 9 or a pharmaceutically acceptable salt thereof, wherein ring E is the above formula (1a-2-1) or (1a-3-1) wherein X⁴ to X⁵ are each independently nitrogen atom or -CR^{a4}-; and R^{a1} to R^{a4} are each independently, (if there are plural CR^{a 4}, each R^{a 4} is also independently), hydrogen atom, halogen atom, C₁₋₄ alkyl (wherein the alkyl may be optionally substituted with the same or different one or more substituents selected from halogen atom or C₁₋₄ alkoxy), C₃₋₇ cycloalkyl (wherein the cycloalkyl may be optionally substituted with the same or different one or more substituents selected from halogen atom, hydroxy group, C₁₋₄ alkyl, or C₁₋₄ alkoxy), or C₁₋₄ alkoxy (wherein the alkoxy may be optionally substituted with the same or different one or more halogen atom(s)).

11. The compound according to claim 9 or a pharmaceutically acceptable salt thereof, wherein ring E is the following formula (1a-2-2) or (1a-3-1): 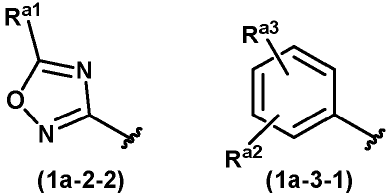 wherein R^{a1} to R^{a3} are each independently hydrogen atom, halogen atom, C₁₋₄ alkyl (wherein the alkyl may be optionally substituted with the same or different one or more substituents selected from halogen atom or C₁₋₄ alkoxy), or C₃₋₇ cycloalkyl (wherein the cycloalkyl may be optionally substituted with the same or different one or more substituents selected from halogen atom, hydroxy group, C₁₋₄ alkyl, or C₁₋₄ alkoxy).

12. The compound according to any one of claims 1 to 11 or a pharmaceutically acceptable salt thereof, wherein the formula is formula (5): 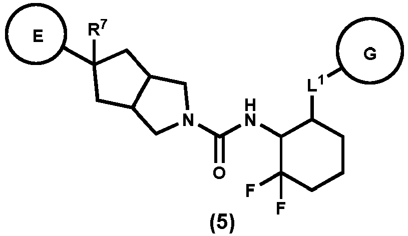 wherein
L¹ is single bond or oxygen atom;
ring G is the following formula (1b-1-1) or (1b-2-1): 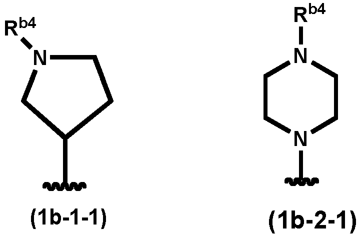 wherein R^{b4} is hydrogen atom or C₁₋₆ alkyl (wherein the alkyl may be optionally substituted with the same or different one or more substituents selected from halogen atom or C₁₋₄ alkoxy);
R⁷ is hydrogen atom or C₁₋₄ alkyl (wherein the alkyl may be optionally substituted with the same or different one or more substituents selected from halogen atom, hydroxy group, C₁₋₄ alkoxy, or C₃₋₇ cycloalkyl);
ring E is the following formula (1a-2-2) or (1a-3-1): 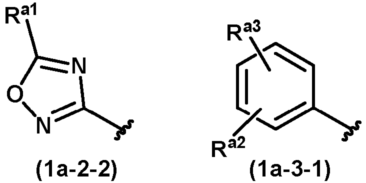 wherein R^{a1} to R^{a3} are each independently hydrogen atom, halogen atom, C₁₋₄ alkyl (wherein the alkyl may be optionally substituted with the same or different one or more substituents selected from halogen atom or C₁₋₄ alkoxy), or C₃₋₇ cycloalkyl (wherein the cycloalkyl may be optionally substituted with the same or different one or more substituents selected from halogen atom, hydroxy group, C₁₋₄ alkyl, or C₁₋₄ alkoxy).

13. The compound according to claim 12 or a pharmaceutically acceptable salt thereof, wherein the ring G is the above formula (1b-1-1) and R^{b4} is C₁₋₄ alkyl which may be optionally substituted with the same or different one or more halogen atom(s).

14. The compound according to claim 12 or a pharmaceutically acceptable salt thereof, wherein the ring G is the above formula (1b-2-1) and R^{b4} is hydrogen atom or C₁₋₄ alkyl wherein the alkyl may be optionally substituted with the same or different one or more substituents selected from halogen atom or C₁₋₄ alkoxy.

15. The compound according to claim 12 or a pharmaceutically acceptable salt thereof, wherein R⁷ is hydrogen atom.

16. The compound according to claim 1 selected from the following compounds:
(3aR,5S,6aS)-5-(5-cyclopropyl-1,2,4-oxadiazol-3-yl)-N-{(1R,6S)-2,2-difluoro-6-[4-(propan-2-yl)piperazin-1-yl]cyclohexyl}hexahydropenta[c]pyrrole-2(1H)-carboxamide,
(3aR,5S,6aS)-N-{(1R,6S)-2,2-difluoro-6-[4-(propan-2-yl)piperazin-1-yl]cyclohexyl}-5-{5-[(1S,2S)-2-fluorocyclopropyl]-1,2,4-oxadiazol-3-yl}hexahydropenta[c]pyrrole-2(1H)-carboxamide,
(1R,5S,6R)-6-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[3.1.0]hexane-3-carboxamide,
(1R,5S,6S)-6-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[3.1.0]hexane-3-carboxamide,
(3aR,5R,6aS)-5-(5-cyclopropyl-1,2,4-oxadiazol-3-yl)-N-{(1R,6S)-2,2-difluoro-6-[4-(propan-2-yl)piperazin-1-yl]cyclohexyl}-5-methylhexahydropenta[c]pyrrole-2(1H)-carboxamide,
(3aR,5R,6aS)-N- f (1R,6S)-2,2-difluoro-6-[4-(propan-2-yl)piperazin-1-yl]cyclohexyl}-5-{5-[(1S,2S)-2-fluorocyclopropyl]-1,2,4-oxadiazol-3-yl}-5-methylhexahydropenta[c]pyrrole-2(1H)-carboxamide,
(3aR,5R,6aS)-5-(5-cyclopropyl-1,2,4-oxadiazol-3-yl)-N-{(1R,6S)-2,2-difluoro-6-[4-(propan-2-yl)piperazin-1-yl]cyclohexyl}hexahydropenta[c]pyrrole-2(1H)-carboxamide,
(3aR,5R,6aS)-N- f (1R,6S)-2,2-difluoro-6-[4-(propan-2-yl)piperazin-1-yl]cyclohexyl}-5-phenylhexahydropenta[c]pyrrole-2(1H)-carboxamide,
(3aR,6aS)-N- f (1R,6S)-2,2-difluoro-6-[4-(propan-2-yl)piperazin-1-yl]cyclohexyl}-5-phenylhexahydropyrrolo[3,4-c]pyrrole-2(1H)-carboxamide,
(3aR,5S,6aS)-N-{(1R,6S)-2,2-difluoro-6-[4-(propan-2-yl)piperazin-1-yl]cyclohexyl}-5-phenylhexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(4-methylphenyl)hexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(2-methylphenyl)hexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(4-fluorophenyl)hexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(3-fluorophenyl)hexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(2-fluorophenyl)hexahydropenta[c]pyrrole-2(1H)-carboxamide,
(1S,6R,8S)-8-(5-cyclopropyl-1,2,4-oxadiazol-3-yl)-N- f (1R,6S)-2,2-difluoro-6-[4-(propan-2-yl)piperazin-1-yl]cyclohexyl}-3-azabicyclo[4.2.0]octane-3-carboxamide,
(1R,6S,8R)-8-(5-cyclopropyl-1,2,4-oxadiazol-3-yl)-N-{(1R,6S)-2,2-difluoro-6-[4-(propan-2-yl)piperazin-1-yl]cyclohexyl}-3-azabicyclo[4.2.0]octane-3-carboxamide,
(3aS,6aR)-N-{(1R,6S)-2,2-difluoro-6-[4-(propan-2-yl)piperazin-1-yl]cyclohexyl}-5-phenyl-3,3a,4,6a-tetrahydropenta[c]pyrrole-2(1H)-carboxamide,
(3aR,6aS)-N-{(1R,6S)-2,2-difluoro-6-[4-(propan-2-yl)piperazin-1-yl]cyclohexyl}-5-phenyl-3,3a,4,6a-tetrahydropenta[c]pyrrole-2(1H)-carboxamide,
(3aR,6aS)-N-{(1R,6S)-2,2-difluoro-6-[4-(propan-2-yl)piperazin-1-yl]cyclohexyl}-5-(4-fluorophenyl)hexahydropyrrolo[3,4-c]pyrrole-2(1H)-carboxamide,
(3aR,6aS)-N-{(1R,6S)-2,2-difluoro-6-[4-(propan-2-yl)piperazin-1-yl]cyclohexyl}-5-(2-fluorophenyl)hexahydropyrrolo[3,4-c]pyrrole-2(1H)-carboxamide,
(3aR,6aS)-N-{(1R,6S)-2,2-difluoro-6-[4-(propan-2-yl)piperazin-1-yl]cyclohexyl}-5-(pyridin-2-yl)hexahydropyrrolo[3,4-c]pyrrole-2(1H)-carboxamide,
(3aR,6aS)-N-{(1R,6S)-2,2-difluoro-6-[4-(propan-2-yl)piperazin-1-yl]cyclohexyl}-5-(pyrimidin-2-yl)hexahydropyrrolo[3,4-c]pyrrole-2(1H)-carboxamide,
(3aR,6aS)-N-{(1R,6S)-2,2-difluoro-6-[4-(propan-2-yl)piperazin-1-yl]cyclohexyl}-5-(3-fluorophenyl)hexahydropyrrolo[3,4-c]pyrrole-2(1H)-carboxamide,
(3aR,6aS)-N-{(1R,6S)-2,2-difluoro-6-[4-(propan-2-yl)piperazin-1-yl]cyclohexyl}-5-(4-methylphenyl)hexahydropyrrolo[3,4-c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(3-methylphenyl)hexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(pyridin-2-yl)hexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(pyrimidin-2-yl)hexahydropenta[c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(pyridin-3-yl)hexahydropenta[c]pyrrole-2(1H)-carboxamide,
(3aR,5R,6aS)-N-[(1R,6S)-2,2-difluoro-6-{[(3 S)-1-(propan-2-yl)pyrrolidin-3-yl]oxy}cyclohexyl]-5-phenylhexahydropenta[c]pyrrole-2(1H)-carboxamide,
(3aR,6aS)-N-[(1R,6S)-2,2-difluoro-6-{[(3 S)-1-(propan-2-yl)pyrrolidin-3-yl]oxy}cyclohexyl]-5-phenylhexahydropyrrolo[3,4-c]pyrrole-2(1H)-carboxamide,
(3aR,5R,6aS)-N-{(1R,6S)-2,2-difluoro-6-[4-(propan-2-yl)piperazin-1-yl]cyclohexyl}-5-(5-methyl-1,2,4-oxadiazol-3-yl)hexahydropenta[c]pyrrole-2(1H)-carboxamide,
(3aR,5R,6aS)-N-[(1R,6S)-2,2-difluoro-6- f methyl[(3S)-1-(propan-2-yl)pyrrolidin-3-yl]amino}cyclohexyl]-5-(5-methyl-1,2,4-oxadiazol-3-yl)hexahydropenta[c]pyrrole-2(1H)-carboxamide,
(3aR,5R,6aS)-N-[(1R,6S)-2,2-difluoro-6-{[(3 S)-1-(propan-2-yl)pyrrolidin-3-yl]oxy}cyclohexyl]-5-(5-methyl-1,2,4-oxadiazol-3-yl)hexahydropenta[c]pyrrole-2(1H)-carboxamide,
(3aR,5R,6aS)-N-[(1R,6S)-2,2-difluoro-6- f methyl[(3S)-1-(propan-2-yl)pyrrolidin-3-yl]amino}cyclohexyl]-5-phenylhexahydropenta[c]pyrrole-2(1H)-carboxamide, (3aR,6aS)-N-[(1R,6S)-2,2-difluoro-6-{methyl[(3S)-1-(propan-2-yl)pyrrolidin-3-yl]amino}cyclohexyl]-5-phenylhexahydropyrrolo[3,4-c]pyrrole-2(1H)-carboxamide,
or a pharmaceutically acceptable salt thereof.

17. A pharmaceutical composition comprising the compound according to any one of claims 1 to 16 or a pharmaceutically acceptable salt thereof.

18. A pharmaceutical composition comprising the compound according to any one of claims 1 to 16 or a pharmaceutically acceptable salt thereof for use in the treatment of narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

19. A medicament for treating a disease associated with orexin receptor type 2, comprising the compound according to any one of claims 1 to 16 or a pharmaceutically acceptable salt thereof.

20. A medicament for treating narcolepsy, idiopathic hypersomnia, hypersomnia, sleep apnea syndrome, narcolepsy syndrome involving narcolepsy-like symptom, hypersomnia associated with Parkinson's disease, hypersomnia associated with dementia with Lewy body, hypersomnia syndrome involving daytime hypersomnia (for example, Kleine-Levin syndrome, major depression accompanied by hypersomnia, dementia with Lewy body, Parkinson's disease, progressive supranuclear palsy, Prader-Willi syndrome, Moebius syndrome, hypoventilation syndrome, Niemann-Pick disease type C, brain contusion, cerebral infarction, brain tumor, muscular dystrophy, multiple sclerosis, acute disseminated encephalomyelitis, Guillain-Barre syndrome, Rasmussen's encephalitis, Wernicke's encephalopathy, limbic encephalitis, Hashimoto encephalopathy), coma, loss of consciousness, obesity (for example, malignant mast cell, extrinsic obesity, hyperinsulinar obesity, hyperplasmic obesity, hypophysial obesity, hypoplasmic obesity, hypothyroid obesity, hypothalamic obesity, symptomatic obesity, childhood obesity, upper body obesity, alimentary obesity, gonadal obesity, systemic mastocytosis, primary obesity, central obesity), insulin resistance syndrome, Alzheimer, impaired consciousness such as coma, side effect or complication caused by anesthesia, sleep disturbance, sleep problem, insomnia, intermittent sleep, night myoclonus, REM sleep interruption, jet lag, jet lag syndrome, sleep disorder of shift workers, dyssomnia, sleep terror, depression, major depression, sleepwalking, enuresis, sleep disorder, Alzheimer's sundown syndrome, disease associated with circadian rhythm, fibromyalgia, condition resulting from decrease in sleeping quality, bulimia, obsessive eating disorder, obesity-related diseases, hypertension, diabetes, elevated plasma insulin level/insulin resistance, hyperlipemia, hyperlipidaemia, endometrial cancer, breast cancer, prostate cancer, colon cancer, cancer, osteoarthritis, obstructive sleep apnea, cholelithiasis, gallstone, heart disease, abnormal heartbeat, arrhythmia, myocardial infarction, congestive heart failure, coronary heart disease, cardiovascular disease, sudden death, polycystic ovary, craniopharyngioma, Prader-Willi syndrome, Froehlich syndrome, growth hormone deficiency, normal variant short stature, Turner syndrome, children suffering from acute lymphoblastic leukemia, syndrome X, reproductive hormone abnormality, decrease of fecundability, infertility, hypogonadism in men, sexual/reproductive-function dysfunction such as hirsutism in women, fetal defect associated with maternity obesity, gastrointestinal motility disorder such as obesity-related gastroesophageal reflux, obesity hypoventilation syndrome (Pickwickian syndrome), respiratory disease such as respiratory distress, inflammation such as vascular systemic inflammation, arteriosclerosis, hypercholesterolemia, hyperuricemia, low back pain, gallbladder disease, gout, renal cancer, secondary risk of obesity such as risk of left ventricle hypertrophy, migraine, headache, neuropathic pain, Parkinson's disease, psychosis, schizophrenia, facial flushing, night sweat, disease in genitalia/urinary system, disease associated with sexual function or fecundability, dysthymic disorder, bipolar disorder, bipolar I disorder, bipolar II disorder, cyclothymic disorder, acute stress disorder, agoraphobia, generalized anxiety disorder, obsessive-compulsive disorder, panic attack, panic disorder, posttraumatic stress disorder, separation anxiety disorder, social phobia, anxiety disorder, acute neurological and psychiatric disorder such as cerebral deficiency developed after heart bypass surgery or heart transplant, stroke, ischemic stroke, cerebral ischemia, spinal cord trauma, head injury, periparturient hypoxia, cardiac arrest, hypoglycemic nerve injury, Huntington's disease, amyotrophic lateral sclerosis, multiple sclerosis, eye damage, retinopathy, cognitive impairment, muscle spasm, tremor, epilepsy, disorder associated with muscle spasm, delirium, amnestic disorder, age-associated cognitive decline, schizoaffective disorder, paranoia, drug addiction, movement disorder, chronic fatigue syndrome, fatigue, medication-induced parkinsonian syndrome, Gilles de la Tourette syndrome, chorea, myoclonus, tic, restless legs syndrome, dystonia, dyskinesia, attention deficit hyperactivity disorder (ADHD), conduct disorder, urinary incontinence, withdrawal symptom, trigeminal neuralgia, hearing loss, tinnitus, nerve injury, retinopathy, macular degeneration, vomiting, cerebral edema, pain, bone pain, arthralgia, toothache, cataplexy, or traumatic brain injury, comprising the compound according to any one of claims 1 to 16 or a pharmaceutically acceptable salt thereof.

21. A medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, comprising the compound according to any one of claims 1 to 16 or a pharmaceutically acceptable salt thereof.

22. A medicament for treating narcolepsy, comprising the compound according to any one of claims 1 to 16 or a pharmaceutically acceptable salt thereof.

23. A medicament for treating idiopathic hypersomnia, comprising the compound according to any one of claims 1 to 16 or a pharmaceutically acceptable salt thereof.

24. A method of treating a disease associated with orexin receptor type 2, which comprises administering a therapeutically effective amount of the compound according to any one of claims 1 to 16 or a pharmaceutically acceptable salt thereof to a patient in need thereof.

25. A method of treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body, which comprises administering a therapeutically effective amount of the compound according to any one of claims 1 to 16 or a pharmaceutically acceptable salt thereof to a patient in need thereof.

26. The compound according to any one of claims 1 to 16 or a pharmaceutically acceptable salt thereof for use in the treatment and/or prophylaxis of a disease associated with orexin receptor type 2.

27. The compound according to any one of claims 1 to 16 or a pharmaceutically acceptable salt thereof for use in the treatment and/or prophylaxis of narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

28. Use of the compound according to any one of claims 1 to 16 or a pharmaceutically acceptable salt thereof, in the manufacture of a medicament for treating a disease associated with orexin receptor type 2.

29. Use of the compound according to any one of claims 1 to 16 or a pharmaceutically acceptable salt thereof, in the manufacture of a medicament for treating narcolepsy, idiopathic hypersomnia, hypersomnia, sleep apnea syndrome, narcolepsy syndrome involving narcolepsy-like symptom, hypersomnia associated with Parkinson's disease, or hypersomnia associated with dementia with Lewy body.

30. A combination medicament comprising a medicament comprising the compound according to any one of claims 1 to 16 or a pharmaceutically acceptable salt thereof and one or more drugs selected from drugs classified as a medicament for treating a disease such as narcolepsy, idiopathic hypersomnia, hypersomnia, sleep apnea syndrome, narcolepsy syndrome involving narcolepsy-like symptom, or a medicament for treating Parkinson's disease or dementia with Lewy body.

31. A medicament comprising the compound according to any one of claims 1 to 16 or a pharmaceutically acceptable salt thereof for treating a disease associated with orexin receptor type 2 in combination with one or more drugs selected from drugs classified as a medicament for treating a disease such as narcolepsy, idiopathic hypersomnia, hypersomnia, sleep apnea syndrome, narcolepsy syndrome involving narcolepsy-like symptom, or a medicament for treating Parkinson's disease or dementia with Lewy body.
